# EUROPEAN PATENT SPECIFICATION

(11) **EP 2 229 444 B1**
(45) Date of publication and mention of the grant of the patent: **30.10.2019**
(21) Application number: 09701822.0
(22) Date of filing: 08.01.2009
(51) Int. Cl.: C12N 15/11, C12N 5/10

(54) **GENERATION OF TUMOR-FREE EMBRYONIC STEM-LIKE PLURIPOTENT CELLS USING INDUCIBLE RECOMBINANT RNA AGENTS**
ERZEUGUNG TUMORFREIER UND PLURIPOTENTER EMBRYO-STAMMZELLEN MITHILFE INDUZIERBARER REKOMBINANTER RNA-WIRKSTOFFE
GÉNÉRATION DE CELLULES PLURIPOTENTES DE TYPE CELLULES SOUCHES EMBRYONNAIRES SANS TUMEUR UTILISANT DES AGENTS D'ARN RECOMBINANT INDUCTIBLE

(30) Priority: 16.01.2008 US 11333; 07.05.2008 US 149725; 08.09.2008 US 191327; 28.11.2008 US 193438
(43) Date of publication of application: 22.09.2010
(73) Proprietor: Lin, Shi-Lung, Arcadia, CA 91007 (US); Wu, David Ts, Arcadia, California 91006 (US)
(72) Inventor: Lin, Shi-Lung, Arcadia, CA 91007 (US); Wu, David Ts, Arcadia, California 91006 (US)
(74) Representative: Walker, Ross Thomson
(86) International application number: PCT/US2009/030432
(87) International publication number: WO 2009/091659

(56) References cited:
- WO-A-2005/056797
- WO-A-2009/058413
- WO-A-2009/079606
- US-A1- 2008 293 143
- LIN SHI-LUNG ET AL: "Role of mir-302 MicroRNA Family in Stem Cell Pluripotency and Renewal" CURRENT PERSPECTIVES IN MICRORNAS (MIRNA) SPRINGER, PO BOX 17, 3300 AA DORDRECHT, NETHERLANDS, 2008, pages 167-185, XP009121067 ISSN: 978-1-4020-8532-1(H)
- LIN SHI-LUNG ET AL: "Mir-302 reprograms human skin cancer cells into a pluripotent ES-cell-like state" RNA, COLD SPRING HARBOR LABORATORY PRESS, WOODBURY, NY, US, vol. 14, no. 10, 28 August 2008 (2008-08-28), pages 2115-2124, XP009108022 ISSN: 1355-8382
- WANG YANGMING ET AL: "DGCR8 is essential for microRNA biogenesis and silencing of embryonic stem cell self-renewal" NATURE GENETICS, vol. 39, no. 3, March 2007 (2007-03), pages 380-385, XP002540097 ISSN: 1061-4036
- ZHANG BAOHONG ET AL: "MicroRNA: A new player in stem cells" JOURNAL OF CELLULAR PHYSIOLOGY, vol. 209, no. 2, November 2006 (2006-11), pages 266-269, XP002540098 ISSN: 0021-9541
- SHCHERBATA H R ET AL: "The MicroRNA pathway plays a regulatory role in stem cell division" CELL CYCLE, vol. 5, no. 2, January 2006 (2006-01), pages 172-175, XP002540099 ISSN: 1538-4101(print) 1551-4005(ele
- HATFIELD S D ET AL: "Stem cell division is regulated by the microRNA pathway" NATURE (LONDON), vol. 435, no. 7044, June 2005 (2005-06), pages 974-978, XP002540100 ISSN: 0028-0836
- SUH MI-RA ET AL: "Human embryonic stem cells express a unique set of microRNAs" DEVELOPMENTAL BIOLOGY, ACADEMIC PRESS, NEW YORK, NY, US, vol. 270, no. 2, 6 May 2004 (2004-05-06), pages 488-498, XP002404396 ISSN: 0012-1606
- HOUBAVIY HRISTO B ET AL: "Embryonic stem cell-specific microRNAs." DEVELOPMENTAL CELL, vol. 5, no. 2, August 2003 (2003-08), pages 351-358, XP002540101 ISSN: 1534-5807
- TAKAHASHI KAZUTOSHI ET AL: "Induction of pluripotent stem cells from adult human fibroblasts by defined factors" CELL, CELL PRESS, CAMBRIDGE, NA, US, vol. 131, no. 5, 30 November 2007 (2007-11-30), pages 861-872, XP002478584 ISSN: 0092-8674

### SEQUENCE LISTING

<110> Lin, Shi-Lung Wu, David TS
<120> Generation of Tumor-Free Embryonic Stem-Like Pluripotent Cells Using Inducible Recombinant RNA Agents
<130> 08P044001USB
<150> 12/149725
   <151> 2008-05-07
<150> 61/011333
   <151> 2008-01-16
<150> 61/191327
   <151> 2008-09-08
<150> 11/278143
   <151> 2006-03-31
<150> 10/439262
   <151> 2003-05-13
<160> 49
<170> PatentIn version 3.5
<210> 1
   <211> 12
   <212> DNA
   <213> Artificial Sequence
<220>
   <223> Synthetic oligonucleotide
<400> 1
   gctaagccag gc 12
<210> 2
   <211> 12
   <212> DNA
   <213> Artificial Sequence
<220>
   <223> Synthetic oligonucleotide
<400> 2
   gcctggctta gc 12
<210> 3
   <211> 17
   <212> RNA
   <213> Artificial Sequence
<220>
   <223> Synthetic oligonucleotides
<400> 3
   uaagugcuuc cauguuu 17
<210> 4
   <211> 8
   <212> DNA
   <213> Artificial Sequence
<220>
   <223> Synthetic oligonucleotide
<400> 4
   gtaagagk 8
<210> 5
   <211> 10
   <212> DNA
   <213> Artificial Sequence
<220>
   <223> Synthetic oligonucleotide
<400> 5
   gwkscyrcag 10
<210> 6
   <211> 7
   <212> DNA
   <213> Artificial Sequence
<220>
   <223> Synthetic oligonucleotide
<400> 6
   tactway 7
<210> 7
   <211> 17
   <212> DNA
   <213> Artificial Sequence
<220>
   <223> Synthetic oligonucleotide
<400> 7
   tytycttttt tttttts 17
<210> 8
   <211> 19
   <212> DNA
   <213> Artificial Sequence
<220>
   <223> Synthetic oligonucleotide
<220>
   <221> misc_feature
   <222> (15)..(15)
   <223> n is a, c, g, or t
<400> 8
   tctctctctc tctcnctag 19
<210> 9
   <211> 23
   <212> RNA
   <213> Artificial Sequence
<220>
   <223> Synthetic oligonucleotide
<400> 9
   uaagugcuuc cauguuuuag ugu 23
<210> 10
   <211> 23
   <212> RNA
   <213> Artificial Sequence
<220>
   <223> Synthetic oligonucleotide
<400> 10
   uaagugcuuc cauguuuugg uga 23
<210> 11
   <211> 23
   <212> RNA
   <213> Artificial Sequence
<220>
   <223> Synthetic oligonucleotide
<400> 11
   uaagugcuuc cauguuuuag uag 23
<210> 12
   <211> 23
   <212> RNA
   <213> Artificial Sequence
<220>
   <223> Synthetic oligonucleotide
<400> 12
   uaagugcuuc cauguuucag ugg 23
<210> 13
   <211> 23
   <212> RNA
   <213> Artificial Sequence
<220>
   <223> Synthetic oligonucleotide
<400> 13
   uaagugcuuc cauguuugag ugu 23
<210> 14
   <211> 42
   <212> DNA
   <213> Artificial Sequence
<220>
   <223> Synthetic oligonucleotide
<400> 14
   gtaagaggat ccgatcgcag gagcgcacca tcttcttcaa ga 42
<210> 15
   <211> 46
   <212> DNA
   <213> Artificial Sequence
<220>
   <223> Synthetic oligonucleotide
<400> 15
   cgcgtcttga agaagatggt gcgctcctgc gatcggatcc tcttac 46
<210> 16
   <211> 42
   <212> DNA
   <213> Artificial Sequence
<220>
   <223> Synthetic oligonucleotide
<400> 16
   gtaagaggat ccgatcgctt gaagaagatg gtgcgctcct ga 42
<210> 17
   <211> 46
   <212> DNA
   <213> Artificial Sequence
<220>
   <223> Synthetic oligonucleotide
<400> 17
   cgcgtcagga gcgcaccatc ttcttcaagc gatcggatcc tcttac 46
<210> 18
   <211> 70
   <212> DNA
   <213> Artificial Sequence
<220>
   <223> Synthetic oligonucleotide
<400> 18
   gtaagaggat ccgatcgcag gagcgcacca tcttcttcaa gttaacttga agaagatggt 60
   gcgctcctga 70
<210> 19
   <211> 74
   <212> DNA
   <213> Artificial Sequence
<220>
   <223> Synthetic oligonucleotide
<400> 19
   cgcgtcagga gcgcaccatc ttcttcaagt taacttgaag aagatggtgc gctcctgcga 60
   tcggatcctc ttac 74
<210> 20
   <211> 47
   <212> DNA
   <213> Artificial Sequence
<220>
   <223> Synthetic oligonucleotide
<400> 20
   cgcgttacta actggtacct cttctttttt tttttgatat cctgcag 47
<210> 21
   <211> 45
   <212> DNA
   <213> Artificial Sequence
<220>
   <223> Synthetic oligonucleotide
<400> 21
   gtcctgcagg atatcaaaaa aaaaagaaga ggtaccagtt agtaa 45
<210> 22
   <211> 689
   <212> DNA
   <213> Heteractis spp.
<400> 22
   atggtgagcg gcctgctgaa ggagagtatg cgcatcaaga tgtacatgga gggcaccgtg     60
   aacggccact acttcaagtg cgagggcgag ggcgacggca accccttcgc cggcacccag    120
   agcatgagaa tccacgtgac cgagggcgcc cccctgccct tcgccttcga catcctggcc 180
   ccctgctgcg agtacggcag caggacgacc ttcgtgcacc acaccgccga gatccccgac 240
   ttcttcaagc agagcttccc cgagggcttc acctgggaga gaaccaccac ctacgaggac 300
   ggcggcatcc tgaccgccca ccaggacacc agcctggagg gcaactgcct gatctacaag 360
   gtgaaggtgc acggcaccaa cttccccgcc gacggccccg tgatgaagaa caagagcggc 420
   ggctgggagc ccagcaccga ggtggtgtac cccgagaacg gcgtgctgtg cggccggaac 480
   gtgatggccc tgaaggtggg cgaccggcac ctgatctgcc accactacac cagctaccgg 540
   agcaagaagg ccgtgcgcgc cctgaccatg cccggcttcc acttcaccga catccggctc 600
   cagatgctgc ggaagaagaa ggacgagtac ttcgagctgt acgaggccag cgtggcccgg 660
   tacagcgacc tgcccgagaa ggccaactg 689
<210> 23
   <211> 27
   <212> DNA
   <213> Artificial Sequence
<220>
   <223> Synthetic oligonucleotide
<400> 23
   ctcgagcatg gtgagcggcc tgctgaa 27
<210> 24
   <211> 27
   <212> DNA
   <213> Artificial Sequence
<220>
   <223> Synthetic oligonucleotide
<400> 24
   tctagaagtt ggccttctcg ggcaggt 27
<210> 25
   <211> 89
   <212> DNA
   <213> Artificial Sequence
<220>
   <223> Synthetic oligonucleotide
<400> 25
   gtaagtggtc cgatcgtcgc gacgcgtcat tactaactat caatatctta atcctgtccc 60
   ttttttttcc acagtaggac cttcgtgca 89
<210> 26
   <211> 89
   <212> DNA
   <213> Artificial Sequence
<220>
   <223> Synthetic oligonucleotide
<400> 26
   tgcacgaagg tcctactgtg gaaaaaaaag ggacaggatt aagatattga tagttagtaa 60
   tgacgcgtcg cgacgatcgg accacttac 89
<210> 27
   <211> 82
   <212> DNA
   <213> Artificial Sequence
<220>
   <223> Synthetic oligonucleotide
<400> 27
   gtccgatcgt cataagtgct tccatgtttt agtgtgctaa gccaggcaca ctaaaacatg 60
   gaagcactta tcgacgcgtc at 82
<210> 28
   <211> 82
   <212> DNA
   <213> Artificial Sequence
<220>
   <223> Synthetic oligonucleotide
<400> 28
   atgacgcgtc gataagtgct tccatgtttt agtgtgcctg gcttagcaca ctaaaacatg 60
   gaagcactta tgacgatcgg ac 82
<210> 29
   <211> 91
   <212> DNA
   <213> Artificial Sequence
<220>
   <223> Synthetic oligonucleotide
<400> 29
   gtccgatcgt cccaccactt aaacgtggat gtacttgctt tgaaactaaa gaagtaagtg 60
   cttccatgtt ttggtgatgg atctcgagct c 91
<210> 30
   <211> 91
   <212> DNA
   <213> Artificial Sequence
<220>
   <223> Synthetic oligonucleotide
<400> 30
   gagctcgaga tccatcacca aaacatggaa gcacttactt ctttagtttc aaagcaagta 60
   catccacgtt taagtggtgg gacgatcgga c 91
<210> 31
   <211> 95
   <212> DNA
   <213> Artificial Sequence
<220>
   <223> Synthetic oligonucleotide
<400> 31
   atctcgagct cgctcccttc aactttaaca tggaagtgct ttctgtgact ttgaaagtaa 60
   gtgcttccat gttttagtag gagtcgctag cgcta 95
<210> 32
   <211> 95
   <212> DNA
   <213> Artificial Sequence
<220>
   <223> Synthetic oligonucleotide
<400> 32
   tagcgctagc gactcctact aaaacatgga agcacttact ttcaaagtca cagaaagcac 60
   ttccatgtta aagttgaagg gagcgagctc gagat 95
<210> 33
   <211> 90
   <212> DNA
   <213> Artificial Sequence
<220>
   <223> Synthetic oligonucleotide
<400> 33
   cgctagcgct acctttgctt taacatggag gtacctgctg tgtgaaacag aagtaagtgc 60
   ttccatgttt cagtggaggc gtctagacat 90
<210> 34
   <211> 90
   <212> DNA
   <213> Artificial Sequence
<220>
   <223> Synthetic oligonucleotide
<400> 34
   atgtctagac gcctccactg aaacatggaa gcacttactt ctgtttcaca cagcaggtac 60
   ctccatgtta aagcaaaggt agcgctagcg 90
<210> 35
   <211> 82
   <212> DNA
   <213> Artificial Sequence
<220>
   <223> Synthetic oligonucleotide
<400> 35
   cgtctagaca taacactcaa acatggaagc acttagctaa gccaggctaa gtgcttccat 60
   gtttgagtgt tcgacgcgtc at 82
<210> 36
   <211> 82
   <212> DNA
   <213> Artificial Sequence
<220>
   <223> Synthetic oligonucleotide
<400> 36
   atgacgcgtc gaacactcaa acatggaagc acttagcctg gcttagctaa gtgcttccat 60
   gtttgagtgt tatgtctaga cg 82
<210> 37
   <211> 10
   <212> DNA
   <213> Artificial Sequence
<220>
   <223> Synthetic oligonucleotide
<400> 37
   gtaagaggat 10
<210> 38
   <211> 6
   <212> DNA
   <213> Artificial Sequence
<220>
   <223> Synthetic oligonucleotide
<400> 38
   guragu 6
<210> 39
   <211> 8
   <212> DNA
   <213> Artificial Sequence
<220>
   <223> Synthetic oligonucleotide
<400> 39
   gtaagagt 8
<210> 40
   <211> 7
   <212> DNA
   <213> Artificial Sequence
<220>
   <223> Synthetic oligonucleotide
<400> 40
   gtagagt 7
<210> 41
   <211> 6
   <212> DNA
   <213> Artificial Sequence
<220>
   <223> Synthetic oligonucleotide
<400> 41
   gtaagt 6
<210> 42
   <211> 12
   <212> DNA
   <213> Artificial Sequence
<220>
   <223> Synthetic oligonucleotide
<400> 42
   gatatcctgc ag 12
<210> 43
   <211> 11
   <212> DNA
   <213> Artificial Sequence
<220>
   <223> Synthetic oligonucleotide
<400> 43
   wuccaagggg g 11
<210> 44
   <211> 20
   <212> DNA
   <213> Artificial Sequence
<220>
   <223> primer
<400> 44
   gaggagttga gggtactgtg 20
<210> 45
   <211> 20
   <212> DNA
   <213> Artificial Sequence
<220>
   <223> primer
<400> 45
   gaggagctga gggcactgtg 20
<210> 46
   <211> 21
   <212> DNA
   <213> Artificial Sequence
<220>
   <223> primer
<400> 46
   gtagaagtgc ctctgccttc c 21
<210> 47
   <211> 27
   <212> DNA
   <213> Artificial Sequence
<220>
   <223> primer
<400> 47
   gaggctggag tagaaggatt gctttgg 27
<210> 48
   <211> 27
   <212> DNA
   <213> Artificial Sequence
<220>
   <223> primer
<400> 48
   ccctcctgac ccatcacctc caccacc 27
<210> 49
   <211> 63
   <212> DNA
   <213> Artificial Sequence
<220>
   <223> primer
<400> 49
   ggccagtgaa ttgtaatacg actcactata gggaggcggt tttttttttt tttttttttt 60
   ttt 63

## Description

### CLAIM OF THE PRIORITY

The present application claims priority to the U.S. Provisional Application Serial No. 61/193,438 filed on November 28, 2008, entitled "Generation of Tumor-Free Embryonic Stem-Like Pluripotent Cells Using Inducible Recombinant RNA Agents". The present application claims priority to the U.S. Provisional Application Serial No. 61/011,333 filed on January 16, 2008, entitled "Generation of Human Embryonic Stem-Like Pluripotent Cells Using Intronic RNA". The present application also claims priority to U.S. Provisional Application Serial No. 61/191,327 filed on September 08, 2008, entitled "Generation of Tumor-Free Embryonic Stem-Like Pluripotent Cells Using Inducible Recombinant RNA Agents". Furthermore, the present application is a continuation-in-part application of the U.S. Patent Applications No. 10/439,262 filed on May 15, 2003, entitled "RNA-Splicing and Processing-Directed Gene Silencing and the Relative Applications Thereof", No. 11/278,143 filed on March 31, 2006, entitled "Novel Transgenic Methods Using Intronic RNA", and No. 12/149,725 filed on May 07, 2008, entitled "Generation of Human Embryonic Stem-Like Cells Using Intronic RNA".

### FIELD OF THE INVENTION

This invention generally relates to a means and method for developing, generating and selecting tumor-free embryonic stem (ES)-like pluripotent cells using transgenic expression of a recombinant tumor-suppressor microRNA (miRNA) or shRNA agents in the cells of interest. More particularly, the present invention relates to a transgenic method and an inducible nucleic acid composition for generating a non-naturally occurring recombinant intron and its components capable of being spliced and processed into small RNA gene silencing effectors (pre-miRNA and/or shRNA) in mammalian cells and then inducing certain specific gene silencing effects on developmental and cell differentiation-associated genes and oncogenes, resulting in reprogramming the cells into an ES-like pluripotent state. Preferably, the small RNA gene silencing effectors include tumor suppressor-like miRNAs such as mir-302a, mir-302b, mir-302c, mir-302d, and their manually re-designed shRNA homologues as well as a combination thereof. In other words, the pluripotent stem-like cells so generated can be cultured under a feeder-free cell cultural condition. The cells of interest include isolated somatic or cancer cells *in vitro, ex vivo* and/or *in vivo.*

### BACKGROUND OF THE INVENTION

Recent research in human stem cells has shown a highly promising potential in transplantation therapy. Nevertheless, the sources for cloning human stem cells are limited and very difficult to control their purity and quality. In 1998, James Thomson *et al*. (*e*.*g*. U.S. Pat. No. 5,843,780, No. 6,200,806, No. 7,029,913, and No. 7,220,584) isolated the first human embryonic stem (ES) cell line from the late blastocysts of human embryos (Thomson et al., (1998) Science 282: 1145-1147). H1 and H9 cells were two typical cell lines derived from these isolated human ES cells. Two years later, Gearhart *et al*. (*e*.*g*. U.S. Pat. No. 6,090,622, No. 6,245,566, and No. 6,331,406) also developed a method to isolate ES-like primordial germ cells from post-blastocyst human embryos. Because the way of these ES cell isolation methods must destroy the original embryos, many ethical and humanity concerns have been raised to question the righteousness of using these ES cells for clinical therapy.

In recent years, problems in therapeutical safety and the informed consent of use are also noticed. For instance, because the ES cell growth requires some undefined factors released from surrounding "feeder" fibroblasts, all human ES cells are preferably grown on a layer of mouse or human fibroblasts (U.S. Pat. No. 6,875,607 to Reubinoff et al.). However, these fibroblast feeders present different surface antigens, which often contaminate the purity of ES cell antigens and may cause immune rejection in patients. Although some feeder-free cultural conditions have been developed, none of these feeder-free approaches are able to maintain the undifferentiated ES cell state for a long time. Unfortunately, human ES cell lines cannot reach a 100% pure population in any currently available cultural condition. Even under the best feeder-containing cultural condition, a few (about 5%-10% or more) ES cells always tend to differentiate into other tissue cell types and lose their stem cell properties. One of the most frequently observed cell types derived from the ES cells is teratoma. Teratoma is a tumor usually derived from human germ line cells, containing multiple tumor-like cell types similar to embryonic endoderm, mesoderm and ectoderm tissues. Therefore, how to prevent feeder cell contamination, increase stem cell purity and reduce the risk of tumor formation are three major tasks of the present stem cell research.

Induced pluripotent stem (iPS) cells were introduced by Takahashi and Yamanaka in 2006 (Cell 126: 663-676). Using retroviral delivery of four transcription factor genes [*Oct3*/*4* (*Oct4*), *Sox2*, *c-Myc* and *Klf4*] into mouse fibroblasts, they successfully reprogrammed and transformed the somatic fibroblasts to ES-like iPS cell lines *in vitro.* The success rate of this approach was estimated to be less than 0.002%-2% in the entire tested cell population. In 2007, the genetic and behavioral properties of these iPS cells were observed to resemble those of the mouse ES cells (Okita et al., (2007) Nature 448: 313-317; Wernig et al., (2007) Nature 448: 318-324). Meanwhile, Yu *et al*. developed new iPS cell lines derived from human fibroblasts, using a similar approach but with a different set of transcription factors including *Oct4*, *Sox2*, *Nanog* and *LIN28* (Yu et al., (2007) Science 318: 1917-1920). Nevertheless, the Yu's method was much less efficient than the Takahashi's method. The advantages of these iPS cell applications were shown to not only solve the ethical problem of the previous ES cell approach but also provide a potential patient-friendly therapy if in conjunction with the somatic cell nuclear transfer (SCNT) technology (Meissner et al., (2006) Nature 439: 212-215). Such an iPS-based SCNT therapy has been tested for treating sickle cell anemia in a transgenic mouse model (Hanna et al., (2007) Science 318: 1920-1923). Yet, there are two problems unsolved; first is the use of retroviral transgenes, and secondly the use of oncogenes (*e*.*g*. *c-Myc* and *Klf4*). Retroviral infection is the only effective means capable of transgenically delivering four large transcription factor genes into a targeted host cell; however, the random insertion of multiple retroviral vectors into the targeted cell genome may also affect other non-target genes. This is problematic because uncertain retroviral insertion often causes cell mutations in particular, when one or more of the transgenes are oncogenes. How to prevent the tumor formation from these transcription factor-induced iPS cells is obscure now.

Another disadvantage of iPS cells is their heterogeneity. To generate an iPS cell, at least three or more different transcription factor genes need to be inserted into the single cell genome through retroviral infection. However, because different retroviral transgenes present different rates of delivery efficiency and insertional mutation, multiple retroviral insertions often result in a variety of transgene combinations in the host cell genomes. Only the cells with proper ratios and numbers of the four transcription factor genes can become iPS cells with good pluripotency. That is why the iPS cells merely represent 0.002%-2% of the entire cell population after retroviral insertion, whereas the other >98% of the cells are transformed by a complexity of uncertain transgene combinations. To collect pure iPS cells with a correct transgene combination, a series of tedious cell selection procedures are required (U.S. Pat. No. 7,250,255 to Shinya Yamanaka). Although the mechanism of iPS cell formation is not clear, this technology does require multiple transcriptional regulators to coordinate the transition between re-activation of embryonic genes and inactivation of developmental signals. The combined effects of *Oct4-Sox2-c-Myc-Klf4* or *Oct4-Sox2-Nanog-LIN28* genes seem to directly activate certain embryonic genes; however, it is uncertain how these effects result in a cancellation of global developmental signals essential for somatic cell differentiation. Despite *Oct4,* all other transgenes used by the iPS methods are actually involved in the initiation of certain tissue cell development. By placing them together, the coordination or disturbance of these developmental signals somehow halts the cell differentiation and then transforms the cell back to an ES-like state. This is not a natural mechanism and may contain uncertain risks, such as cell mutation and tumor formation.

Experiments of somatic cell nuclear transfer (SCNT) have shown that hybrids of somatic nuclei and oocyte cytoplasm can form pluripotent stem-like cells, indicating that certain maternal elements in the oocyte cytoplasm rather than the transcription factors in the cell nuclei play an important role in nuclear reprogramming (Simonsson and Gurdon, (2004) Nat Cell Biol. 6: 984-990). In natural fertilized eggs and early zygotes before the morula stage, maternal elements are responsible for the regulation and maintenance of normal stem cell renewal and pluripotency, completely without the risk of tumor formation. That is why embryonic cells before the 32-64-cell (morula) stage are all the same and totipotent. Maternal elements are produced during oogenesis and deposited in a mature oocyte required for initial embryogenesis. Mouse oocytes lacking Dicer, a conserved ribonuclease required for microRNA biogenesis, arrest in the division phase of meiosis I, indicating that microRNAs are one of major maternal elements in oocytes (Murchison et al, (2007) Genes Dev. 21: 682-693). In a murine oocyte, RNAs occupy a large volume of maternal elements, corresponding to about 45% of the whole genomic transcriptome (Stitzel et al, (2007) Science 316: 407-408). During maternal-zygotic transition, these maternal RNAs are quickly degraded and the transcription of zygotic genes starts as early as at the 2-4-cell stage to produce signals for embryonic development (O'Farrell et al, (2004) Curr. Biol. 14: R35-45). It is conceivable that many of these maternal RNAs are inhibitors of the zygotic genes in order to synchronize developmental signals and maintain the totipotent/pluripotent ES cell renewal at the initial stage of embryonic development. Thus, maternal RNAs are likely one of the key maternal elements essential for ES cell maintenance and renewal.

In sum, the natural way of ES cell maintenance and renewal relies on certain maternal RNAs (as inhibitors) rather than the four transcription factors (as activators) used in the iPS technology. To generate ES-like pluripotent cells mimicking the natural mechanism of ES cell maintenance and renewal, a new strategy is highly desired to identify and evaluate the functions of these maternal RNAs. The identified maternal RNAs may be delivered into human adult stem or somatic cells to either maintain the stem cell properties or reprogram the somatic cells into an ES-like state, or both. Therefore, there remains a need for an effective, simple and safe method as well as agent composition for generating ES-like pluripotent cells, preferably using maternal RNAs.

WO2009/058413 discloses a method for developing, generating and selecting human embryonic stem (h ES)-like cells using transgenic expression of intronic hairpin RNA agents.

### SUMMARY OF THE INVENTION

The present invention provides an in vitro method for reprogramming at least a cell into at least a pluripotent stem-like cell in accordance with claim 1. The scope of the present invention is defined by the claims. Any subject matter present herein that falls outside of the claims is provided for information purposes only.

In other words, the present invention is a method for developing, generating and selecting embryonic stem (ES)-like pluripotent cells, using ectopic expression of hairpin-like recombinant microRNA (miRNA) agents, such as mir-302a, mir-302b, mir-302c, mir-302d, and their manually re-designed miRNA precursors (pre-miRNA) and/or small hairpin RNA (shRNA) homologues as well as a combination thereof. The design of non-naturally occurring/man-made/artificial mir-302 agents include mismatched and perfectly matched constructs of small hairpin RNA (shRNA) and/or small interfering RNA (siRNA) homologues or clusters, all of which may improve the target specificity and reduce the copy number of mir-302 required for transgene delivery and gene silencing.

Native microRNA (miRNA) is usually about 18-27 nucleotides (nt) in length and capable of either directly degrading its targeted messenger RNA (mRNA) or suppressing the translation of the target mRNA, depending on their mutual complementarity. The mir-302 family (mir-302s) is a group of highly homologous intergenic miRNAs sharing over 89% homology and conserved in almost all mammals. Mir-302s consists of four members which are transcribed together as a non-coding RNA cluster containing mir-302b, mir-302c mir-302a, mir-302d and mir-367 in a 5' to 3' direction (Suh et al., (2004) Dev. Biol. 270: 488-498). Although mir-367 and mir-302s are co-expressed, their functions are actually different from each other in view of their distinct seed motifs against different sets of target genes. The expression of mir-302s has been found to be extremely high in many mammalian early zygotes and embryonic stem (ES) cells (Tang et al., (2007) Genes Dev. 21: 644-648; Suh et al., (2004) Dev. Biol. 270: 488-498). They are presented most abundantly in slow-growing ES cells and quickly decrease after cell differentiation and/or proliferation. Mouse oocytes lacking Dicer, a conserved ribonuclease required for miRNA biogenesis, arrest in the division phase of meiosis I, indicating that these miRNAs play a critical role in oogenesis (Murchison et al., (2007) Genes Dev. 21: 682-693). Given that miRNAs are characterized as small inhibitory RNAs capable of suppressing the translation of target genes with high complementarity (Bartel, D.P. (2004) Cell 116: 281-297), mir-302s is likely a key maternal inhibitor responsible for preventing any possible premature differentiation of ES cells during early embryogenesis. These findings suggest that the mir-302 family plays an important role in normal ES cell maintenance and renewal.

All mir-302 members share an identical (100%) sequence in their 5' first seventeen (17) nucleotides, including the entire seed motif, and an overall 83%-96% homology in their 23-nucleotide mature miRNA sequences. The seed motif is located in the first 5' eight nucleotides of a mature miRNA sequence, which determines the binding specificity and efficiency between the miRNA and its target genes. Based on the prediction of "TARGETSCAN" (http://www.targetscan.org/vert_42/) and "PICTAR-VERT" (http://pictar.bio.nyu.edu/cgi-bin/PicTar_vertebrate.cgi?) programs linked to the Sanger miRBase:: Sequences website (http://microrna.sanger.ac.uk/), they are directed against almost the same cellular genes, including over 445 conserved genes in human and mouse. Moreover, mir-302 also shares certain overlapping target genes with mir-93, mir-367, mir-371, mir-372, mir-373, and mir-520 familial members. Most of these target genes are developmental signals and transcriptional factors involved in initiation and/or facilitation of lineage-specific cell differentiation during early embryogenesis (Lin et al., (2008b) RNA 14: 2115-2124). Many of these target genes are also well-known oncogenes. Therefore, the function of mir-302s is more likely to suppress the global production of developmental signals and differentiation-related transcription factors rather than to create transcriptional stimulation on certain embryonic signaling pathways like the previous iPS methods. Furthermore, since many of these targeted developmental signals and differentiation-related transcription factors are oncogenes, mir-302s may also function as a tumor suppressor to prevent the deviation of normal ES cell renewal into tumor formation. In other words, the present invention provides a method for generating tumor-free ES-like pluripotent cells. For example, insulin-like growth factors (IGF) are potent developmental signals for the differentiation of neuron-specific cell lineage via either the Ras/Raf/mitogen-activated protein kinase (MAPK) or the phosphatidylinositol 3-kinase (PI3K)/Akt signal transduction pathway. The same signaling pathways are also involved in many tumor/cancer transformations, such as brain tumor, breast cancer, lung cancer, prostate cancer, and skin melanoma. The inventors found that over eighteen members of the IGF receptor (IGFR)-Ras/PI3K signaling pathways are strong targets of mir-302s, indicating that there is an extremely tight blockade of neuronal cell differentiation in mammalian oocytes and ES cells. Similar inhibitory effects of mir-302s on many other various cell lineages are also observed. Because of these evidences, the inventors believe that mir-302s is the key regulator for normal ES cell maintenance and renewal, which may be able to reprogram differentiated somatic cells into a homogeneous ES-like state.

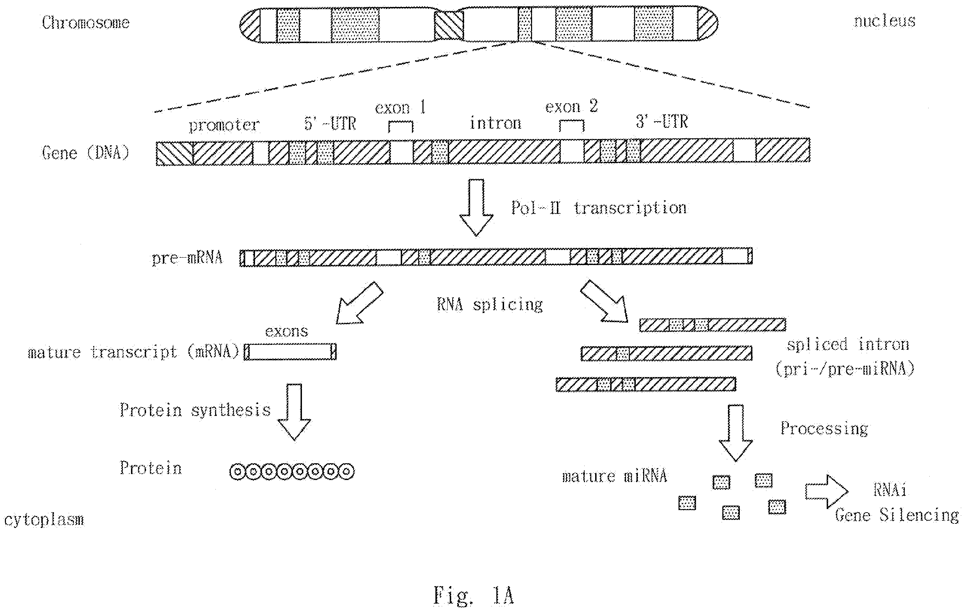

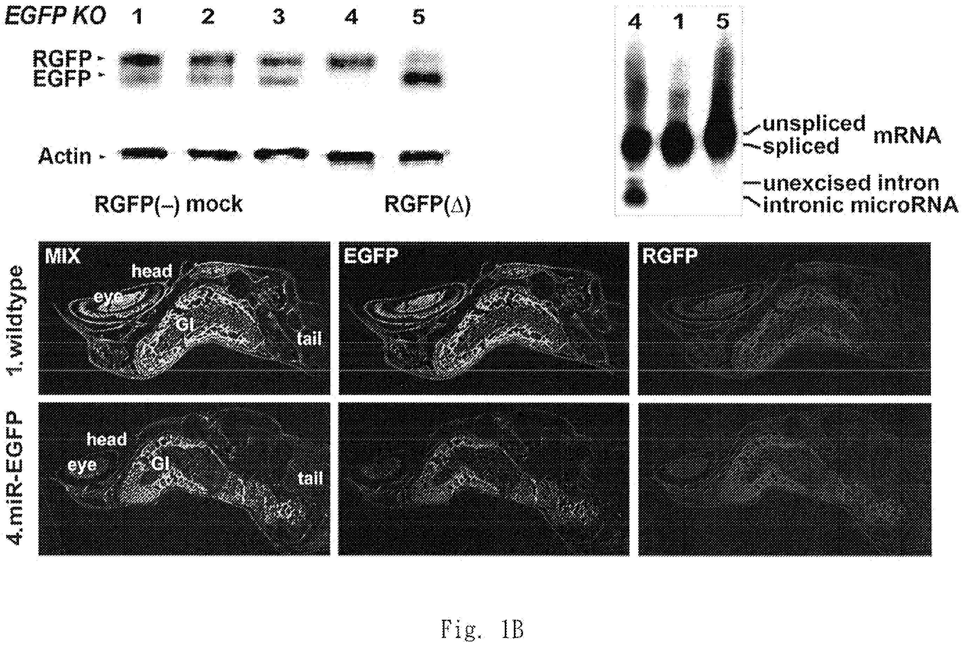

To test the function of mir-302s, the inventors have developed a Pol-II-driven miRNA expression system based on the natural intronic miRNA biogenesis mechanism (Fig.1A) and successfully used this system to generate native miRNAs as well as man-made shRNAs *in vitro* and *in vivo* (Fig.1B). In broad definition, the intron is a non-coding sequence of a gene, including in-frame intron, 5'-untranslated region (5'-UTR) and 3'-UTR. Our previous studies have demonstrated that effective mature miRNAs can derive from these intron regions of mammalian genes, namely intronic miRNA (Lin et al. (2003) Biochem Biophys Res Commun. 310: 754-760; Lin et al. (2005) Gene 356: 32-38). Intronic miRNA expression is a prevalent event in mammals because approximately 50% of mammalian miRNAs are encoded within the introns of protein-coding genes (Rodriguez et al., (2004) Genome Res. 14: 1902-1910). As shown in Fig.1A, intronic miRNA biogenesis relies on a coupled interaction between nascent Pol-II-mediated pre-mRNA transcription and intron splicing/excision, occurring within certain nuclear regions proximal to genomic perichromatin fibrils (Ghosh et al., (2000) RNA 6: 1325-1334; Lin et al. (2008a) Frontiers in Bioscience 13: 2216-2230). These miRNAs are transcribed within the primary transcripts of their host genes (pre-mRNAs) by type-II RNA polymerases (Pol-II) and spliced by spliceosomes and other RNaseIII endonucleases to form mature miRNAs (Lin *et al*., 2003; Danin-Kreiselman et al., (2003) Mol Cell 11: 1279-1289); however, *Drosha* may be not required for this process (Ruby et al., (2007) Nature 448: 83-86). As a result, intronic miRNA biogenesis is tightly regulated by multiple intracellular surveillance systems, including Pol-II transcription, RNA splicing, exosomal processing and nonsense-mediated RNA decay (NMD). In other words, the miRNA-like gene-silencing effector is released by an intracellular mechanism selected from the group consisting of RNA splicing, exosomal processing, nonsense-mediated decay, and a combination thereof. Due to this high intracellular surveillance, the problem of RNA over-saturation found in other shRNA/siRNA expression systems can be prevented, leading to a more effective, target-specific and safer gene silencing effect on targeted genes (Lin *et al*., 2008a).

By mimicking the natural intronic miRNA pathway (Fig.1A), the inventors devise a novel intronic miRNA expression system to transcribe a recombinant transgene of red-shifted fluorescent protein *(RGFP),* namely *SpRNAi-RGFP*, which contains a man-made/artificial splicing-competent intron (*SpRNAi*) capable of producing intronic miRNA and/or shRNA-like gene silencing effectors. The *SpRNAi* is co-transcribed within the pre-mRNA of the *SpRNAi-RGFP* gene by Pol-II and cleaved out by RNA splicing. Subsequently, the spliced *SpRNAi* is further processed into mature gene silencing effectors, such as native miRNAs and man-made shRNAs, so as to trigger specific posttranscriptional gene silencing (PTGS) effects on target genes. Meanwhile, after intron splicing, the exons of the *SpRNAi-RGFP* gene transcript are linked together to form a mature mRNA for translation of a RGFP marker protein useful for identifying the miRNA/shRNA expression. Alternatively, some functional protein exons may be used in place of RGFP to provide additional gene functions, such as embryonic stem (ES) gene markers for somatic cell reprogramming. In other words, the gene silencing effector can induce an intracellular gene silencing effect through posttranscriptional gene silencing, translational suppression, RNA interference, and/or nonsense-mediated decay. Given that there are currently over 1000 native miRNA species found in vertebrates without clear function and many more new miRNAs continue to be identified, our intronic miRNA expression system may serve as a powerful tool for testing these miRNA functions *in vitro* and *in vivo.*

The *SpRNAi* intron includes several consensus nucleotide components, consisting of a 5'-splice site (SEQ.ID.NO.4), a branch-point motif (BrP; SEQ.ID.NO.6), a poly-pyrimidine tract (PPT; SEQ.ID.NOs.7 and 8), and a 3'-splice site (SEQ.ID.NO.5). In addition, a hairpin miRNA or shRNA precursor is inserted in between the 5'-splice site and the BrP motif. This portion of intron usually forms a lariat structure during RNA splicing and processing. Moreover, the 3'-end of *SpRNAi* contains a multiple translational stop codon region (T codon) to increase the accuracy of intronic RNA splicing and NMD processing. When presented in a cytoplasmic mRNA, this T codon signals the activation of the NMD system to degrade any unstructured RNA accumulated in the cell. However, the highly structured shRNA and precursor miRNA (pre-miRNA) will be preserved for further *Dicer* cleavage to form mature siRNA and miRNA, respectively. For transgenic expression, we manually incorporate the *SpRNAi* in the *DraII* restriction site (208^{th} nucleotide) of the *RGFP* gene (SEQ.ID.NO.22). This forms a recombinant *SpRNAi-RGFP* transgene. The cleavage of *RGFP* with DraII generates an AG-GN nucleotide break with three recessing nucleotides in each end, which will form 5'- and 3'-splice sites, respectively, after *SpRNAi* insertion. Because this intronic insertion disrupts the integrity of RGFP protein, which can be recovered by intron splicing, we are able to determine the release of intronic miRNA/shRNA and the maturation of *RGFP* mRNA through the appearance of red RGFP in the affected cells. The *RGFP* gene also contains multiple exonic splicing enhancers (ESEs) to increase RNA splicing accuracy and efficiency.

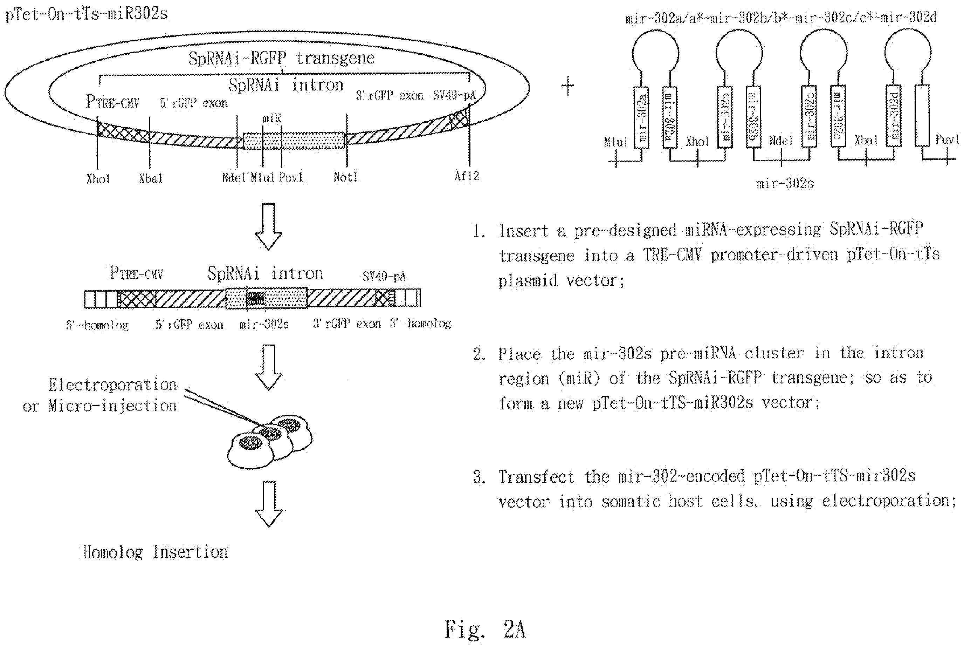

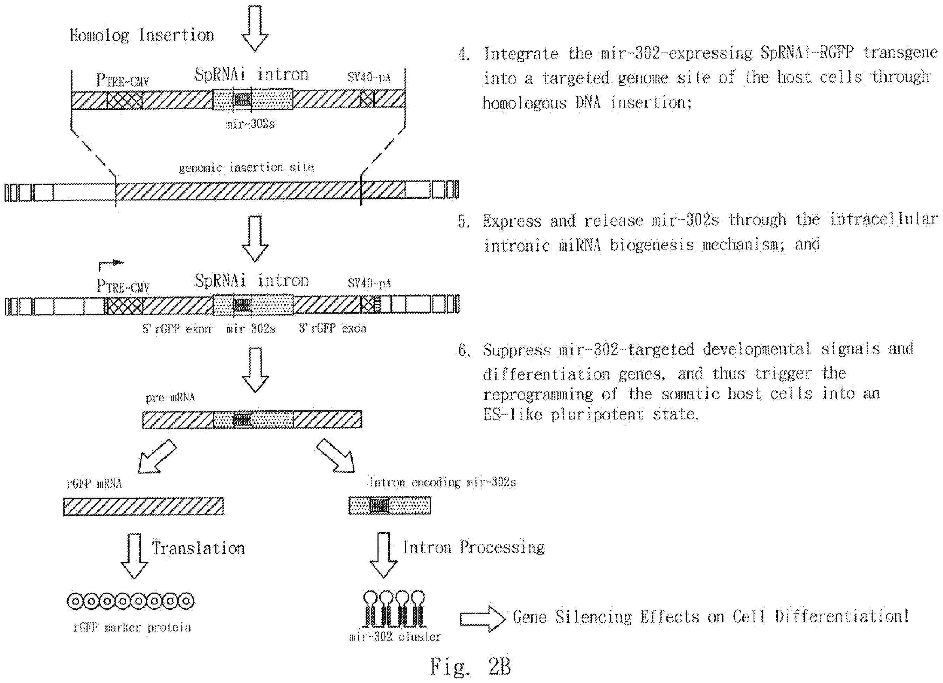

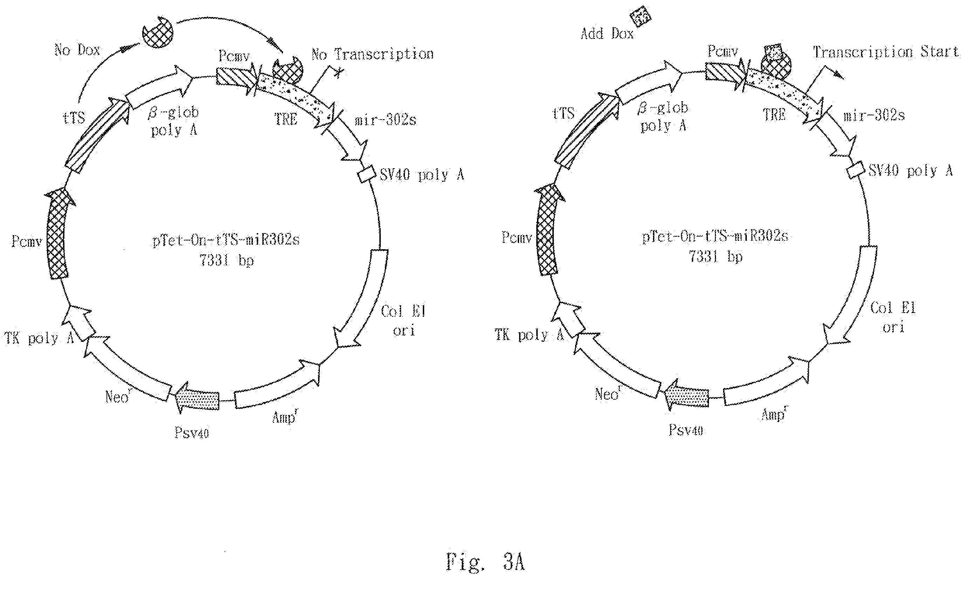

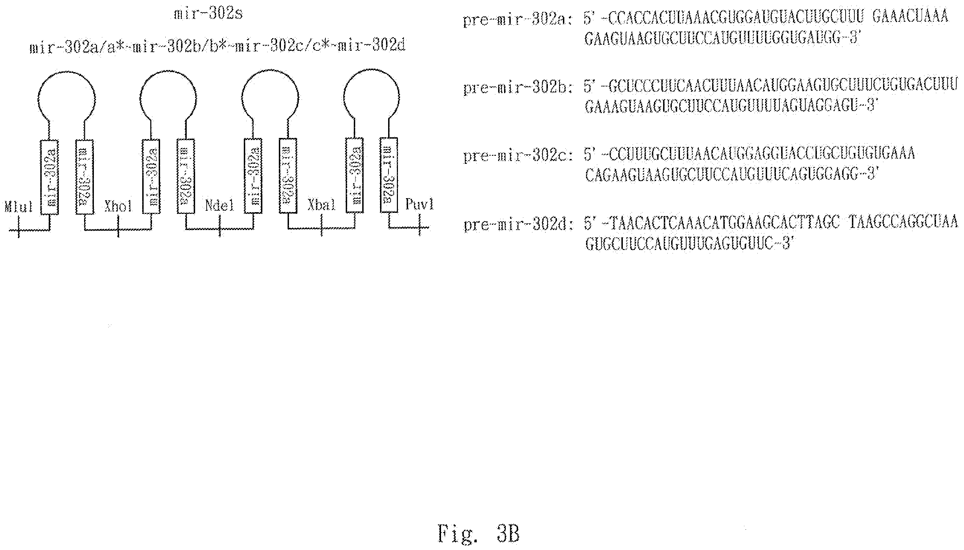

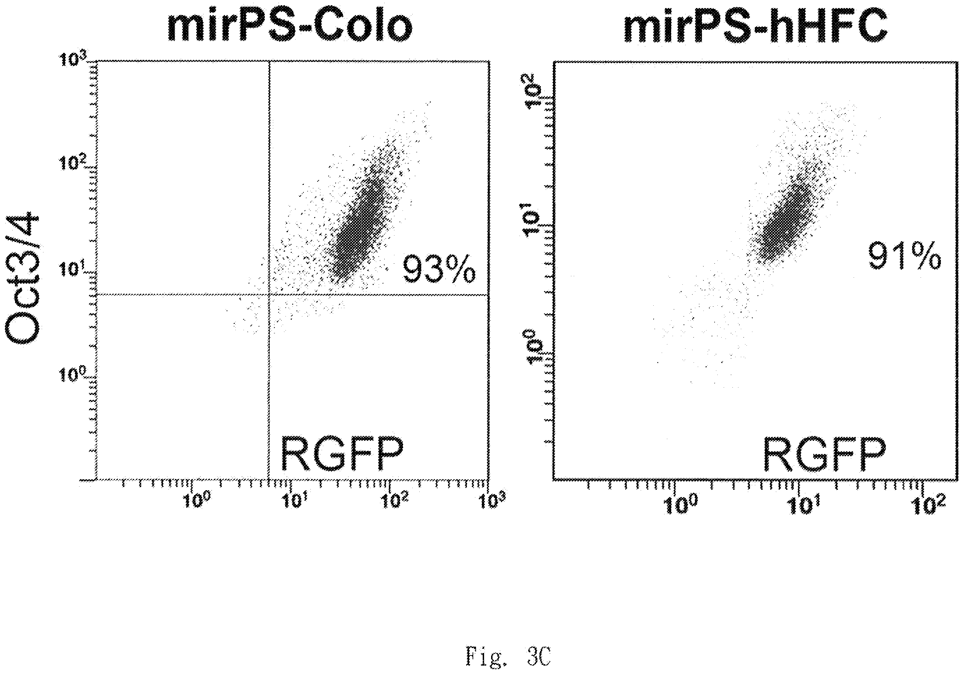

In a preferred embodiment, the present invention is an inducible miRNA/shRNA expression system (Figs.2A and 2B), which improves the control of miRNA/shRNA expression levels *in vitro* and *in vivo.* This improvement not only adopts safer electroporation/micro-injection methods for transgene delivery in place of tumor-prone retroviral infection but also prevent possible RNA over-accumulation in the transfected cells. Based on this improvement, the inventors have successfully generated various mir-302-transduced pluripotent stem (mirPS) cell lines derived from human primary cultures of normal epidermal skin cells (mirPS-hpESC), normal hair follicle cells (mirPS-hHFC), and cancerous breast adenocarcinoma MCF7 (mirPS-MCF7), prostate carcinoma PC3 (mirPS-PC3) and skin melanoma Colo 829 (mirPS-Colo) cells. As shown in Figs.2A and 2B, the inventors first incorporated a man-made mir-302s pre-miRNA/shRNA construct (Fig.3B) into the intronic insertion site (*i*.*e*. *MluI-PvuI* restriction/cloning site) of an *SpRNAi-RGFP* transgene, and then insert the *SpRNAi-RGFP* transgene into the multiple cloning site (*i*.*e*. *XhoI-ClaI* restriction site) of a doxycyclin (Dox)-inducible *pTet*-*On*-*tTS* vector, so as to form a *pTet-On-tTS-mir302s* transgene vector (Fig.3A). In other words, the recombinant nucleic acid composition (such as *SpRNAi*, *SpRNAi-RGFP* and so on) contains a drug-inducible gene expression vector. Besides, the *SpRNAi-RGFP* can also be incorporated into a gene expression vector selected from the group consisting of plasmid, viral vector, retrotransposon and a combination thereof. The *SpRNAi-RGFP* transgene is flanked with a 370 base-pair (bp) homologous region for recombinational insertion into a target site of the host cell genome. For transgenic delivery, the *pTet*-*On*-*tTS*-*mir302s* vector (10-30 µg) is mixed with the host cells (200-2000) in a hypoosmolar PH buffer (400 µl; Eppendorf) and electroporation is performed at 400-450 V for 100 µsec to deliver the transgene into the host cell genomes. Positively transgenic mirPS cells are isolated and collected 72 hours later, using FACS flow cytometry selection with both anti-RGFP and anti-Oct3/4 monoclonal antibodies (Fig.3C). The success rate of this novel mir-302s transgene approach is measured to be over 91%, which is much higher that the 0.002%-2% rate found in the previous iPS methods. All sequences of the man-made mir-302s are chemically synthesized based on the sequence databases of the Sanger miRBase::Sequences program. The *pTet*-*On*-*tTS* vector encodes a *CMV*-driven *tTS* inhibitor gene to inactivate the *TRE-CMV* promoter of the transgene. In the presence of doxycyclin (Dox), the inhibitory function of tTS is neutralized by Dox and thus the *SpRNAi-RGFP* transgene and its encoding mir-302s are expressed.

In another preferred embodiment, the present invention provides a genetic engineering method for construction of an artificial/man-made *SpRNAi* intron containing at least one desired insert for producing mir-302s or mir-302-like miRNA, shRNA and/or antisense RNA gene silencing effectors. The *SpRNAi* is formed by linkage of synthetic oligonucleotide elements required for RNA splicing, such as 5'-splice site, BrP, PPT, 3'-splice site, and some linker oligonucleotides. An oligonucleotide synthesizer can chemically produce and link these elements. In other words, the intron (such as *SpRNAi*) is synthesized by a chemically synthesizing method. Alternatively, the linkage of these elements can be achieved by enzymatic restriction and ligation. In other words, the intron (such as *SpRNAi*) is also formed by a nucleotide recombination method. The intron (such as *SpRNAi*) so obtained can be either directly used for transfection into the cells of interest or further incorporated into a host gene for co-expression along with the gene transcript (pre-mRNA). Thus, the presently invented recombinant nucleic acid composition further contains a recombinant intron encoding at least an RNA gene silencing effector like mir-302. In general, the methods for intron insertion include enzymatic restriction/cloning, homologous DNA recombination, transposon incorporation, jumping gene integration, retroviral infection, and a combination thereof. The host gene is selected from the group of fluorescent protein (GFP) marker genes, embryonic stem (ES) marker genes, luciferase, lac-Z reporter genes, viral genes, transposons, jumping genes, artificially recombinant genes, and natural cellular genes. In other words, the recombinant nucleic acid composition further includes a plurality of exons, which are selected from the group of fluorescent protein marker genes, luciferase genes, lac-Z reporter genes, embryonic stem cell marker genes, viral genes, bacterial genes, cellular marker genes, jumping genes, transposons and a combination thereof. Without limitation, the present invention preferably uses a modified red fluorescent protein *(RGFP)* gene for indicating the expression of mir-302s.

In one aspect, the *SpRNAi* intron contains a 5'-splice site homologous to either 5'-GTAAGAGK-3' (SEQ.ID.NO.4) or GU(A/G)AGU motifs (SEQ.ID.NO.38) (*i*.*e*. 5'-GTAAGAGGAT-3'(SEQ.ID.NO.37), 5'-GTAAGAGT-3'(SEQ.ID.NO.39), 5'-GTAGAGT-3'(SEQ.ID.NO.40) and 5'-GTAAGT-3'(SEQ.ID.NO.41)), while its 3'-end is a 3'-acceptor splice site that is homologous to either GWKSCYRCAG (SEQ.ID.NO.5) or CT(A/G)A(C/T)NG motifs (*i*.*e*. 5'-GATATCCTGC AG-3'(SEQ.ID.NO.42), 5'-GGCTGCAG-3' and 5'-CCACAG-3'). In other words, the intron of the recombinant nucleic acid composition contains a 5'-donor splice site, an intronic insert site, a branch point motif, a poly-pyrimidine tract, and a 3'-acceptor splice site. Moreover, a branch point sequence is located between the 5'- and 3'-splice sites, containing homology to 5'-TACTWAY-3' (SEQ.ID.NO.6) motifs, such as 5'-TACTAAC-3' and 5'-TACTTAT-3'. In other words, the branch point motif includes or is homologous to the SEQ.ID.NO.6 sequence The adenosine "A" nucleotide of the branch-point sequence forms a part of (2'-5')-linked lariat intron RNA by cellular (2'-5')-oligoadenylate synthetases and spliceosomes in almost all spliceosomal introns. Furthermore, a poly-pyrimidine tract is closely located between the branch-point and 3'-splice site, containing a high T or C content sequence homologous to either 5'-(TY)m(C/-)(T)nS(C/-)-3' (SEQ.ID.NO.7) or 5'-(TC)nNCTAG(G/-)-3' (SEQ.ID.NO.8) motifs. The symbols of "m" and "n" indicate multiple repeats ≥ 1; most preferably, the m number is equal to 1∼3 and the n number is equal to 7∼12. The symbol "-" refers a nucleotide that can be skipped in the sequence. There are also some linker nucleotide sequences for the connection of all these synthetic intron components. Based on the guideline of 37 CFR 1.822 for symbols and format to be used for nucleotide and/or amino acid sequence data, the symbol W refers to an adenine (A) or thymine (T)/uracil (U), the symbol K refers to a guanine (G) or thymine (T)/uracil (U), the symbol S refers to a cytosine (C) or guanine (G), the symbol Y refers to a cytosine (C) or thymine (T)/uracil (U), the symbol R refers to an adenine (A) or guanine (G), and the symbol N refers to an adenine (A), cytosine (C), guanine (G) or thymine (T)/uracil (U)."

In another aspect, multiple transgenes and/or vectors expressing various intronic gene silencing effectors may be used to achieve gene silencing on multiple target genes. Alternatively, multiple gene silencing effectors may be generated from one intronic insert. In other words, the intronic insert site contains at least a gene silencing effector homologous to mir-302. For example, it has been reported that the ectopic expression of one anti-*EGFP* pre-miRNA-containing intron in zebrafish generates two different size miRNAs, namely miR-EGFP(282/300) and miR-EGFP(280-302), indicating that one insert of the *SpRNAi* may generate multiple gene-silencing effectors (Lin *et al*., 2005). In certain cases, intronic gene-silencing effectors can hybridize with a target gene transcript (*i*.*e*. mRNA) to form double-stranded siRNAs for triggering secondary RNA interference (RNAi) effects. Because these gene-silencing effectors are constantly produced from the transgene vector, it will alleviate the concerns of fast RNA degradation *in vivo.* The advantage of this strategy is in its stable delivery through the vector-based transgene transfection or viral infection, providing a reliable long-term gene silencing efficacy. Moreover, the present invention may produce RNAi-related gene silencing effectors, including miRNA, shRNA and siRNA, under the control of a specific RNA promoter selected from the group consisting of type-II RNA polymerase (Pol-II), viral polymerase, type-III RNA polymerase (Pol-III), and tetracycline responsive element-controlled RNA polymerase (TRE) promoters. The viral promoters are Pol-II-like RNA promoters isolated from cytomegalovirus (CMV), retrovirus long-terminal region (LTR), hepatitis B virus (HBV), adenovirus (AMV), and adeno-associated virus (AAV). For example, a lentiviral LTR promoter is sufficient to provide up to 5 x 10⁵ copies of pre-mRNA transcripts per cell. It is also feasible to insert a drug-sensitive repressor (*i*.*e*. *tTS*) in front of the viral polymerase promoter in order to control the transcription rate of the gene silencing effectors. The repressor can be inhibited by a chemical drug or antibiotics selected from the group of G418, tetracycline, doxycyclin, neomycin, ampicillin, kanamycin, and their derivatives, *etc.* In other words, the expression of the presently invented recombinant nucleic acid composition can be regulated by a drug like antibiotic derivatives, such as a tetracycline derivative. For example, doxycyclin is one of tetracycline derivatives.

In accordance with the present invention, the desired intronic RNA insert is excised and released by intracellular machineries and then triggers a desired gene silencing effect on specific gene targets with high complementarity to the RNA insert, while the exons of the host gene transcript are linked together to form mature mRNA for producing a desirable protein function in particular, a reporter or marker protein selected from the group of red/green fluorescent protein (RGFP/EGFP), embryonic gene marker, luciferase, lac-Z, and their derivatives. The presence of the reporter/marker protein is useful for identifying the levels and locations of the intronic gene silencing effectors expressed in the affected cells, facilitating confirmation of the resulting gene silencing effects. Alternatively, mature mRNA formed by the linkage of exons may be useful in conventional gene therapy to replace impaired or missing gene function, or to increase a specific gene expression. On the other hand, the gene silencing effectors (homologous to mir-302) may also include antisense RNA, ribozyme, short temporary RNA (stRNA), tiny non-coding RNA (tncRNA), Piwi-interacting RNA (piRNA), double-stranded RNA (dsRNA), siRNA, shRNA, miRNA, and their precursors (*i*.*e*. pri-/pre-miRNA). The use of these intronic gene silencing effectors is a powerful tool for silencing unwanted target genes selected from the group consisting of foreign genes, pathogenic transgenes, viral genes, mutant genes, oncogenes, disease-associated non-coding RNA genes and many other types of protein-coding as well as non-coding cellular genes.

Because the stem-loop structures of some native pre-miRNAs are too large and/or complicated to fit in the *SpRNAi-RGFP* transgene, the inventors often use a manually re-designed tRNA^{met} loop (*i*.*e*. 5'-(A/U)UCCAAGGGGG-3') (SEQ.ID.NO.43), to replace the native pre-miRNA loops. The tRNA^{met} loop has been shown to efficiently facilitate the export of manually re-designed miRNAs from nucleus to cytoplasm through the same Ran-GTP and Exportin-5 transporting mechanisms as native miRNAs do (Lin et al. (2005) Gene 356: 32-38). Advantageously, the present invention now uses a pair of manually improved pre-mir-302 loops, including 5'-GCTAAGCCAG GC-3' (SEQ.ID.NO.1) and 5'-GCCTGGCTTA GC-3' (SEQ.ID.NO.2), which provide the same nuclear export efficiency as the native pre-miRNAs but not interfere with the tRNA exportation. Also, this improvement enhances the formation of mir-302a-mir-302a* and mir-302c-mir-302c* duplexes, which may increase the overall function and stability of mir-302s. The design of these new pre-miRNA loops is modified by the combination of the tRNA^{met} loop and the short stem-loops of mir-302b/mir-302a, which are highly expressed in embryonic stem cells but not in other differentiated tissue cells. Thus, the use of these recombinant/man-made/artificial pre-miRNA/shRNA loops in mir-302s will not interfere with the native miRNA pathway in our body, resulting in a much less cytotoxicity and more safety.

The cluster of familial mir-302 pre-miRNAs is formed by hybridization and linkage/ligation of synthetic mir-302 homologues, consists of four parts: mir-302a, mir-302b, mir-302c and mir-302d pre-miRNAs in a 5' to 3' direction (Fig.3B). All of these manually re-designed mir-302 miRNA/shRNA molecules possess an identical 5'-end in their first 17 nucleotides [*e*.*g*. 5'-UAAGUGCUUC CAUGUUU-3' (SEQ.ID.NO.3)]. Synthetic oligonucleotides used for DNA recombination of the mir-302 pre-miRNA cluster are listed: including mir-302a-sense, 5'-GTCCGATCGT CCCACCACTT AAACGTGGAT GTACTTGCTT TGAAACTAAA GAAGTAAGTG CTTCCATGTT TTGGTGATGG ATCTCGAGCT C-3' (SEQ.ID.NO.29); mir-302a-antisense, 5'-GAGCTCGAGA TCCATCACCA AAACATGGAA GCACTTACTT CTTTAGTTTC AAAGCAAGTA CATCCACGTT TAAGTGGTGG GACGATCGGA C-3' (SEQ.ID.NO.30); mir-302b-sense, 5'-ATCTCGAGCT CGCTCCCTTC AACTTTAACA TGGAAGTGCT TTCTGTGACT TTGAAAGTAA GTGCTTCCAT GTTTTAGTAG GAGTCGCTAG CGCTA-3' (SEQ.ID.NO.31); mir-302b-antisense, 5'-TAGCGCTAGC GACTCCTACT AAAACATGGA AGCACTTACT TTCAAAGTCA CAGAAAGCAC TTCCATGTTA AAGTTGAAGG GAGCGAGCTC GAGAT-3' (SEQ.ID.NO.32); mir-302c-sense, 5'-CGCTAGCGCT ACCTTTGCTT TAACATGGAG GTACCTGCTG TGTGAAACAG AAGTAAGTGC TTCCATGTTT CAGTGGAGGC GTCTAGACAT-3' (SEQ.ID.NO.33); mir-302c-antisense, 5'-ATGTCTAGAC GCCTCCACTG AAACATGGAA GCACTTACTT CTGTTTCACA CAGCAGGTAC CTCCATGTTA AAGCAAAGGT AGCGCTAGCG-3' (SEQ.ID.NO.34); mir-302d-sense, 5'-CGTCTAGACA TAACACTCAA ACATGGAAGC ACTTAGCTAA GCCAGGCTAA GTGCTTCCAT GTTTGAGTGT TCGACGCGTC AT-3' (SEQ.ID.NO.35); and mir-302d-antisense, 5'-ATGACGCGTC GAACACTCAA ACATGGAAGC ACTTAGCCTG GCTTAGCTAA GTGCTTCCAT GTTTGAGTGT TATGTCTAGA CG-3' (SEQ.ID.NO.36) (Sigma-Genosys, St. Louis, MO). Alternatively, we may use the manually re-designed shRNA formed by the hybrid of synthetic miR-302s-sense, 5'-GTCCGATCGT CATAAGTGCT TCCATGTTTT AGTGTGCTAA GCCAGGCACA CTAAAACATG GAAGCACTTA TCGACGCGTC AT-3' (SEQ.ID.NO.27) and mir-302s-antisense, 5'-ATGACGCGTC GATAAGTGCT TCCATGTTTT AGTGTGCCTG GCTTAGCACA CTAAAACATG GAAGCACTTA TGACGATCGG AC-3' (SEQ.ID.NO.28), in place of the mir-302 pre-miRNA cluster for easy intronic insertion. In other words, one of the preferred gene silencing effectors is a recombinant nucleic acid sequence formed by the hybrid of SEQ.ID.NO.27 and SEQ.ID.NO.28. The mir-302 shRNA shares over 91% homology to all native mir-302 members and targets the same cellular genes in human.

For intronic insertion of the mir-302 pre-miRNA/shRNA, given that the insertion site of the recombinant *SpRNAi-RGFP* transgene is flanked with a *PvuI* and an *MluI* restriction/cloning site at its 5'- and 3'-ends, respectively, the primary insert can be easily removed and replaced by various pre-miRNA/shRNA inserts (*e*.*g*. mir-302 pre-miRNA/shRNA), which possess matched cohesive ends to the *PvuI* and an *MluI* restriction sites. By changing the intronic inserts directed against various gene transcripts, the present invention of the intronic mir-302s expression system can be used as a powerful tool for inducing targeted gene silencing *in vitro* and *in vivo.* For size confirmation and transgene purification, the mir-302-inserted *SpRNAi-RGFP* construct (10 ng) is amplified by polymerase chain reaction (PCR) with a pair of oligonucleotide primers [*i*.*e*. 5'-CTCGAGCATG GTGAGCGGCC TGCTGAA-3' (SEQ.ID.NO.23) and 5'-TCTAGAAGTT GGCCTTCTCG GGCAGGT-3' (SEQ.ID.NO.24)] for 25-30 cycles at 94°C, 52-57°C and then 68°C each for 1 min. The resulting PCR product (∼900-1100 bp) is fractionated on a 2% agarose gel, and then extracted and purified by a gel extraction kit (Qiagen, CA). After the DNA sequence is confirmed, the purified mir-302-inserted *SpRNAi-RGFP* transgene is further inserted into the restriction/cloning site (*i*.*e*. a *XhoI-ClaI* site) of a *pTet*-*On*-*tTS* vector to form a *pTet-On-tTS-mir302s* transgene expression vector for intracellular expression (Fig.3A).

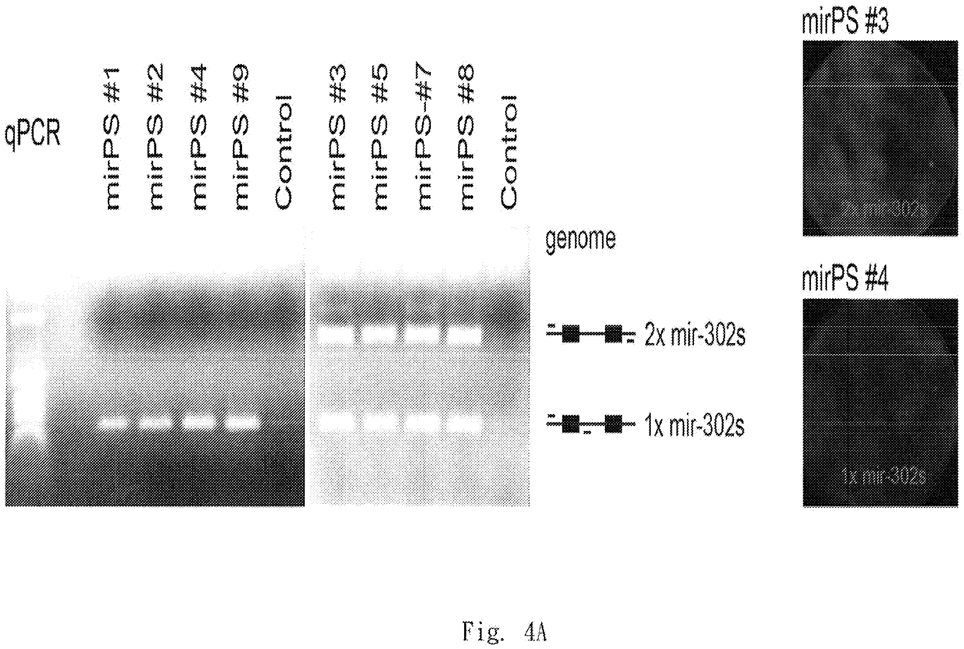

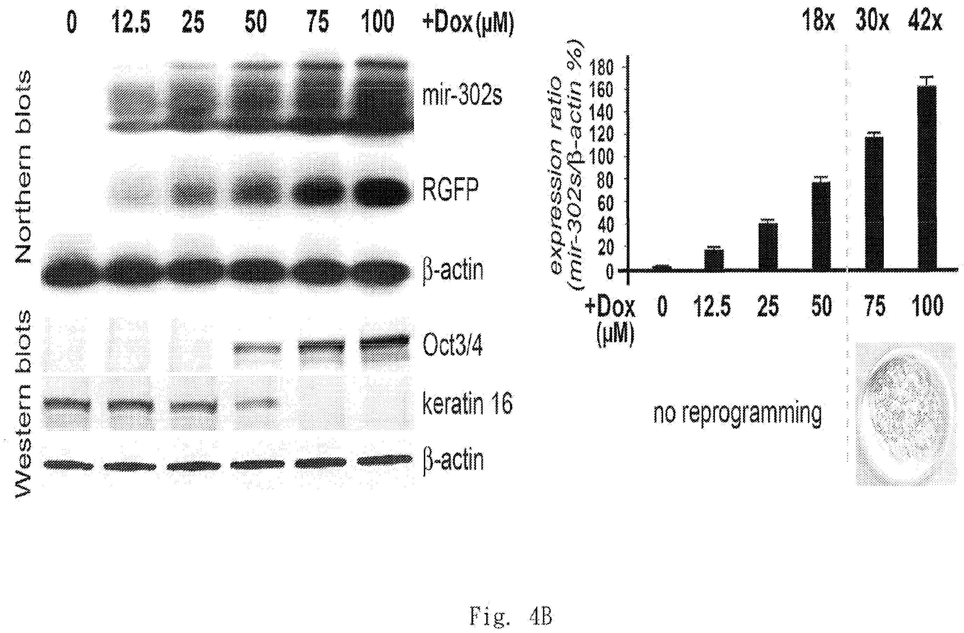

Delivery of the *pTet-On-tTS-mir302s* transgene vector into mammalian cells can be accomplished using a method selected from the group of liposomal/chemical transfection, electroporation, gene gun penetration, transposon/retrotransposon insertion, jumping gene integration, micro-injection, and retroviral/lentiviral infection. To prevent the risks of random transgene insertion and cell mutation, the inventors preferably use electroporation in conjunction with homologous recombination to deliver the *pTet-On-tTS-mir302s* transgene vector into the host cells of interest. For example, the *SpRNAi-RGFP* transgene is flanked with a 370-bp homologous region for recombinational insertion into the human chromosome 6 close to the 3'-proximity of the LOC727977 locus region, where encodes no known gene. The precise insertion of *SpRNAi-RGFP* in this single location has been detected (Fig.4A). Hence, the expression of the *SpRNAi-RGFP* transgene and its encoding mir-302s is totally dependent on the activation of the *TRE-CMV* promoter of the *pTet*-*On*-*tTS* vector, in the presence of Dox. The inventors have tested this novel approach of inducible mir-302s expression in both human normal and cancerous somatic cells, including normal epidermal skin cells, normal hair follicle cells, cancerous breast adenocarcinoma MCF7, prostate carcinoma PC3, and skin melanoma Colo cells. In other words, the mammalian cell to be reprogrammed into a pluripotent stem - like state is selected from the group consisting of a human cell, normal somatic cell, diseased somatic cell, a tumor or cancerous cell, a human hair follicle cell, a human skin cell, and a combination thereof. The mir-302-transduced pluripotent stem (mirPS) cells so obtained all carry only one or two concomitant copies of the *SpRNAi-RGFP* transgene in the same genome location (Fig.4A), indicating that the total mir-302 concentration may affect the survival of these mirPS cells. We have also observed that the expression levels of mir-302s and its marker RGFP mRNAs are concurrently increased corresponding to the elevation of Dox concentrations (Fig.4B). The overall mir-302 concentration must be expressed over 30 folds but less than 50 folds in order to trigger the somatic cell reprogramming into ES-like pluripotent stem cells. The currently available direct (exonic) siRNA/shRNA expression systems driven by a Pol-III or CMV promoter do not work due to their low expression rates in hair follicle and skin cells (merely ∼16-fold increase). This is probably because the template of the native mir-302 cluster is too short and structural to be directly transcribed by a Pol-III or CMV promoter. Therefore, the present invention provides an inducible mechanism by a defined drug (*i*.*e*. Dox) to control the level of mir-302s expression *in vitro* and *in vivo,* which serves as a second safeguard in addition to the intracellular surveillance systems. No cytotoxicity resulting from RNA accumulation or over-saturation is detected in the mirPS cells of the present invention.

The present invention has adopted a novel design and strategy of the inducible transgene expression system, namely *pTet-On-tTS-mir302s* as aforementioned, and uses it for transgenically expressing the members or homologues of the mir-302 family (mir-302s) in human somatic/cancer cells, thus to reprogram these somatic/cancer cells into an embryonic stem (ES)-like pluripotent state. In one preferred embodiment, the present invention provides a method for using a drug-inducible recombinant nucleic acid composition capable of being delivered, transcribed and processed into mir-302-like miRNA/shRNA molecules/homologues in human cells and thus inducing specific gene silencing effects on mir-302-targeted developmental and differentiation-associated genes in the cells, comprising the steps of: a) providing: i) a cell substrate expressing a plurality of developmental and differentiation-associated genes targeted by mir-302s, and ii) a recombinant nucleic acid composition capable of transcribing an isolated RNA encoding a plurality of non-coding mir-302 miRNAs/shRNAs or their homologues, which are in turn processed into mature mir-302 miRNAs/shRNAs or their homologues by intracellular mechanisms and thus able to suppress the functions of the target genes in the cell substrate; b) treating the cell substrate with the recombinant nucleic acid composition under conditions such that the target gene functions in the cell substrate are inhibited. Most preferably, the drug-inducible recombinant nucleic acid composition is a *Tet-On* vector containing the *SpRNAi-RGFP* transgene inserted with either a recombinant mir-302 family cluster (mir-302s; hybrid of SEQ.ID.NOs.29-36) or a manually re-designed mir-302 shRNA homologue (*i*.*e*. hybrid of SEQ.ID.NOs.27 and 28). The cell substrate may express the mir-302 miRNA/shRNA and its target genes either *in vitro, ex vivo* or *in vivo.* After that, the cell substrate is reprogrammed or transformed into an ES-like state, which not only presents standard ES cell markers, such as *Oct3*/*4*, *SSEA3, SSEA4, Sox2, Nanog,* and *LIN*-*28*, but also contains a highly demethylated genome similar to the zygotic genome undergoing stem cell reprogramming.

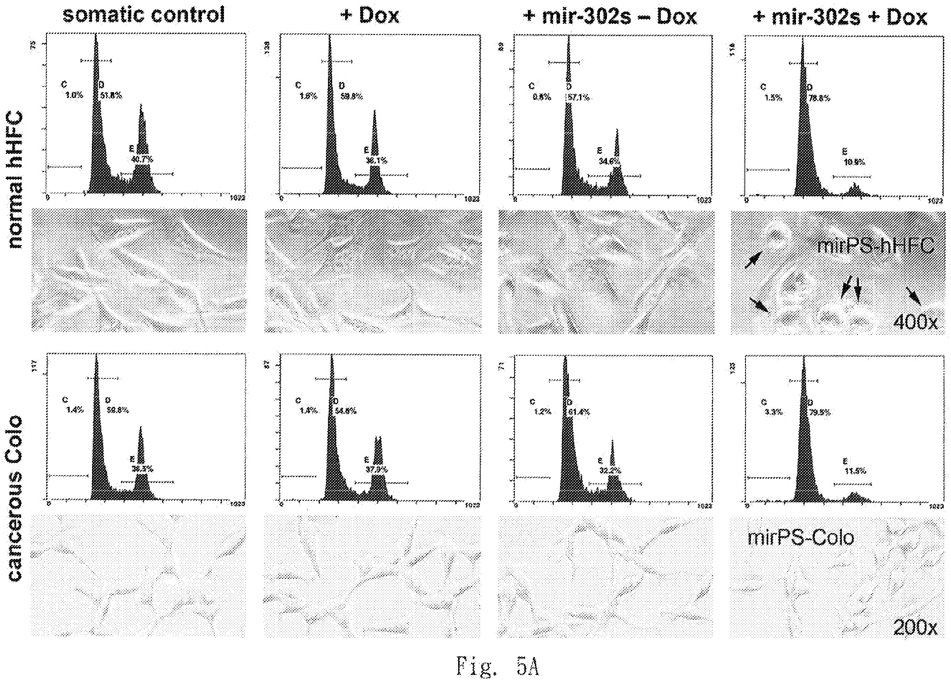

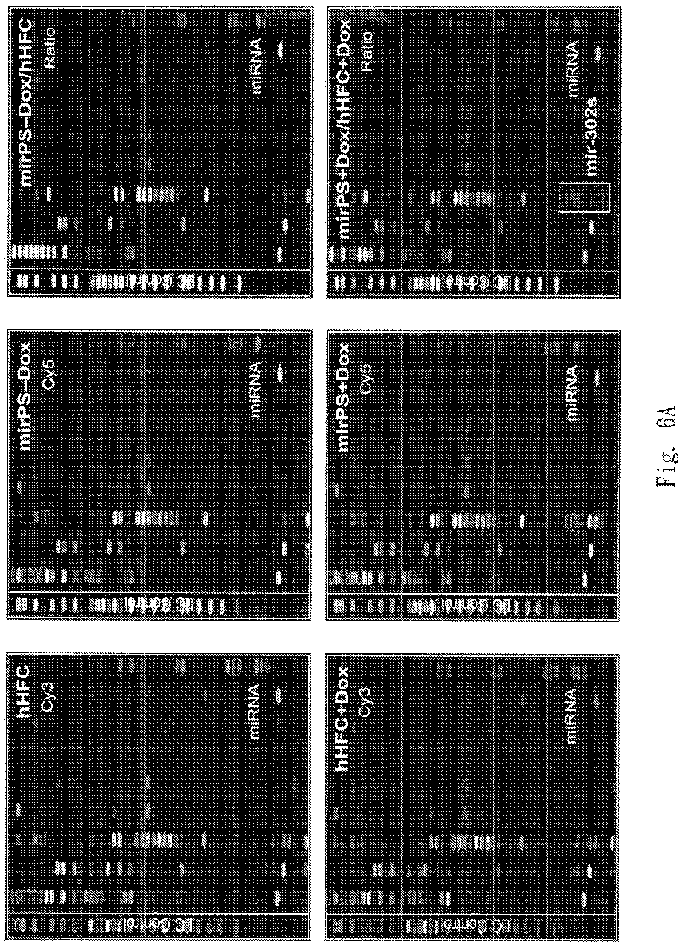

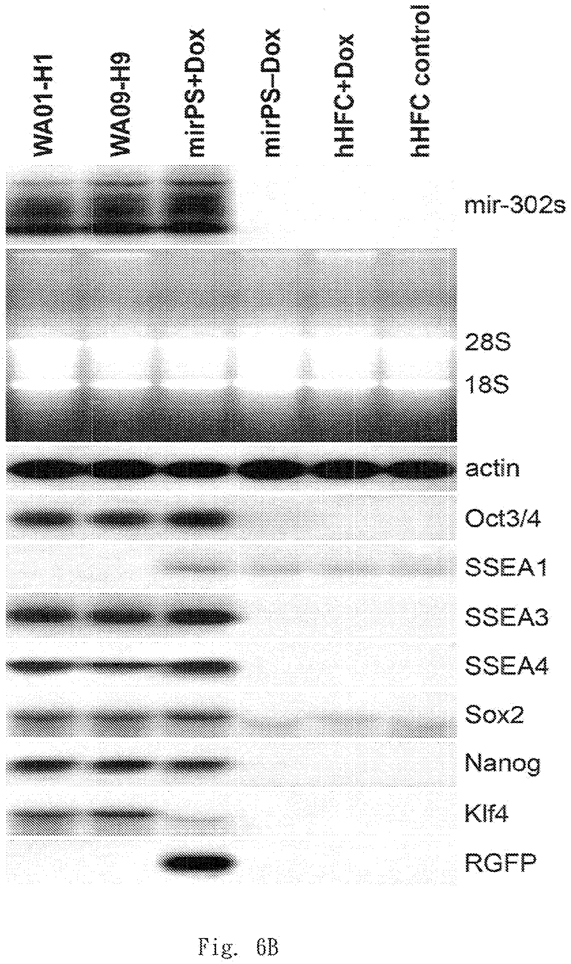

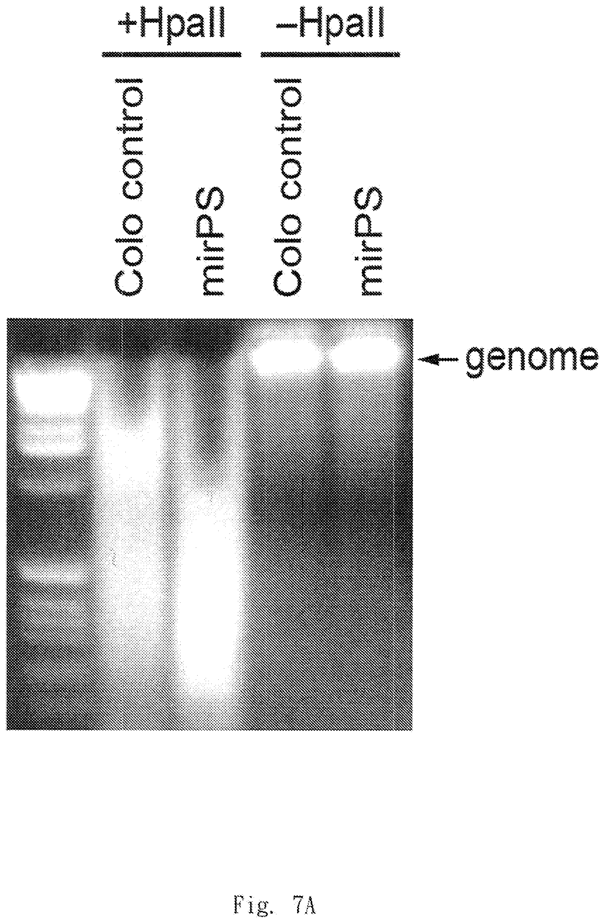

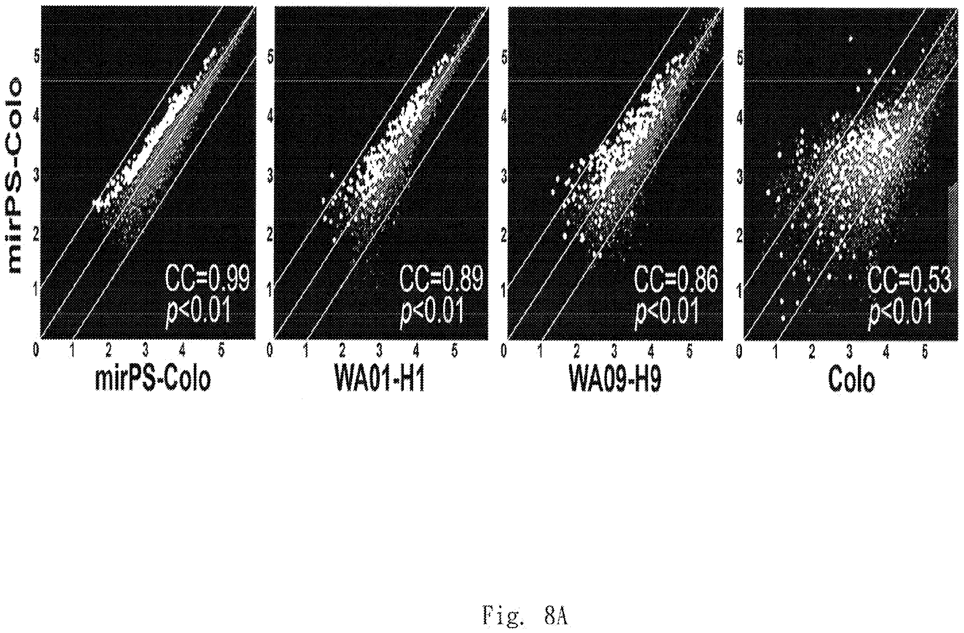

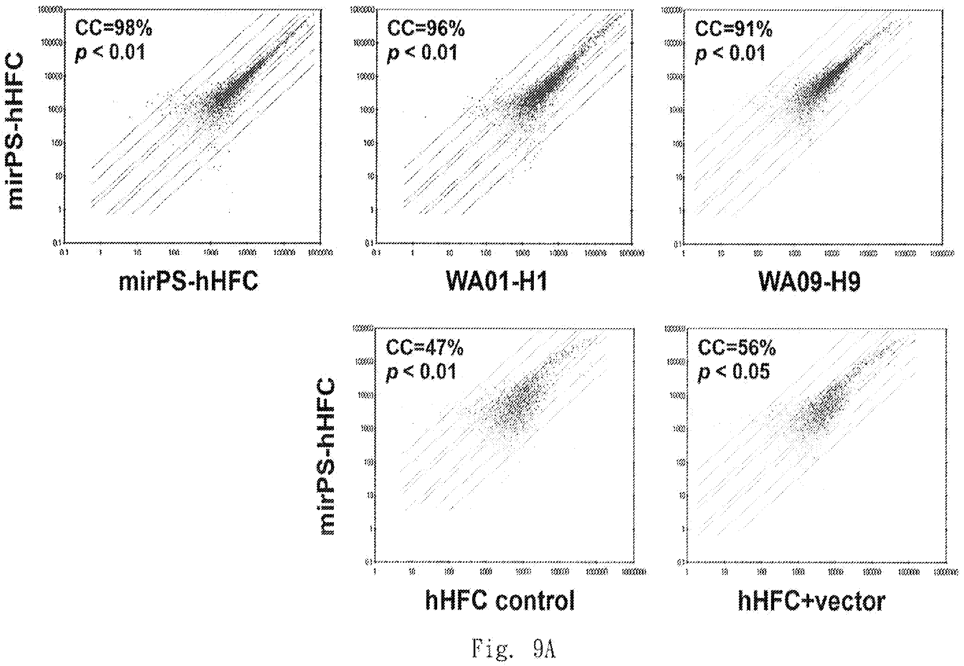

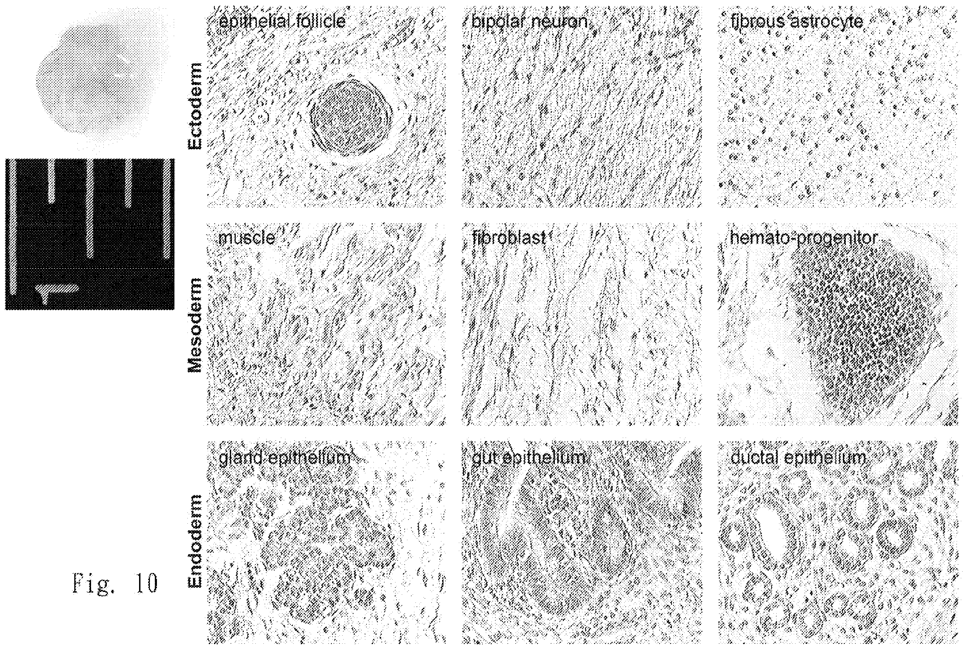

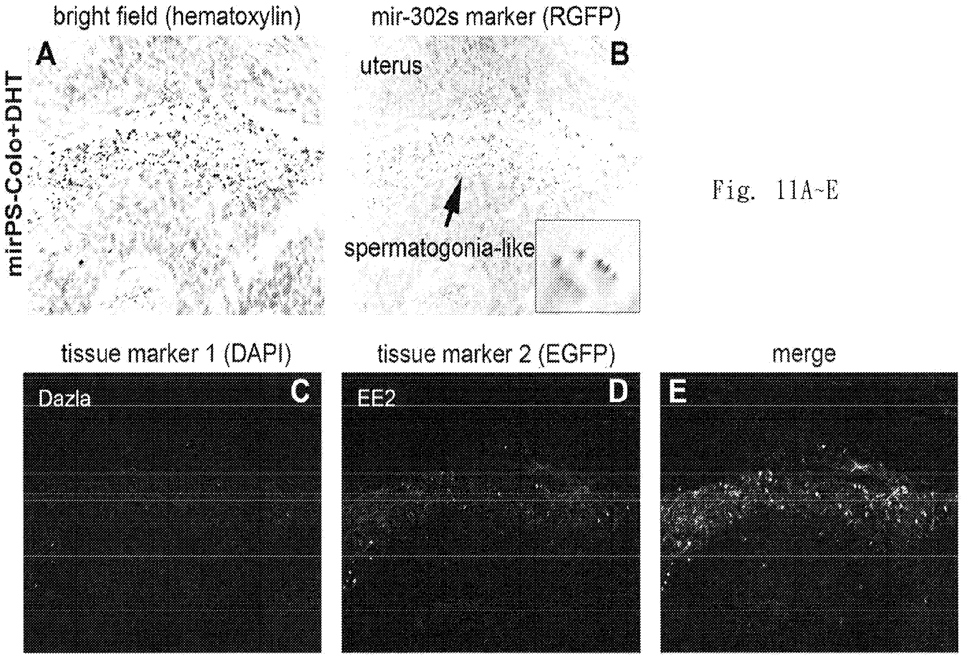

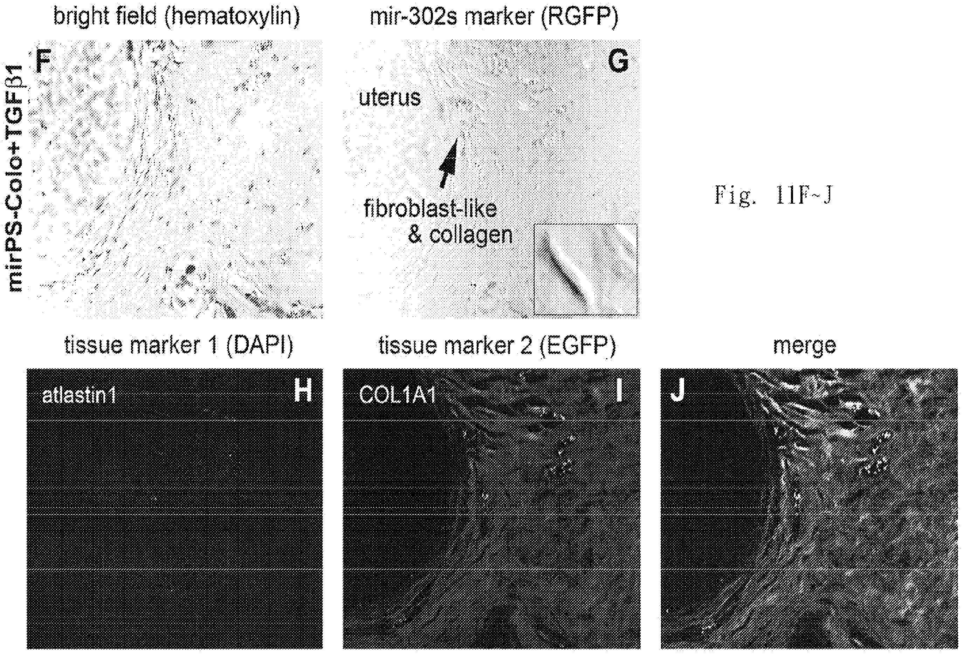

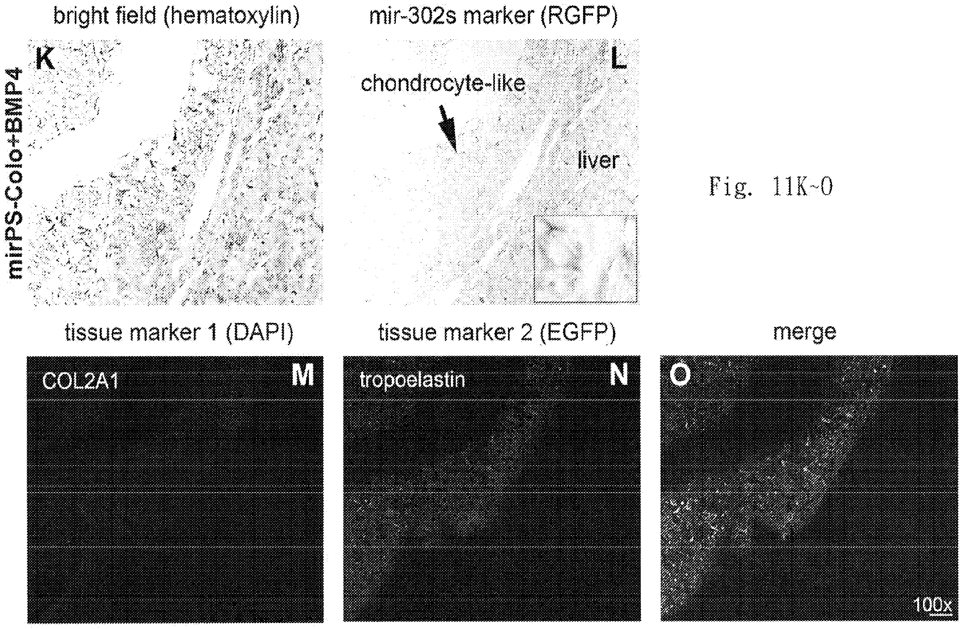

Using the present invention, the inventors have gathered evidence for the success of mir-302-induced pluripotent stem (mirPS) cell generation in seven areas: First, two sibling mirPS cell lines derived from the same cell type have been generated; one is from human normal hair follicle cells (hHFC) and the other from cancerous melanoma Colo cells, both of which can form embryoid bodies *in vitro* (Figs.5A-C). Second, the elevated expression of mir-302s has been confirmed using microRNA (miRNA) microarray and northern blot analyses of the mirPS transcriptomes (Figs.6A-B). Third, the elevated expression of standard embryonic stem (ES) cell markers has been detected, including Oct3/4, SSEA-3, SSEA-4, Sox2 and Nanog (Figs.6B, 8B-C and 9B). Fourth, global genomic DNA demethylation has been observed, similar to the status of a zygotic genome undergoing the reprogramming event (Figs.7A-C). Fifth, genome-wide gene expression patterns of these mirPS cells have been shown to share over 86% high similarity to those of human ES WA01 (H1) and WA09 (H9) cells (Figs.8A and 9A). Sixth, in vivo transplantation of the mirPS cell-derived embryoid bodies (EB) into immunocompromised SCID-beige mice can form teratoma-like tissue cysts containing all three embryonic germ layers (ectoderm, mesoderm and definitive endoderm) (Fig. 10). However, unlike teratomas, these tissue cysts form a very nice and clean boundary to their surrounding tissues. Also, the growth of these cysts in mice is halted approximately 2.5-week post-transplantation. It seems that there is a self-regulation mechanism limiting the random growth of these mirPS-derived EB cells *in vivo.* Last, the differentiation of mirPS cells can be guided to form various somatic and germ-line tissue cell types, such as neuronal progenitors, chondrocytes, fibroblasts and spermatogonia-like primordial cells, using treatments of various hormones and/or growth factors *in vitro* (Figs.11A-O). Furthermore, the inventors have successfully used electroporation-based transgene delivery to form these mir-302-induced mirPS cell lines, preventing the risks of retroviral infection and cell mutation (Figs.2A and 2B). These findings provide strong evidences for microRNA (miRNA)-induced stem cell generation in that the ectopic expression of mir-302s is able to not only reprogram both adult somatic and cancer cells into an ES-like pluripotent state but also maintain the renewal and pluripotency of these ES-like mirPS cells under a feeder-free cultural condition. Given that the function of mir-302s can reprogram both normal and cancerous tissue cells to ES-like pluripotent stem cells with a high 91%-93% success rate, the findings of this novel invention may provide beneficial applications in both stem cell and cancer therapies.

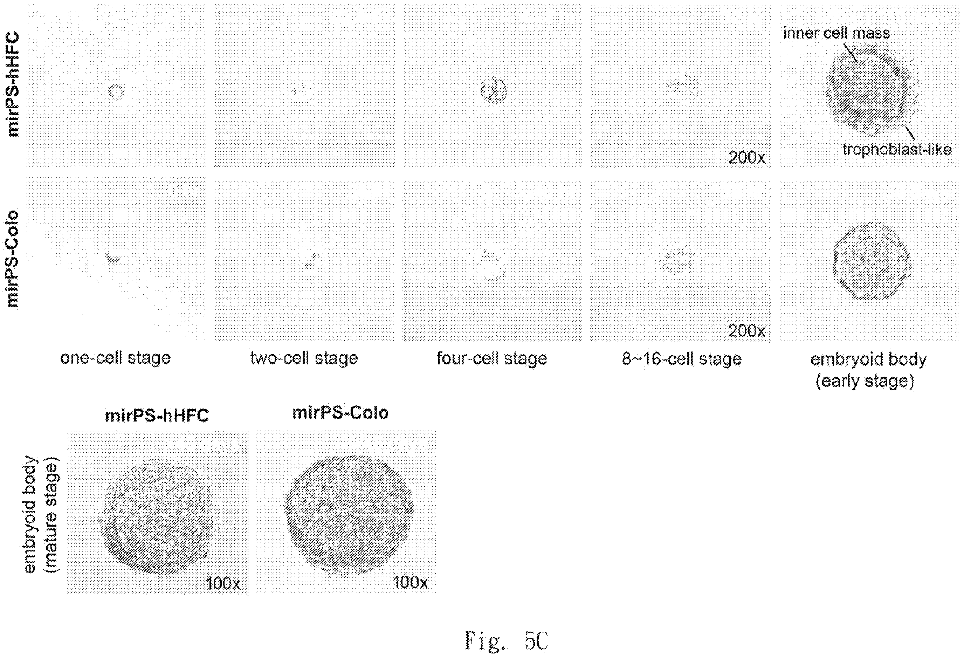

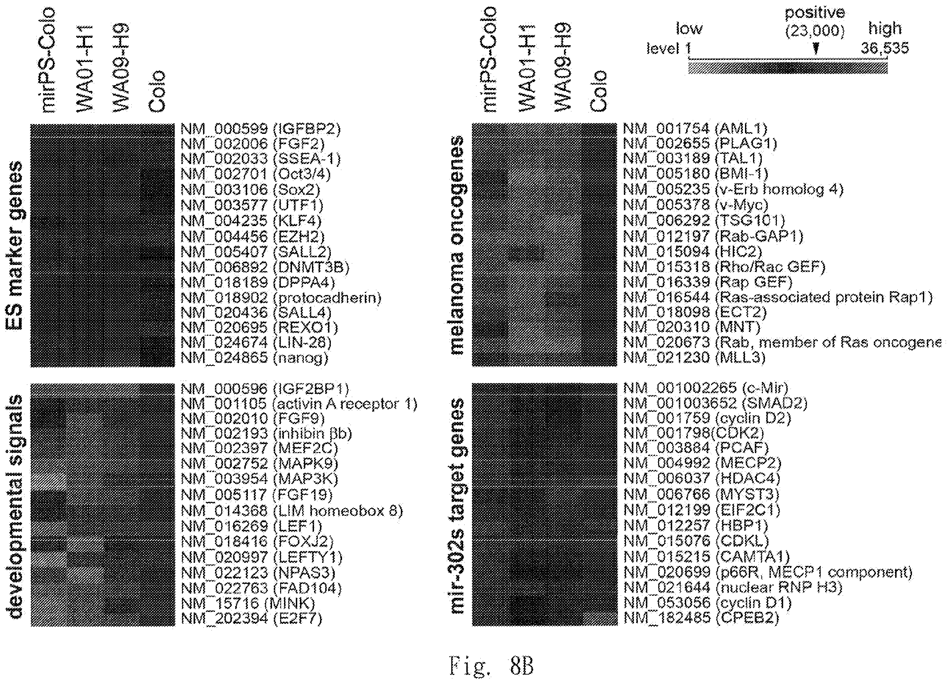

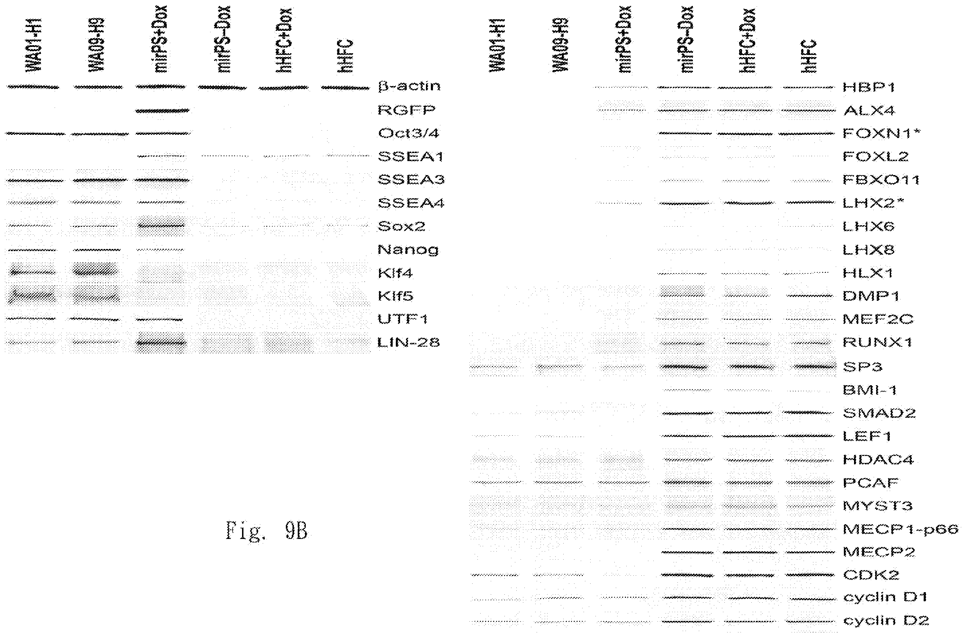

From the above findings, the inventors have also learned that mir-302 is able to not only significantly inhibit the expression of cyclin-dependent kinase 2 (CDK2), cyclin D1 and D2 to attenuate rates of cell proliferation and migration, but also suppress the MECP2 and MECP1-p66 activities to induce global demethylation of genomic DNAs (Figs.8B-C and 9B). It has been well known that cyclin E-dependent CDK2 is required for the entry of S-phase cell cycle and inhibition of CDK2 results in G1-phase checkpoint arrest, whereas cyclin D1 can override G1-phase arrest in response to DNA damage. Based on this principle, the inhibition of both CDK2 and cyclin D1 by mir-302 suggests a fact that mirPS cells have a very slow division rate. As shown in FigS.5A and 5C, the average cell cycle of mirPS cells is about 20-24 hours, much slower than those of their somatic/cancerous counterparts (approximately 4-6 hours per cell cycle). Tumor/cancer cells cannot live with a cell proliferation rate such slow. By controlling the cell cycle rate, mir-302 also affects cell survival. Therefore, the result of this cancer-stem cell cycle transition may provide a significant benefit in cancer therapy. In addition, the suppression of MECP2 and MECP1-p66 activities is consistent with the results of Figs.7A-C, which show the epigenetic reprogramming of malignant cancer cells into benign mirPS cells. It is conceivable that the mirPS cells so obtained from patients may further help to repair the tissue damages of cancers. By inhibiting the cellular genes essential for genomic imprinting and cell fate determination, mir-302s is able to not only reprogram differentiated somatic/cancer cells into an ES-like pluripotent state but also maintain this ES-like state under a feeder-free cultural condition. Furthermore, since the inhibition of CDK2, cyclin D1 and D2 can also attenuate the cell proliferation and migration rates of tumor/cancer cells, the role of mir-302s has been found to be a strong tumor suppressor against tumor cell growth and formation (Lin *et al*., 2008b). This tumor suppressor feature of mir-302s may help to generate tumor-free induced pluripotent stem cells for clinical transplantation, stem cell and cancer therapies. According to our present findings, the mechanism of mir-302-induced mirPS cell reprogramming is much safer, clearer and more understandable than that of the transcription factor-induced iPS cells.

In sum, our newly invented mir-302s expression system provides a safe and powerful tool for novel ES-like pluripotent stem cell generation, particularly derived from primary cultures of somatic hair follicle cells due to its easy access. Because the intronic miRNA pathway is tightly regulated by multiple intracellular surveillance systems, such as mRNA transcription, RNA splicing, exosomal processing and NMD, it is considered to be much more effective and safer than the conventional siRNA/shRNA pathway (Lin *et al*., 2008a). Advantageously, there are at least five breakthroughs in the present invention. First, one mir-302-expressing transgene can replace all four large transcription factor genes used in the previous iPS methods for generating more homogeneous ES-like pluripotent stem cells derived from just a few somatic cells of patients, improving the stem cell purity and compatibility to patients' immune systems. Second, because the total size of the mir-302-expressing transgene is relatively small (about 1 kilo-bases), the transgene delivery is extremely high (over a 91 % success rate) compared with the maximal 2% in the iPS methods. Third, the generation and culture of mirPS cells are performed completely under a feeder-free condition without the risk of feeder antigen contamination. Fourth, no oncogene is used, preventing the risks of cell mutation and tumor formation. Last, the inventors use electroporation in place of retroviral infection to deliver the single mir-302-expressing transgene, preventing the risk of random retroviral insertion into the host cell genome, which often causes insertional mutagenesis. In fact, mir-302 has been shown to be a strong tumor suppressor, which can even reprogram various tumor/cancer cells to ES-like pluripotent stem cells (the priority under U.S. Pat. Application No. 12/149,725 to Lin et al). Taken together, these advantages have solved the three major problems of the iPS methods, preventing the risks of retroviral infection, oncogenic mutation and uncertain tumorigenecity.

In addition to the general function of the present invention in generating ES-like pluripotent cell lines, the potential applications of the present invention further includes methods for maintenance of feeder-free and tumor-free ES cell cultural conditions, prevention of cancerous cell differentiation and transformation, isolation of pure or homogeneous stem cell populations, *in vitro* cloning and purification of adult stem cell lines, *in vitro* guidance of stem cell differentiation into pure somatic tissues, and development of transplantation and stem cell therapies using the ES-like pluripotent stem cells so obtained. The present invention can also be used as a tool for studying stem cell functions and mechanisms or providing a composition and method for altering the characteristics of a stem cell toward specific utilization. In other embodiments, the ES-like pluripotent stem cells of the present invention can be generated from the group of normal and cancerous somatic cells as well as adult stem cells of mammals, such as human, monkey, dog, cat, rat and maybe mouse.

### BRIEF DESCRIPTION OF THE DRAWINGS

Referring particularly to the drawings for the purpose of illustration only and not limitation, there is illustrated:
Figs.1A-B depict the mechanism of intronic miRNA biogenesis and its relative gene silencing effects. (A) Intronic miRNA is transcribed as a part of precursor messenger RNA (pre-mRNA), containing protein-coding exons and non-coding introns. The introns are spliced out of pre-mRNA and some of their secondary structures are further excised into small miRNA-like molecules capable of triggering targeted gene silencing, while the exons are ligated together to form a mature mRNA for marker protein synthesis. (B) Example of transgenic transfection of a pre-designed intronic miRNA into a *Tg(actin-GAL4: UAS-gfp)* strain zebrafish shows a strong gene silencing effect on targeted green EGFP expression (> 80% suppression, left lane 4), whereas other off-target inserts present no effects, including (from lanes 1 to 5) an empty intron without miRNA (1), an intron with a pre-miRNA insert against either *HIV-p24* (2) or *integrin β1* (3), and an intron with the anti-*EGFP* pre-miRNA insert but without a functional 5'-splice site (5). The anti-*EGFP* pre-miRNA is inserted in the 5'-proximal intron region of a red-shifted fluorescent marker *(RGFP)* gene. Northern blot analyses (right) reveal that mature miRNAs are generated only from the spliced products of the pre-miRNA-inserted *RGFP,* but not the empty *RGFP(-)* or the defective *RGFP*(*Δ*), indicating the requirement of RNA splicing in the intronic miRNA biogenesis.
Figs.2A and 2B depict the constructs and strategy of using a modified *Tet-On* vector containing the *SpRNAi-RGFP* transgene, namely *pTet*-*On*-*tTS*-*mir302s*, to express the mir-302s pre-miRNA cluster or shRNA. An electroporation-based transgene delivery approach is used to transfect the mir-302-expressing *SpRNAi-RGFP* transgene into targeted somatic/cancer cells.
Figs.3A-C show the reprogramming of human normal hair follicle hHFC and cancerous melanoma Colo cells into ES-like pluripotent stem (mirPS) cells, using electroporation-based transfection of a pre-designed *Tet-On* mir-302-expressing transgene. (A) Structure of the recombinant mir-302-expressing transgene, namely *SpRNAi-RGFP*, in a *Tet-On* inducible vector, namely *pTet-On-tTS-miR302s*. The *SpRNAi-RGFP* transgene is flanked with a 370 base-pair (bp) homologous region for recombinational insertion into a target site of the human cell genome. (B) Construct of the mir-302 pre-miRNA cluster (mir-302s), which is incorporated as a part of the intron of the *SpRNAi-RGFP* transgene. (C) Selection of positive mir-302-transduced mirPS cells using FACS flow cytometry sorting with antibodies against RGFP markers. The success rate of the presently invented transgene delivery is measured to be about 91%-93%.
Figs.4A-B show the integration of the *Tet-On* mir-302-expressing *SpRNAi-RGFP* transgene in the mirPS cell genome and resulting in the inducible expression of the mir-302 familial members (mir-302s) under the control of various doxycyclin (Dox) concentrations. (A) Quantitative PCR (qPCR; left) analyses of the genomic DNAs isolated from different mirPS cell lines, showing that all mirPS cells carry only one or two concomitant copies of the transgene, whereas no transgene is detected in the original hHFC and Colo cells (control). Fluorescent *in-situ* hybridization (FISH; right) assays further show that the transgene is inserted in a specific site of the human genome. Such restricted transgene insertion suggests that the concentration of total mir-302 expression may affect the survival of mirPS cells. (B) Northern blotting and bar chart demonstration of the inducible mir-302s expression corresponding to the Dox concentrations.
Figs.5A-C show the changes of reprogramming from human normal hair follicle (hHFC) and cancerous melanoma Colo cells to ES-like pluripotent stem (mirPS) cells. (A) Changes of morphologies and cell proliferation rates in the mir-302-transduced cells, namely mirPS-hHFC and mirPS-Colo. Both of an ES-like round cell shape and a much slower cell renewal rate are found in mir-302-transduced cells with doxycyclin (Dox) induction. (B) Formation of embryoid bodies (EB) derived from the mirPS cells and the guided differentiation into neuronal progenitor cells with positive Tuj1 and/or ABCA2 markers. (C) Time-course EB formation from a single mirPS cell after limiting dilution. The cell cycles are estimated to be approximately 20-24 hours.
Figs.6A-B show the correlation between mir-302s transfection and ES marker expression. (A) Microarray analyses of global miRNA expression, revealing that all mir-302 familial members (mir-302s) are highly expressed in the mirPS cells with Dox induction, but not the original somatic cells (n = 3, p < 0.01). (B) Northern blot and western blot analyses, showing that the mirPS cells with Dox induction express abundant ES cell markers, including Oct3/4 (Oct4), SSEA-3, SSEA-4, Sox2 and Nanog but less Klf4 (n = 4, *p* < 0.01), highly similar to those observed in human ES WA01-H1 and WA09-H9 cells.
Figs.7A-D show the patterns of genomic DNA demethylation in various mirPS cell lines. (A) HpaII cleavage showing the loss of global CpG methylation at a genome-wide scale in mirPS cells. (B) Bisulfite modification of unmethylated ACGT into AUGT sites in the 9,400-bp regulatory region of the Oct3/4 promoter, showing a significant increase of unmethylated ACTG (or AUCT) sites in all mirPS cells. (C) Bisulfite DNA sequencing, showing the detailed methylation maps flanking the initiation site of the Oct3/4 promoter. Black and white circles indicate the methylated and unmethylated cytosine sites, respectively. (D) Loss of ability to migrate in mirPS-PC3 cells compared with its original metastatic cancer PC3 cells.

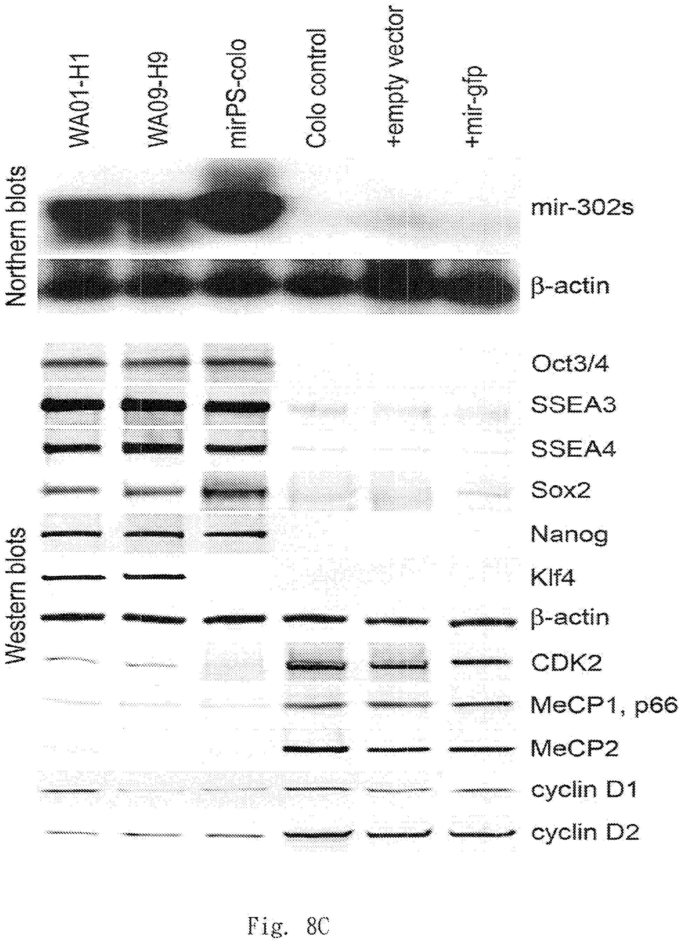

Figs.8A-B show genome-wide gene expression analyses among Colo, mirPS-Colo and human ES WA01-H1 (H1) and WA09-H9 (H9) cells. (A) Comparison of altered gene expression patterns using Human genome GeneChip U133A&B and plus 2.0 arrays (Affymetrix), showing high similarity between mirPS-Colo and H1 (89%) as well as H9 (86%), but not the cancerous Colo (53%) cells. White dots referred the highly variable genes as compared to the stably expressed genes (green dots). (B) Functional clustering of microarray-identified differentially expressed genes, demonstrating that a significant increase of ES cell markers and a marked decrease of melanoma oncogenes, developmental signals, and mir-302-targeted cell proliferation and DNA methylation genes are detected in mirPS cells, which highly resembled those in H1 and H9 cells (n = 4, p < 0.01). Fig.8C shows Northern blot and Western blot analyses, confirming the correlation among the expression patterns of mir-302s, human ES cell markers and predicted mir-302 target genes in mirPS-Colo cells, similar to those in human ES H1 and H9 cells except for Klf4 expression (n = 3, *p* < 0.01).
Figs.9A-B show genome-wide gene expression analyses among hHFC, mirPS-hHFC and human ES WA01-H1 (H1) and WA09-H9 (H9) cells. (A) Comparison of altered gene expression patterns using Human genome GeneChip U133 plus 2.0 arrays (Affymetrix), showing a high similarity between mirPS-hHFC and H1 (96%) as well as H9 (91%), but not the somatic hHFC (47%-56%) cells. (B) Western blot analyses, confirming the correlation among the expression patterns of mir-302s, human ES cell markers and predicted mir-302 target genes in mirPS-hHFC cells, similar to those in human ES H1 and H9 cells except for the expression of Klf4 and Klf5. The listed mir-302 target genes include seventeen transcriptional regulators, one histone deacetylase (HDA4), two methyl CpG-binding proteins (MECP1-p66 and MECP2), and three cell cycle checkpoint proteins (CDK2, cyclin D1 and D2).
Fig.10 shows teratoma-like primordial tissues derived from the mirPS transplants in the uterus or peritoneal cavity of female pseudopregnant immunocompromised SCID-beige mice (n/total = 6/6). These differentiated tissues included all three embryonic germ layers - ectoderm, mesoderm and endoderm, as determined by their distinct cell morphologies after hematoxylin and eosin (H & E) staining. Microscopic photographs were taken with the Nikon TE2000 system at 200x magnification.
Figs.11A-O show the guided pluripotency of mirPS cells. Treatments of DHT, TGF-β1 and BMP4, respectively from top to bottom, induce the mirPS cell differentiation into spermatogonia-like (A-E), fibroblast (F-J) and chondrocyte (K-O) tissue cells, in immunocompromised mice ex vivo. The immunocompromised nude mice serve as an in vivo environment mimicking transplantation therapy. Microscopic photographs shown from left to right indicate hematoxylin staining with differential interference contrast (A, F, K), bright field labeled with transgenic mir-302 marker RGFP (red) (B, G, L), immunostaining of the first tissue marker labeled with 4,6-diamidino-2-phenylindole (blue DAPI) (C, H, M), immunostaining of the second tissue marker labeled with fluorescein (green EGFP) (D, I, N), and merge of all three fluorescent markers (E, J, O). Small windows in the RGFP-bright fields show the morphologies of differentiated mirPS cells at high magnification (600 x).

### DETAILED DESCRIPTION OF THE INVENTION

Although specific embodiments of the present invention will now be described with reference to the drawings, it should be understood that such embodiments are by way of example only and merely illustrative of but a small number of the many possible specific embodiments which can represent applications of the principles of the present invention.

The present invention provides a novel nucleic acid composition and transgenic method for reprogramming the genetic and behavioral characteristics of mammalian somatic/cancer cells into an embryonic stem (ES)-like pluripotent state, using inducible recombinant microRNA (miRNA)-like small hairpin RNAs (shRNAs). In other words, the present invention provides a method for reprogramming at least a mammalian cell into at least a pluripotent stem-like cell. The method comprises several steps as followings: providing at least a cell substrate expressing a plurality of cellular genes targeted by mir-302; providing at least a recombinant nucleic acid composition capable of being delivered, transcribed and processed into at least a gene silencing effector homologous to mir-302 in the cell substrate; and treating the cell substrate with the recombinant nucleic acid composition under a condition that the cellular genes targeted by mir-302 are suppressed. Unlike previous shRNA designs, the presently invented shRNAs may contain a mismatched stem-arm similar to the precursors of native mir-302s (pre-mir-302s). Further, the presently invented shRNAs may also contain an improved pre-mir-302 stem-loops, such as 5'-GCTAAGCCAG GC-3' (SEQ.ID.NO.1) and 5'-GCCTGGCTTA GC-3' (SEQ.ID.NO.2), which can provide the same nuclear export efficiency as native pre-miRNAs but not interfere with the tRNA exportation. In other words, the gene silencing effector contains a sequence homologous to either SEQ.ID.NO.1 or SEQ.ID.NO.2. Without being bound by any particular theory, such reprogramming is directed to a newly discovered mir-302-mediated gene silencing mechanism, triggered by transfection of a recombinant transgene capable of expressing either a mir-302 family cluster (mir-302s) or a mir-302-homologous shRNA.

In a preferred embodiment, the design of the presently invented transgene expression is based on the pathway of natural intronic miRNA biogenesis (Fig.1A). The inventors have devised a novel nucleic acid composition to express a recombinant transgene encoding a red-shifted fluorescent protein *(RGFP),* namely *SpRNAi*-*RGFP*, which contains a man-made/artificial splicing-competent intron (*SpRNAi*) capable of producing intronic miRNA and/or shRNA-like gene silencing effectors through intracellular RNA splicing and processing mechanisms (Lin *et al*., 2003, 2006a,b). The protocols for design and construction of the *SpRNAi* intron and *SpRNAi-RGFP* transgene were described in Example 1. The *SpRNAi* is co-transcribed within the primary transcripts (pre-mRNA) of the *SpRNAi*-*RGFP* gene by mammalian type-II RNA polymerases (Pol-II) and cleaved out by RNA splicing/processing. Subsequently, the spliced *SpRNAi* is further processed into mature gene silencing effectors, such as native miRNAs and man-made shRNAs, so as to trigger specific posttranscriptional gene silencing (PTGS) effects on target genes. In this case, the present invention produces a recombinant mir-302s and/or an shRNA homologous to the mir-302s. Meanwhile, after intron splicing, the exons of the *SpRNAi-RGFP* gene transcript are linked together to form a mature *RGFP* mRNA for translation into a red fluorescent marker protein useful for identifying the desired miRNA/shRNA expression. Alternatively, some functional protein exons may be used in place of RGFP to provide additional gene functions, such as ES-cell marker genes *Oct4, Sox2, Nanog, LIN*-*28*, *SSEA3* and *SSEA4*.

In another preferred embodiment, the present invention provides a novel method for reprogramming mammalian somatic/cancer cells into ES-like pluripotent stem cells (Figs.2A and 2B), using an isolated drug-inducible nucleic acid composition capable of being delivered, transcribed and processed into mir-302-like miRNA/shRNA molecules/homologues in mammalian cells and thus inducing specific gene silencing effects on mir-302-targeted developmental and differentiation-associated genes in the cells, comprising the steps of: a) providing a cell substrate expressing a plurality of developmental and differentiation-associated genes targeted by mir-302s, and b) providing a recombinant nucleic acid composition capable of transcribing an isolated RNA encoding a plurality of non-coding mir-302 miRNAs/shRNAs or their homologues, which are in turn processed into mature mir-302 miRNAs/shRNAs or their homologues by intracellular mechanisms and thus able to suppress the functions of the target genes in the cell substrate; c) treating the cell substrate with the recombinant nucleic acid composition under conditions such that the target gene functions in the cell substrate are inhibited. Most preferably, the drug-inducible recombinant nucleic acid composition is a *Tet-On* vector containing the *SpRNAi-RGFP* transgene inserted with either a recombinant mir-302 family cluster (mir-302s; hybrid of SEQ.ID.NOs.29-36) or a manually re-designed mir-302 shRNA homologue (*i*.*e*. hybrid of SEQ.ID.NOs.27 and 28). In other words, the recombinant nucleic acid composition contains a drug-inducible gene expression vector. Besides, the recombinant nucleic acid composition may contain a gene expression vector selected from the group consisting of plasmid, viral vector, retrotransposon and a combination thereof. Furthermore, recombinant nucleic acid composition contains a *Tet-On* or *Tet-Off* gene expression vector. The cell substrate may express the mir-302 miRNA/shRNA and its target genes either *in vitro, ex vivo* or *in vivo.* The protocols for mir-302 miRNA/shRNA construction and transgene delivery were described in Examples 2 and 3.

The inventors take advantage of intracellular spliceosomal, exosomal and NMD systems to catalyze the release of intronic mir-302 miRNA/shRNA agents from the *SpRNAi-RGFP* transgene. Through a sequential DNA recombination of intracellular spliceosomal components on several snRNP-recognition sites of the *SpRNAi* [*e.g.* binding motifs required for snRNPs U1, U2 and U4/U6.U5 tri-snRNP including 5'-splice site (SEQ.ID.NO.4), branch-point motif (BrP; SEQ.ID.NO.6), poly-pyrimidine tract (PPT; SEQ.ID.NO.7 or 8), and 3'-splice site (SEQ.ID.NO.5)], the methods for assembly of synthetic snRNP-recognition elements into a *SpRNAi* intron and the incorporation of such an artificially recombinant *SpRNAi* into an isolated inducible *RGFP* gene to form the *SpRNAi-RGFP* transgene are described in Example 1 and Figs.2A and 2B, respectively. In addition, the *SpRNAi* further contains an intronic insert site located between the 5'-splice site and BrP motif for cloning and expressing a recombinant mir-302 miRNA/shRNA agent. The construction of a recombinant mir-302 family cluster (mir-302s) or a manually re-designed mir-302 shRNA homologue is described in Example 2 and Fig.3B. The transfection of the recombinant mir-302s miRNA/shRNA into the cells of interest and the selection of the positively transgenic cells are described in Example 3 and Fig.3C. In other words, the pluripotent stem-like cell is selectively isolated using mir-302 microRNAs or Oct3/4 as markers. The assays used for evaluating the reprogramming of mammalian somatic/cancer cells to ES-like pluripotent cells are described in Examples 4-12. The results of the assays are shown in Figs.4-11.

### Design and construction of an inducible SpRNAi-RGFP transgene expression system capable of expressing mir-302-like miRNA or shRNA.

The present invention uses an inducible *Tet-On*/*Off* intronic miRNA/shRNA expression system, namely *pTet-On-tTS-miR302s* (Fig.3A), for triggering the transgenic expression of mir-302-like gene silencing effectors under the control of doxycyclin induction and through the mechanism of intracellular intronic miRNA biogenesis (Fig.1A). The construction of *pTet-On-tTS-miR302s* is described in Examples 1 and 2. After transfected into the cells of interest, the *pTet-On-tTS-miR302s* expression vector transcribes a TRE-Pol-II-driven recombinant transgene, namely *SpRNAi-RGFP*, which contains an artificial splicing-competent intron (*SpRNAi*) capable of producing intronic gene silencing effectors (Figs.3A and 3B), such as hairpin-like miRNA and shRNA. Incorporation of *SpRNAi* into a red-shifted fluorescent protein gene *(RGFP)* is genetically engineered by sequential ligation of several synthetic DNA sequences as shown in Example 1. *SpRNAi* comprises a precursor miRNA or shRNA insert (site), which can be released by intracellular RNA splicing and processing mechanisms, such as components of spliceosomes, exosomes and NMD systems, and then triggers an intronic RNA-mediated gene silencing mechanism. Other RNA transcripts capable of being used to carry and generate *SpRNAi* include hnRNA, mRNA, rRNA, tRNA, snoRNA, snRNA, smnRNA, viral RNA, pre-microRNA and their precursors as well as derivatives.

As shown in Example 1, *SpRNAi* was synthesized and incorporated into an intron-free red-shifted fluorescent protein gene (*RGFP* or *rGFP*) to form a *SpRNAi-RGFP* transgene, which was mutated from the HcRedl chromoproteins of *Heteractis crispa*. Because the inserted *SpRNAi* disrupted the functional fluorescent protein structure of RGFP, we were able to determine the intron removal and *RGFP*-mRNA maturation through the reappearance of red fluorescent light emission at the 570-nm wavelength in a successfully transfected cell or organism (Fig.1B). Construction of this recombinant *SpRNAi-RGFP* transgene was based on the natural structures of a spliceosomal intron in a precursor messenger RNA (pre-mRNA). The major components of *SpRNAi* include several snRNP recognition sites and linkers, such as 5'- and 3'-splice sites in the ends for precise cleavage, a branch point motif (BrP) for splicing recognition, a poly-pyrimidine tract (PPT) for spliceosomal interaction, linkers for connection of each of these components and some restriction sites for desired intronic insertion. Structurally listed from the 5' to 3' direction, the presently invented *SpRNAi* contains a 5'-splice site, an intronic insert homologous to mir-302-like gene silencing effectors, a branch point motif (BrP), a poly-pyrimidine tract (PPT), and a 3'-splice site. In addition, some translational termination codons (T codon) may be located in the linker sequences close to the 3'-splice site of *SpRNAi*.

Generically, the 5'- splice site is a nucleotide sequence containing or homologous to either 5'-GTAAGAGK-3' (SEQ.ID.NO.4) or GU(A/G)AGU motifs (such as 5'-GTAAGAGGAT-3' (SEQ.ID.NO.37), 5'-GTAAGAGT-3', 5'-GTAGAGT-3' and 5'-GTAAGT-3'), while the 3'- splice site is a nucleotide sequence containing or homologous to either GWKSCYRCAG (SEQ.ID.NO.5) or CT(A/G)A(C/T)NG motifs (such as 5'-GATATCCTGC AG-3'(SEQ.ID.NO.42), 5'-GGCTGCAG-3' and 5'-CCACAG-3'). Moreover, a branch point sequence is located between the 5'- and 3'-splice sites, containing homology to 5'-TACTWAY-3' (SEQ.ID.NO.6) motifs, such as 5'-TACTAAC-3' and 5'-TACTTAT-3'. The adenosine "A" nucleotide of the branch-point sequence forms a part of (2'-5')-linked lariat intron RNA by cellular (2'-5')-oligoadenylate synthetases and spliceosomes in almost all spliceosomal introns. Furthermore, a poly-pyrimidine tract is closely located between the branch-point and 3'-splice site, containing a high T or C content oligonucleotide sequence homologous to either 5'-(TY)m(C/-)(T)nS(C/-)-3' (SEQ.ID.NO.7) or 5'-(TC)nNCTAG(G/-)-3' (SEQ.ID.NO.8) motifs. The symbols of "m" and "n" indicate multiple repeats ≥ 1; most preferably, the m number is equal to 1∼3 and the n number is equal to 7∼12. The symbol "-" refers a nucleotide that can be skipped in the sequence. There are also some linker nucleotide sequences for the connection of all these intron components. Based on the guideline of 37 CFR 1.822 for symbols and format to be used for nucleotide and/or amino acid sequence data, the symbol W refers to an adenine (A) or thymine (T)/uracil (U), the symbol K refers to a guanine (G) or thymine (T)/uracil (U), the symbol S refers to a cytosine (C) or guanine (G), the symbol Y refers to a cytosine (C) or thymine (T)/uracil (U), the symbol R refers to an adenine (A) or guanine (G), and the symbol N refers to an adenine (A), cytosine (C), guanine (G) or thymine (T)/uracil (U)." For all of the above spliceosomal recognition components, the deoxythymidine (T) nucleotide is replaceable with uridine (U).

To test the function of a spliced *SpRNAi* insert, various gene silencing effector constructs can be cloned into the intronic insertion site of the recombinant *SpRNAi-RGFP* transgene. The intronic insertion site contains multiple restriction and cloning sites, which may be recognized by restriction enzymes selected from the group of *AatII, AccI*, *AfIII*/*III, AgeI*, *ApaI*/*LI*, *AseI, Asp718I*, *BamHI*, *BbeI, BclI*/*II, BglII, BsmI, Bsp120I*, *BspHI*/*LU111*/*120I, BsrI*/*BI*/*GI, BssHII*/*SI*, *BstBI*/*U1*/*XI, ClaI, Csp6I, DpnI, DraI*/*II*. *EagI, Ecl136II*, *EcoRI*/*RII*/*47III*, *EheI, FspI, HaeIII*, *HhaI, HinPI, HindIII, HinfI HpaI*/*II, KasI*, *KpnI*, *MaeII*/*III*, *MfeI, MluI, MscI, MseI, NaeI, NarI, NcoI, NdeI, NgoMI*, *NotI, NruI, NsiI, PmlI*, *Ppu10I, PstI, PvuI*/*II, RsaI, SacI*/*II*, *SalI, Sau3AI, SmaI, SnaBI, SphI, Sspl, StuI*, *Tail, TaqI*, *XbaI, XhoI, XmaI* endonuclease and a combination thereof. These intronic inserts are DNA templates that can be transcribed as highly secondary structures selected from the group consisting of lariat-form RNA, short-temporary RNA (stRNA), antisense RNA, small-interfering RNA (siRNA), double-stranded RNA (dsRNA), short-hairpin RNA (shRNA), microRNA (miRNA), Piwi-interacting RNA (piRNA), ribozyme, and their precursors as well as derivatives in either sense or antisense conformation, or both, and a combination thereof.

For the convenience of transgene delivery into the cell or organism of interest, the *SpRNAi-RGFP* transgene of the present invention is preferably incorporated into an expression-competent vector, selected from the group consisting of DNA transgene, plasmid, retrotransposon, transposon, jumping gene, viral vector, and a combination thereof. Such a transgene expression vector so obtained is preferably introduced into the cell or organism with a highly efficient gene delivery method selected from the group consisting of chemical/liposomal transfection, electroporation, transposon-mediated DNA recombination, jumping gene insertion, viral infection, micro-injection, gene-gun penetration, and a combination thereof. In other words, the recombinant nucleic acid composition is introduced into said mammalian cell by a gene delivery method selected from the group consisting of liposomal transfection, chemical transfection, transgenic DNA recombination, viral infection, transposon insertion, jumping gene insertion, micro-injection, electroporation, gene-gun penetration, and a combination thereof The vector may further contain at least a viral, Pol-II, or Pol-III promoter, or a combination thereof, for expressing the *SpRNAi-RGFP* transgene. Most preferably, the transgene is incorporated into a *Tet-On*/*Off* vector and transgenically delivered into target cells with electroporation, as shown in Example 3. Moreover, the vector may contain a Kozak consensus translation initiation site to increase translation efficiency in eukaryotic cells, multiple SV40 polyadenylation signals downstream of the *SpRNAi-RGFP* transgene, a pUC origin of replication for propagation in prokaryotic cells, at least two restriction sites for incorporation of the *SpRNAi-RGFP* construct in the vector, an optional SV40 origin for replication in mammalian cells that express the SV40 T antigen, and an optional SV40 early promoter for expressing an antibiotic resistance gene in replication-competent prokaryotic cells. In other words, the recombinant nucleic acid composition is selected from the group consisting of a tetracycline responsive element, a viral or type-II RNA polymerase (Pol-II) promoter, or both, a Kozak consensus translation initiation site, polyadenylation signals, a plurality of restriction/cloning sites and a combination thereof. Furthermore, the recombinant nucleic acid composition is selected from the group consisting of a pUC origin of replication, a SV40 early promoter for expressing at least an antibiotic resistance gene in replication-competent prokaryotic cells, an optional SV40 origin for replication in mammalian cells and a combination thereof. The expression of antibiotic resistance genes is used to serve as a selective marker for isolating positive clones with the transgene expression. The antibiotics are selected from the group consisted of penicillin G, ampicillin, neomycin, paromycin, kanamycin, streptomycin, erythromycin, spectromycin, phophomycin, tetracycline, rifapicin, amphotericin B, gentamycin, chloramphenicol, cephalothin, tylosin, and a combination thereof.

The strategy of intronic miRNA/shRNA expression using the *SpRNAi-RGFP* transgene has been tested in a Tg(*actin*-GAL4:USA-gfp) strain zebrafish *in vivo* to target against its green *EGFP* gene expression. As shown in Example 6 and Fig.1B, the liposomal transfection of a *SpRNAi-RGFP* plasmid expressing an artificially recombinant anti-*EGFP* pre-miRNA insert (lane 4) displayed a very strong gene silencing effect on *EGFP* (>80% gene knockdown), whereas no other silencing effect was observed in those inserts indicated by lanes from left to right: 1, blank vector control (Ctl); 2, pre-miRNA insert targeting *HIV-p24* (mock); 3, antisense *EGFP* insert without the hairpin loop structure (anti); and 5, reverse pre-miRNA sequence which is completely complementary to the anti-*EGFP* pre-miRNA (miR*). Also, no effect was detected on off-target genes, such as marker RGFP and housekeeping β-actin, suggesting that such intronic miRNA-mediated gene silencing is highly target-specific. Furthermore, by northern blot analysis (Fig.1B, right), we have observed the generation of small intronic gene silencing effectors derived only from the designed *SpRNAi-RGFP* gene transcript (middle lane), but not from a natural transcript of the intron-free *RGFP* (left lane) or a transcript of a defective *SpRNAi-RGFP* construct without the functional 5'-splice site (right lane), while spliced *RGFP* exons were linked together to form mature RNA for translating the marker red fluorescent protein.

### Design and construction of a recombinant mir-302 familial cluster and a mir-302-like shRNA homologue

Because the hairpin-loop structures of some native pre-miRNAs are too large and/or complicated to fit in the *SpRNAi-RGFP* transgene, the inventors have designed a modified tRNA^{met} loop (*i*.*e*. 5'-(A/U)UCCAAGGGGG-3')(SEQ.ID.NO.43), to replace the native pre-miRNA loops. The tRNA^{met} loop has been shown to efficiently facilitate the export of manually re-designed miRNAs from nucleus to cytoplasm through the same Ran-GTP and Exportin-5 transporting mechanisms as native miRNAs do (Lin *et al*., 2005). Advantageously, the present invention now uses a pair of manually improved pre-mir-302 loops, including 5'-GCTAAGCCAG GC-3' (SEQ.ID.NO.1) and 5'-GCCTGGCTTA GC-3' (SEQ.ID.NO.2), which provide the same nuclear export efficiency as the native pre-miRNAs but not interfere with the tRNA exportation. Also, this improvement enhances the formation of mir-302a-mir-302a* and mir-302c-mir-302c* duplexes, which may stabilize the overall function of mir-302s. The design of these new pre-miRNA loops is modified by the combination of the tRNA^{met} loop and the short stem-loops of mir-302b/mir-302a, which are highly expressed in embryonic stem cells but not in other differentiated tissue cells. Thus, the use of these man-made/artificial pre-miRNA loops in mir-302s will not interfere with the native miRNA pathway in our body, resulting in a much less toxicity and more safety.

The mature sequences of mir-302a, mir-302b, mir-302c and mir-302d are 5'-UAAGUGCUUC CAUGUUUUGG UGA-3' (SEQ.ID.NO.10), 5'-UAAGUGCUUC CAUGUUUUAG UAG-3' (SEQ.ID.NO.11), 5'-UAAGUGCUUC CAUGUUUCAG UGG-3' (SEQ.ID.NO.12), and 5'-UAAGUGCUUC CAUGUUUGAG UGU-3' (SEQ.ID.NO.13), respectively. These mir-302 familial gene-silencing effector share a highly conserved 5'-end region in their first seventeenth nucleotides (100% homology), identical to 5'-UAAGUGCUUC CAUGUUU-3' (SEQ.ID.NO.3). In other words, the gene silencing effector contains a sequence homology or complementarity, or both, to SEQ.ID.NO.3. In design of sequences homologous to these mir-302 sequences, thymine (T) can be used in place of uracil (U).

As described in Example 2, the cluster of familial mir-302 pre-miRNAs was formed by hybridization and linkage/ligation of synthetic mir-302 homologues, consists of four parts: mir-302a, mir-302b, mir-302c and mir-302d pre-miRNAs in a 5' to 3' direction (Fig.3B). All of these manually re-designed mir-302 miRNA/shRNA homologues possessed an identical 5'-end in their first 17 nucleotides [e.g. 5'-UAAGUGCUUC CAUGUUU-3' (SEQ.ID.NO.3)]. Alternatively, we could use a manually re-designed mir-302 shRNA in place of the mir-302 pre-miRNA cluster for easy intronic insertion. The re-designed mir-302 shRNA was formed by hybridization of synthetic miR-302s-sense, 5'-GTCCGATCGT CATAAGTGCT TCCATGTTTT AGTGTGCTAA GCCAGGCACA CTAAAACATG GAAGCACTTA TCGACGCGTC AT-3' (SEQ.ID.NO.27) and mir-302s-antisense, 5'-ATGACGCGTC GATAAGTGCT TCCATGTTTT AGTGTGCCTG GCTTAGCACA CTAAAACATG GAAGCACTTA TGACGATCGG AC-3' (SEQ.ID.NO.28). This re-designed mir-302 shRNA shared over 91% homology to all native mir-302 members and targeted the same mir-302-targeted genes in human.

### Delivery of the inducible mir-302-expressing SpRNAi-RGFP transgene into primary cultures of human normal hair follicle (hHFC) and cancerous melanoma Colo cells

Given that the intronic insertion site of the recombinant *SpRNAi-RGFP* transgene is flanked with a *PvuI* and an *MluI* restriction/cloning site at its 5'- and 3'-ends, respectively, the primary insert can be easily removed and replaced by various pre-miRNA/shRNA inserts (*e*.*g*. mir-302 pre-miRNA/shRNA), which possess matched cohesive ends to the *PvuI* and an *MluI* restriction sites. By changing the intronic inserts directed against various gene transcripts, the intronic miRNA/shRNA expression system can be used as a powerful tool for inducing targeted gene silencing *in vitro* and *in vivo.* In experiments, the mir-302 pre-miRNA/shRNA was first inserted in the *SpRNAi-RGFP* transgene and then the transgene was incorporated into the cloning site (*i*.*e*. a *XhoI-ClaI* site) of the *pTet*-*On*-*tTS* vector, so as to form a *pTet-On-tTS-miR302s* transgene expression vector (Fig.3A). After that, the *pTet-On-tTS-mir302s* vector (10-30 µg) was mixed with the host cells (200-2000) in a hypoosmolar PH buffer (400 µl; Eppendorf) and electroporation was performed at 400-450 V for 100 µsec to deliver the transgene into the host cell genomes. Positively transgenic cells were isolated and collected 72 hours later, using FACS flow cytometry selection with both anti-RGFP and anti-Oct3/4 monoclonal antibodies (Fig.3C). The success rate of this novel mir-302s transgene approach was measured to be over 91%. Because the *SpRNAi-RGFP* transgene was flanked with a homologous region for recombinational insertion into a specific genome site containing no genes (Fig.4A), the expression of its encoding mir-302 miRNA/shRNA effector was totally dependent on the Dox-induced activation of the *TRE-CMV* promoter of the *pTet*-*On*-*tTS* vector. The *pTet*-*On*-*tTS* vector has contained a *CMV*-driven *tTS* inhibitor gene to inactivate the *TRE-CMV* promoter of the transgene. In the presence of doxycyclin (Dox), the function of tTS was inhibited by Dox and thus the *SpRNAi-RGFP* transgene and its encoding mir-302s were expressed (Fig.4B).

### Reprogramming of human normal and cancerous somatic cells into an ES-like state under a feeder-free cultural condition

As described in Examples 1-2 and Figs.3A-3B, we have designed and constructed an inducible *SpRNAi-RGFP* transgene encoding either a manually linked mir-302a-mir-302b-mir-302c-mir-302d (mir-302s) pre-miRNA or a re-designed mir-302-like shRNA [e.g. a hairpin-like sequence containing 5'-UAAGUGCUUC CAUGUUUUAG UGU-3' (SEQ.ID.NO.9)], and then tested it for silencing the developmental and differentiation-related target genes in various somatic and cancer cells, such as human normal hair follicle cells (hHFC) and cancerous melanoma Colo cells.

Following the procedures shown in Figs.2A and 2B, the inventors transgenically delivered the recombinant mir-302s pre-miRNA into hHFC cells and the re-designed mir-302 shRNA homologue (SEQ.ID.NO.9) into Colo cells, respectively. In other words, the gene silencing effector is a recombinant hairpin-like RNA including a sequence homologous to SEQ.ID.NO.9. After ectopic mir-302 expression induced by Dox, all these mir-302-tranduced/induced pluripotent stem (mirPS) cell lines transformed their morphologies (lower panels) from spindle to round, indicating that they may not only lose ability to migrate but also have a very slow cell renewal rate similar to the growth of ES cells (Fig.5A). Flow cytometry analyses (upper panels; Example 7) comparing DNA content to cell cycle stages further showed an over 67% reduction in the mitotic cell population of mirPS cells, suggesting that the cell proliferation rates were much slower than those of their somatic/cancerous origins. In average, they divided every 20-24 hours under a feeder-free cultural condition, containing either DMEM/F12 or RPMI 1640/B27 medium supplemented with 10% charcoal-stripped FBS, 4 mM L-glutamine, 1 mM sodium pyruvate, 5 ng/ml activin, 5 ng/ml noggin, 3 ng/ml bFGF and an equal mixture of 0.5 µM Y-27632 and 0.5 µM GSK-3 inhibitor XV, namely mirPS cell cultural medium, at 37°C under 5% CO₂. In other words, the pluripotent stem-like cells can be cultured feeder-free under either DMEM/F12 or RPMI 1640/B27 medium supplemented with 10% charcoal-stripped FBS, 4 mM L-glutamine, 1 mM sodium pyruvate, 5 ng/ml activin, 3 ng/ml bFGF, and an equal mixture of 0.5 µM Y-27632 and 0.5 µM GSK-3 inhibitor XV. The first (left) and second (right) peaks of the flow cytometry charts represented the levels of resting G0/G1 and mitotic M phase cell populations in the entire tested cell population. The mitotic cell population (M phase) was decreased from 41% to 11% in hHFC and from 36% to 11% in Colo cells after the mir-302s transfection, whereas there was no significant change in either cell morphology or cell proliferation rate after transfection with an empty *SpRNAi-RGFP* vector and Dox (+Dox) or a mir-302-expressing *SpRNAi-RGFP* vector without Dox (+mir-302s-Dox). Based on these findings, we demonstrate that ectopic mir-302s expression is able to reprogram both normal and cancerous human somatic cells into an ES-like cell morphology and rate of cell division.

### Formation of embryoid bodies derived from various mirPS cells

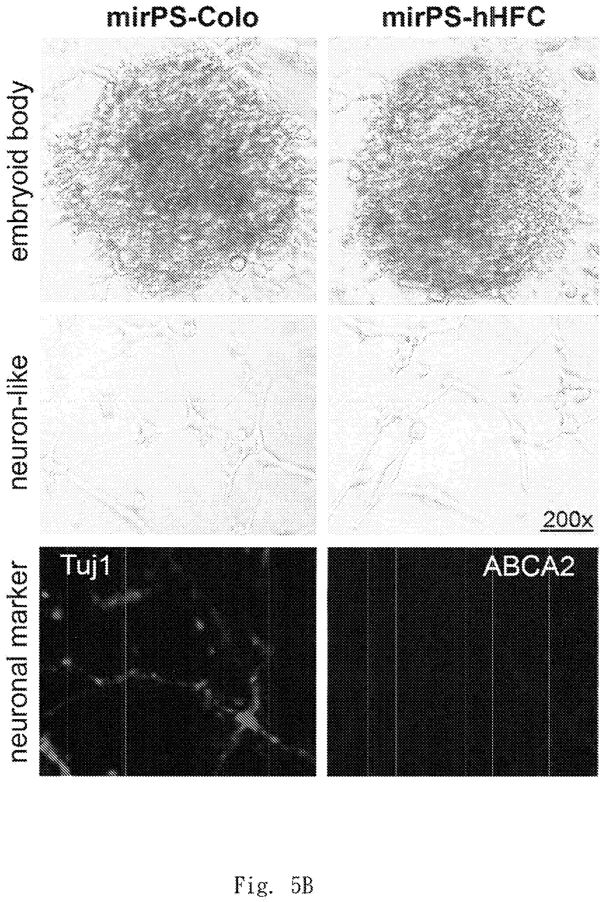

All the mirPS cells were able to form compact colonies reminiscent of embryoid bodies (EB) derived from human embryonic stem (ES) cells (Fig.5B). In other words, it proves that the pluripotent stem-like cells reprogrammed by the present invention can form embryoid bodies. When dissociated with a mixture of trypsin-EDTA and collagenase IV and then cultivated in RPMI 1640 medium supplemented with only 10% FBS, these EB-like cells differentiated into neuronal progenitor cells, many of which expressed neuronal markers Tuj1 and/or ABCA2. After limiting dilution and further culture in the feeder-free DMEM/F12 medium supplemented with 10% charcoal-stripped FBS, 4 mM L-glutamine, 1 mM sodium pyruvate, 5 ng/ml activin, 3 ng/ml bFGF and an equal mixture of 0.5 µM Y-27632 and 0.5 µM GSK-3 inhibitor XV, each mirPS cell could continue to form a pure EB for sub-culturing and/or transplantation/implantation assays (Fig.5C). In view of these ES-like stem cell properties, we then examined the expression of mir-302s and ES cell markers in these mirPS cells as well as compared with human ES WA01-H1 and WA09-H9 cells, cited for reference only.

### MicroRNA (miRNA) microarray analyses of mir-302s expression in mirPS cells

To confirm the transgenic mir-302 expression in the mirPS cells, we performed microRNA (miRNA) microarray analyses described in Example 9. As shown in Fig.6A, miRNA microarray analyses showed that the expression rates of all mir-302 members (most down right, white square circle) were markedly increased in the mirPS-hHFC cells after Dox treatment (100 µM) compared with the original somatic cells (control). The expression levels of mir-302s in mirPS cells were proportionally corresponding to the concentrations of Dox induction (Fig.4B), as determined by northern blotting. The same result has also been seen in mirPS-Colo cells (Lin *et al*., 2008b). At the early EB stage, small RNAs from each cell line were isolated using a *mir*Vana™ miRNA isolation kit (Ambion, Inc., Austin, TX). The purity and quantity of the isolated small RNAs were assessed using 3.5% formaldehyde-agarose gel electrophoresis and spectrophotometer measurement (Bio-Rad, Hercules, CA), and then immediately submitted to LC Sciences (San Diego, CA) for microarray analysis. In the Cy3 and Cy5 intensity images (blue background), as signal intensity increases from level 1 to level 65,535 the corresponding color changes from blue to green, to yellow, and to red. In the Cy5/Cy3 ratio image (black background), when Cy3 level is higher than Cy5 level the color is green; when Cy3 level is equal to Cy5 level the color is yellow; and when Cy5 level is higher than Cy3 level the color is red. Because the mature RNA sequences between native mir-302 familial members and our manually re-designed mir-302 pre-miRNA/shRNA agents share very high homology (>91%), this result indicates that the re-designed mir-302 agents can replace the function of native mir-302s.

Based on this result, we also found that the elevation of mir-302 expression may further increase the expression of some other miRNAs, such as mir-92, mir-93, mir-200c, mir-367, mir-371, mir-372, mir-373, mir-374, and the whole mir-520 familial members. Analyses of the predicted target genes of these miRNAs, using the "TARGETSCAN" (http://www.targetscan.org/vert_42/) and "PICTAR-VERT" (http://pictar.bio.nyu.edu/cgi-bin/PicTar_vertebrate.cgi?) programs linked to the Sanger miRBase::Sequences website (http://microrna.sanger.ac.uk/), demonstrated that mir-302s share over 400 target genes with these miRNAs, suggesting that these miRNAs may also play important roles in maintaining stem cell pluripotency and renewal. These conserved target genes include, but not limited, members of RAB/RAS-related oncogenes, ECT-related oncogenes, pleiomorphic adenoma genes, E2F transcription factors, cyclin D binding Myb-like transcription factors, HMG-box transcription factors, Sp3 transcription factors, transcription factor CP2-like proteins, NFkB activating protein genes, cyclin-dependent kinases (CDKs), MAPK-related kinases, SNF-related kinases, myosin light chain kinases, TNF-alpha-induce protein genes, DAZ-associated protein genes, LIM-associated homeobox genes, DEAD/H box protein genes, forkhead box protein genes, BMP regulators, Rho/Rac guanine nucleotide exchange factors, IGF receptors, endothelin receptors, left-right determination factors, cyclins, p53 inducible nuclear protein genes, RB-like 1, RB binding protein genes, Max-binding protein genes, c-MIR cellular modulator of immune recognition, Bcl2-like apoptosis facilitator, protocadherins, integrin β4/β8, inhibin, ankyrins, SENP1, NUFIP2, FGF9/19, SMAD2, CXCR4, EIF2C, PCAF, MECP2, histone acetyltransferase MYST3, nuclear RNP H3, and many nuclear receptors and factors. Most of these genes are highly involved in embryonic development and/or tumorigenecity of tumors/cancers.

### Identification of standard human ES marker expression, i.e. Oct3/4, SSEA-3, SSEA-4

### Sox2 and Nanog

As shown in Figs.6B and 9B, the mirPS cells strongly expressed many standard human ES cell markers, such as Oct3/4, SSEA-3, SSEA-4, Sox2 and Nanog, whereas none of these markers were detected in the original somatic cells (hHFC control) and the somatic cells transfected with an empty *SpRNAi-RGFP* vector and doxycyclin (hHFC+Dox) or a mir-302s vector without doxycyclin (mirPS-Dox). The expression patterns of these ES markers were very similar to those of the human ES WA01-H1 and WA09-H9 cells, cited for reference only, as determined by both northern and western blot analyses at the mRNA and protein levels. These results indicate that ectopic expression of mir-302s is able to reprogram adult somatic/cancer cells to ES-like pluripotent stem cells, which present many standard human ES markers. The same result has also been observed in mirPS-Colo cells (Fig.8C).

Oct3/4 (also termed Oct-3 or Oct-4) is one of the POU transcription factors, which is mainly and highly expressed in totipotent embryonic stem and germ cells (Scholer et al., (1989) EMBO J. 8: 2543-2550; Rosner et al., (1990) Nature 345: 686-692). A critical level of Oct3/4 expression is required to maintain stem cell self-renewal and pluripotency. Down-regulation of Oct3/4 results in differentiation of embryonic stem cells into divergent developmental programs. SSEA proteins, SSEA-1, -3 and -4, are originally identified by monoclonal antibodies recognizing lacto- and globo-glycolipids on the surface of pre-implantation-stage murine embryos and teratocarcinoma stem cells, but not on their differentiated derivatives (Solter et al., (1978) Proc. Natl. Acad. Sci. USA 75: 5565-5569). Undifferentiated primate embryonic stem (ES) cells, human embryonic cancer (EC) and ES cells all express SSEA-3 and SSEA-4, but not SSEA-1 (Thomson et al., (1998) Science 282: 1145-1147). SSEA-3 and SSEA-4 are synthesized during oogenesis and mainly presented in the membranes of oocytes, zygotes and early cleavage-stage embryos (Shevinsky et al., (1982) Cell 30: 697-705). Sox2 functions as a core transcription factor in maintaining pluripotency, but this function is not specific to embryonic stem cells (Boyer et al., (2005) Cell 122: 947-956). Therefore, based on such understanding, the mirPS cells likely present all characteristics of these human ES markers.

### Assessment of genomic DNA demethylation (reprogramming)

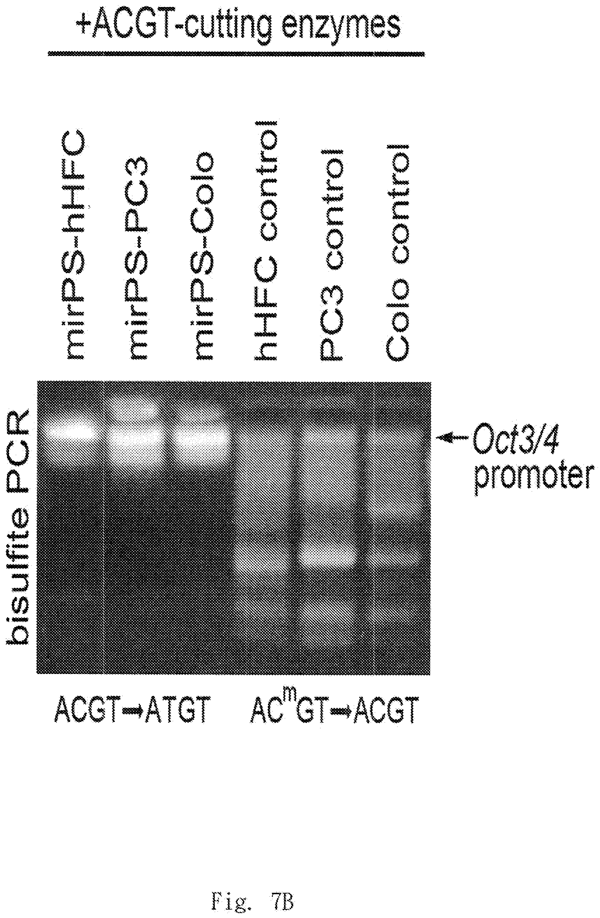

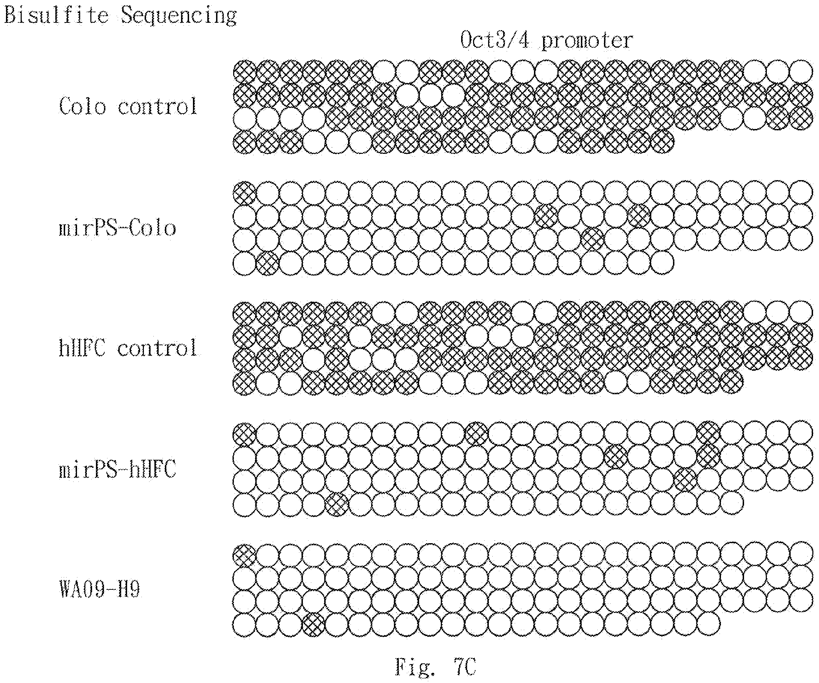

Change of epigenetic modification underlines another unique feature of ES cells in particular, genomic demethylation (Hochedlinger et al., (2006) Nature 441: 1061-1067). In order to reprogram a cell into its ES state, many embryonic genes need to be re-activated by DNA demethylation, such as *Oct3*/*4.* To assess this epigenetic effect in the mirPS cells, we first performed whole genome digestion with *HpaII*, a restriction enzyme that was sensitive to CpG methylation and cleaved only an unmethylated CCGG rather than a methylated CCGG site. Fig.7A showed that the digested DNA fragments from somatic cell controls were over twice lager than those from the mirPS cells, indicating that the overall mirPS cell genome is highly demethylated. Further assessment in the *Oct3*/*4* gene promoter region was performed using bisulfite-genomic PCR and sequencing (Takahashi and Yamanaka, 2006). Bisulfite converted all unmethylated cytosine to uracil. Because unmethylated ACGT sites were changed into AUGT sites, the digestion of ACGT-cutting restriction enzymes failed to cleave these isolated regions in the mirPS cell genomes (Fig.7B). The detailed demethylation maps shown by the bisulfite DNA sequencing further demonstrated that over 90% methylation sties of the *Oct3*/*4* gene promoter region were lost in the mirPS cells resembling those occurring in the human ES WA09-H9 cells (Fig.7C), suggesting that a genome-wide reprogramming event did occur to re-activate the *Oct3*/*4* gene expression. The above CpG demethylation assays are shown in Example 8.

In experiments to determine the demethylation sites in *Oct3*/*4* gene promoter, we first treated the isolated genomic DNAs with bisulfite (CpGenome DNA modification kit, Chemicon, CA), which converted all unmethylated cytosine to uracil, and then isolated the *Oct3*/*4* 5'-upstream promoter region using polymerase chain reaction (long template PCR extension kit, Roche, IN). After that, the PCR products were collected and digested with an equal mixture (5U each) of multiple ACGT-cutting restriction enzymes, containing *AclI* (AACGTT), *BmgBI* (CACGTC), *PmlI* (CACGTG), *SnaBI* (TACGTA) and *HpyCH4IV* (ACGT). Because the unmethylated ACGT sites in this region were changed into AUGT sites by bisulfite, which could not be cleaved with the ACGT-cutting restriction enzymes, the result of Fig.7B indicated that over four methylated ACGT sites in the control hHFC, PC3 and Colo cells were changed to demethylated sites in the corresponding mirPS cells. This mir-302-mediated demethylation of the *Oct3*/*4* gene promoter may therefore contribute to the re-activation of *Oct3*/*4* gene expression in the mirPS cells.

### Lost of ability to migrate in the mirPS cells derived from metastatic cancers

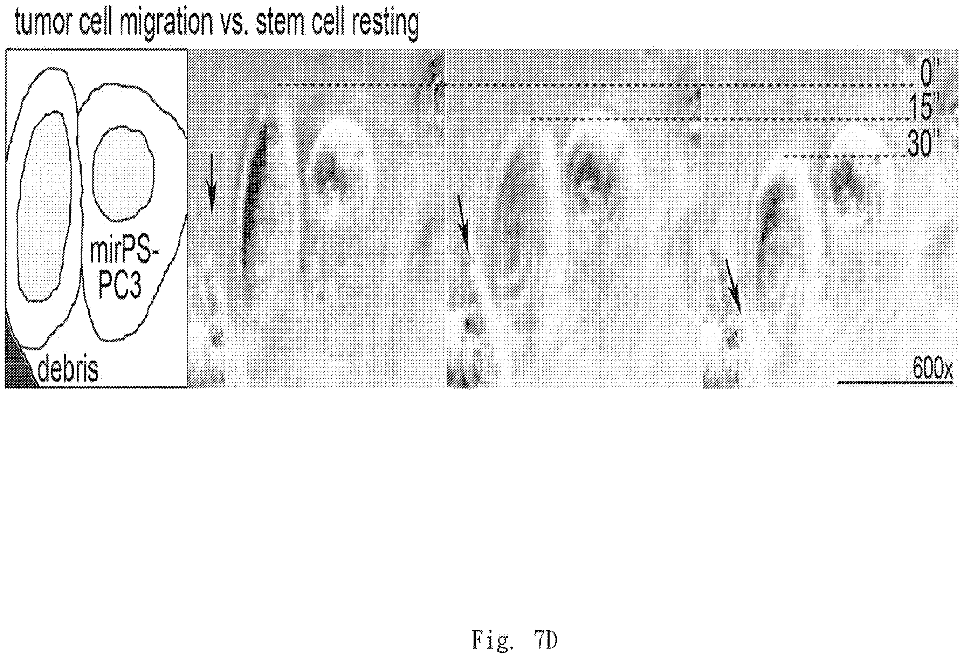

Human ES cells do not migrate. Lost of cell migration is often observed in the mirPS cells derived from fast metastatic cancer cell lines, such as mirPS-PC3 cells. Given that ES cells tend to rest in one place and form embryoid bodies *in situ*, this may explain why the metastatic human prostatic carcinoma PC3 cells lost their ability to migrate after ectopic mir-302 transfection. Alternatively, mir-302 may silence certain cell migration-associated genes, such as genes of microtubule-associated protein 1B (*MAPIB*), actin-like protein (*ACTL6A*), ankyrin 2 (*ANK2*), amyloid β precursor A4 (*APP*), myosin light polypeptide kinase (*MYLK*), to prevent the normal cell migration and cancer cell invasion. As shown in Fig.7D and Example 12, metastatic PC3 cells quickly migrated over time, whereas mirPS-PC3 cells remained stationary. No morphological change was observed in all other controls. Thus, the presently invented transgenic mir-302s expression is sufficient to transform human cancer cells into a more ES-like cell morphology and rate of cell division, suggesting a very beneficial use in cancer therapy. This result suggests a potential therapeutical application for delivering the mir-302s into cancer/tumor cells, which may not only reprogram the malignant cancer/tumor cells into useful ES-like stem cells but also reduce the chance of cancer metastasis. More advantageously, since these mirPS cells are generated from patients' own cells and thus are immune-compatible to the patients, a novel transplantation therapy can be developed using these mirPS cells to repair the cancer/tumor-damaged tissues without the risk of immune rejection.

### Identification of global ES marker expression using gene microarray analysis

Genome-wide gene profiling may provide insights into the genetic alterations involved in the mir-302-mediated reprogramming event. After the co-expression of standard ES cell markers and transgenic mir-302s is confirmed, we further performed human genome microarray analysis to screen changes in genome-wide gene expression patterns in the cells before and after the ectopic mir-302 expression as well as between the mirPS and other human ES cells, such as WA01-H1 and WA09-H9, cited for reference only. The detailed protocols are shown in Example 10. The changes in over 47,000 human gene expression patterns were assessed using Affymetrix gene microarrays (GeneChip U133A&B and U133 plus 2.0 arrays). We first duplicated the microarray tests using the same mirPS sample and selected two hundred most variable genes (white dots) from one of the tests for further comparison. As shown in Figs.8A (mirPS-Colo) and 9A (mirPS-hHFC), the overall changes of duplicated tests were less than one fold (most left), indicating that the background variation was very limited. Based on the scattering patterns of all microarray-identified genes, we then calculated the correlation coefficiency (CC) between the results of two compared transcriptome libraries. A CC rate was given to show the percentage of similarity in the genome-wide gene expression patterns with a threshold of only one-fold change. Under such stringent CC rate definition, we found that the gene expression patterns of mirPS cells were very similar to those of human ES WA01-H1 (>89%) and WA09-H9 (>86%) cells, whereas only a low 47%-53% CC rate was shown between the mirPS cells and their somatic/cancerous cell origins. This strong genetic correlation between human ES and mirPS cells suggests that mir-302s may need to alter thousands of cellular gene expressions, which are involved in the reprogramming process of a somatic/cancer cell into an ES-like mirPS cell. For example, the elevation of many ES gene expressions and shutdown of numerous oncogenic, developmental and mir-302-targeted cell cycle-related genes were consistently and concurrently observed in the mirPS and human ES cell results, as shown in Fig.8B. With reference to Fig.9B, in regard to the gene expression patterns of the mirPS cells, it is also noted that SSEA-1 is moderately expressed in the mirPS cells, whereas Klf4 is not.

The list of some major differentially expressed genes between Colo and mirPS-Colo cells was shown in Fig.8B. In Fig.8B, we noted that cell-cycle checkpoint genes, *i.e.* CDK2, cyclin D1 and D2, and DNA methylation facilitator, i.e. MECP2 and MECP1-p66, were all confirmed to be strong targets for mir-302s. In other words, the pluripotent stem-like cells express abundant mir-302 microRNAs and Oct4, but limited CDK2, cyclin D1, MECP1-p66 and MECP2. The same result was also observed in Figs.8C and 9B. It is known that cyclin E-dependent CDK2 is required for the entry of S-phase cell cycle and inhibition of CDK2 results in G1-phase checkpoint arrest, whereas cyclin D1 can override G1-phase arrest in response to DNA damage. Based on this principle, the suppression of both CDK2 and cyclin D1 in mirPS cells revealed a fact that the cell cycle of mir-302-transfected cancer cells could reach a very slow cell division rate as shown in Fig.5A. The result of such cancer-stem cell cycle transition may therefore provide a significant benefit in cancer therapy. In addition, the suppression of MECP2 and MECP1-p66 activities was consistent with the results of Figs.7A-C, which indicated the epigenetic reprogramming of malignant cancer cells into benign mirPS cells. It is conceivable that the mirPS cells so obtained from patients may further help to repair the tissue damages of tumors/cancers. Taken together, all these findings indicate that the presently invented mir-302 transgene method can be used to reprogram the genetic profiles of human somatic/cancer cells into a highly ES-like expression pattern, similar to those of human ES cells.

### Pluripotency of the mirPS cells

Pluripotency defines the most important characteristic of an ES cell. Through *in vitro* manipulations with different factors and/or hormones, human ES cells can differentiate into the three embryonic germ layers (ectoderm, mesoderm and definitive endoderm) - the founders of all adult tissues. In the absence of any treatment, xenograft transplantation of the mirPS-derived embryoid bodies into the uterus or peritoneal cavity of female pseudopregnant immunocompromised SCID-beige mice could form teratoma-like tissue cysts (Fig. 10). These cysts were not observed in other tissue locations. However, unlike teratomas, these tissue cysts formed a very nice and clean boundary to their surrounding tissues. Also, the growth of these cyst structures in mice was slowed down approximately 2.5-week post-transplantation. It seems that there is a self-regulation mechanism limiting the random growth of these mirPS cells *in vivo.* This self-regulation mechanism may also prevent the tumor formation from these mirPS cells, offering a means for designing and developing tumor-free pluripotent stem cells for clinical trials and therapies.

### In-vitro molecular guidance of mirPS cell differentiation

In definition, a pluripotent stem cell can differentiate into various cell types similar to the tissue cells derived from embryonic ectoderm, mesoderm and/or endoderm. For example, using *in vitro* treatments of various growth factors and/or hormones, we could direct the differentiation of ES-like mirPS cells into several somatic and/or germ-line tissue cell types, including neuronal progenitors (Fig.5B), spermatogonia-like cells (Figs.11A-E), fibroblasts (Figs.11F-J), and chondrocytes (Figs.11K-O). Markers for these special tissue lineages have been identified with immunohistochemical (IHC) detection, showing neuron-specific Tuj1 and ABCA2, germ line-specific Dazla and EE2, fibroblast-specific atlastin1 and type I pro-collagen (COL1A1), and chondrocyte-specific tropoelastin and type II pro-collagen (COL2A1), respectively. In other words, the pluripotent stem-like cell generated by the present invention can differentiate into a germ line-like cell, spermatogonia-like cell, normal somatic cell, fibroblast, chondrocyte, and a combincation thereof. No sign of tumor formation was observed in these differentiated mirPS cells. In fact, many oncogenes are known to be the targets of mir-302s, based on the prediction of "TARGETSCAN" and "PICTAR-VERT" programs at the Sanger website. Moreover, mir-302s can suppress cyclin-dependent kinase 2 (CDK2), cyclin D1 and D2 to prevent the fast growth of tumor cells (Lin *et al*., 2008b). These findings signified the tumor-free pluripotency of the mirPS cells. It is conceivable that many more tissue cell types may be induced from the mirPS cells, using various molecular treatments.

Performing experiments under a feeder-free cultural condition *in vitro*, we have shown the successful guidance of mirPS cell differentiation into three relatively homogeneous somatic cell types (Figs.11A-O and Example 11). First, by treating the mirPS cells with a native androgen, dihydrotestosterone (DHT 50 ng/ml), for 6 hours in a feeder-free culture dish and then transplanting the treated cells (10⁵) into the uterus of a 6-week-old female immunocompromised SCID-beige mouse *in vivo,* a cyst of spermatogonia-like cells were generated one week later in the transplantation site (Figs.11A-E). Second, by treating with transforming growth factor-betal (TGF-β1 100 ng/ml) for 12 hours and then following the same transplantation procedure, the mirPS cells differentiated into fibroblasts and start to secret collagens within just one week (Figs.11F-J). Last, by treating with bone morphogenetic protein 4 (BMP4 100 ng/ml) for 12 hours and then xenografting the cells into the liver of a 6-week-old immunocompromised SCID-beige mouse, the mirPS cells differentiated into chondrocytes surrounding by calcified precipitates (Figs.11K-O). The use of immunocompromised nude mice was to provide an *in vivo* environment mimicking transplantation therapy. These findings provide strong evidence that we have successfully used the presently invented mir-302 transgene method to generate new ES-like pluripotent stem cell lines, which can be guided into multiple tissue cell types under a feeder-free condition in *vitro* and *in vivo.* Therefore, the present invention is able to not only reprogram differentiated somatic/cancer cells into an ES-like state but also maintain the ES-like renewal and pluripotency under a feeder-free cultural condition.

Thus, using an inducible mir-302-expressing transgene, the present invention provides a powerful new tool and strategy for generation of ES-like mir-302-induced pluripotent stem (mirPS) cells, particularly derived from primary cultures of human somatic and cancer cells. Most preferably, the mirPS cells are derived from human normal hair follicles due to their easy access. Because the intronic miRNA biogenesis pathway is well regulated by multiple intracellular surveillance systems, including components of mRNA transcription, RNA splicing, exosomal processing and NMD mechanisms, the presently invented intronic mir-302 expression is considered to be much safer than previous Pol-III (U6/H1)-driven siRNA/shRNA expression systems. In fact, the inventors have observed that the Pol-III-driven expression system tends to over-express mir-302 and causes cell cycle arrest and death at the G1 phase. In conjunction with a drug-inducible (*Tet-On*/*Off*) vector, the present invention can further offer a controllable means for not only reprogramming mammalian somatic/cancer cells into ES-like mirPS cells but also guiding them to form useful normal tissue cells. Because mir-302 has been found to be a strong tumor suppressor, the mirPS cells generated by the present invention are free of the risk of tumor formation.

Advantageously, there are at least five breakthroughs in the present invention. First, one mir-302-expressing transgene can replace all four large transcription factor genes used in the previous iPS methods for generating more homogeneous ES-like pluripotent stem cells derived from just a few somatic cells of patients, improving the stem cell purity and compatibility to patients' immune systems. Second, because the total size of the mir-302-expressing transgene is relatively small (about 1 kilo-bases), the transgene delivery is extremely high (over a 91 % success rate) compared with the maximal 2% in the previous iPS methods. Third, the generation and culture of mirPS cells are performed completely under a feeder-free condition without the risk of feeder antigen contamination. Fourth, no oncogene is used, preventing the risks of cell mutation and tumor formation. Last, the present invention uses electroporation in place of retroviral infection to deliver the single mir-302-expressing transgene, preventing the risk of random retroviral insertion into the host cell genome, which often causes insertional mutagenesis. Taken together, these advantages have solved the three major problems of the previous iPS methods, preventing the risks of retroviral infection, oncogenic mutation and uncertain tumorigenecity.

### A. Definitions

To facilitate understanding of the invention, a number of terms are defined below:
Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. A nucleoside containing at least one phosphate group bonded to the 3' or 5' position of the pentose is a nucleotide.
Oligonucleotide: a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof.
Nucleic Acid: a polymer of nucleotides, either single or double stranded.
Nucleotide Analog: a purine or pyrimidine nucleotide that differs structurally from A, T, G, C, or U, but is sufficiently similar to substitute for the normal nucleotide in a nucleic acid molecule.
Nucleic Acid Composition: a nucleic acid composition refers to polynucleotides such as deoxyribonucleic acid (DNA) and ribonucleic acid (RNA) in either single-stranded or double-stranded molecular structures.
Gene: a nucleic acid whose nucleotide sequence codes for an RNA and/or a polypeptide (protein). A gene can be either RNA or DNA.
Base Pair (bp): a partnership of adenine (A) with thymine (T), or of cytosine (C) with guanine (G) in a double stranded DNA molecule. In RNA, uracil (U) is substituted for thymine. Generally the partnership is achieved through hydrogen bonding.
Precursor messenger RNA (pre-mRNA): primary ribonucleotide transcripts of a gene, which are produced by type-II RNA polymerase (Pol-II) machineries in eukaryotes through an intracellular mechanism termed transcription. A pre-mRNA sequence contains a 5'-end untranslated region, a 3 '-end untranslated region, exons and introns.
Intron: a part or parts of a gene transcript sequence encoding non-protein-reading frames, such as in-frame intron, 5'-untranslated region (5'-UTR) and 3'-UTR.
Exon: a part or parts of a gene transcript sequence encoding protein-reading frames.
Messenger RNA (mRNA): assembly of pre-mRNA exons, which is formed after intron removal by intranuclear spliceosomal machineries and served as a protein-coding RNA for protein synthesis.
cDNA: a single stranded DNA that is complementary to an mRNA sequence and does not contain any intronic sequences.
Sense: a nucleic acid molecule in the same sequence order and composition as the homologous mRNA. The sense conformation is indicated with a "+", "s" or "sense" symbol.
Antisense: a nucleic acid molecule complementary to the respective mRNA molecule. The antisense conformation is indicated as a "-" symbol or with an "a" or "antisense" in front of the DNA or RNA, *e.g*., "aDNA" or "aRNA".
5'-end: a terminus lacking a nucleotide at the 5' position of successive nucleotides in which the 5'-hydroxyl group of one nucleotide is joined to the 3'-hydroyl group of the next nucleotide by a phosphodiester linkage. Other groups, such as one or more phosphates, may be present on the terminus.
3'-end: a terminus lacking a nucleotide at the 3' position of successive nucleotides in which the 5'-hydroxyl group of one nucleotide is joined to the 3'-hydroyl group of the next nucleotide by a phosphodiester linkage. Other groups, most often a hydroxyl group, may be present on the terminus.
Template: a nucleic acid molecule being copied by a nucleic acid polymerase. A template can be single-stranded, double-stranded or partially double-stranded, depending on the polymerase. The synthesized copy is complementary to the template, or to at least one strand of a double-stranded or partially double-stranded template. Both RNA and DNA are synthesized in the 5' to 3' direction. The two strands of a nucleic acid duplex are always aligned so that the 5' ends of the two strands are at opposite ends of the duplex (and, by necessity, so then are the 3' ends).
Nucleic Acid Template: a double-stranded DNA molecule, double stranded RNA molecule, hybrid molecules such as DNA-RNA or RNA-DNA hybrid, or single-stranded DNA or RNA molecule.
Conserved: a nucleotide sequence is conserved with respect to a pre-selected (referenced) sequence if it non-randomly hybridizes to an exact complement of the pre-selected sequence.
Complementary or Complementarity or Complementation: used in reference to polynucleotides (i.e. a sequence of nucleotides) related by the base-pairing rules. For example, the sequence "A-G-T" is complementary to the sequence "T-C-A," and also to "T-C-U." Complementation can be between two DNA strands, a DNA and an RNA strand, or between two RNA strands. Complementarity may be "partial" or "complete" or "total". Partial complementarity or complementation occurs when only some of the nucleic acid bases are matched according to the base pairing rules. Complete or total complementarity or complementation occurs when the bases are completely matched between the nucleic acid strands. The degree of complementarity between nucleic acid strands has significant effects on the efficiency and strength of hybridization between nucleic acid strands. This is of particular importance in amplification reactions, as well as in detection methods that depend on binding between nucleic acids. Percent complementarity or complementation refers to the number of mismatch bases over the total bases in one strand of the nucleic acid. Thus, a 50% complementation means that half of the bases were mismatched and half were matched. Two strands of nucleic acid can be complementary even though the two strands differ in the number of bases. In this situation, the complementation occurs between the portion of the longer strand corresponding to the bases on that strand that pair with the bases on the shorter strand.
Homologous or Homology: refers to a polynucleotide sequence having similarities with a gene or mRNA sequence. A nucleic acid sequence may be partially or completely homologous to a particular gene or mRNA sequence, for example. Homology may also be expressed as a percentage determined by the number of similar nucleotides over the total number of nucleotides.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize between the two strands with consequent hydrogen bonding.
Hybridize and Hybridization: the formation of duplexes between nucleotide sequences which are sufficiently complementary to form complexes via base pairing. Where a primer (or splice template) "hybridizes" with target (template), such complexes (or hybrids) are sufficiently stable to serve the priming function required by a DNA polymerase to initiate DNA synthesis. There is a specific, i.e. non-random, interaction between two complementary polynucleotides that can be competitively inhibited.
Posttranscriptional Gene Silencing: a targeted gene knockout or knockdown effect at the level of mRNA degradation or translational suppression, which is usually triggered by either foreign/viral DNA transgenes or small inhibitory RNAs.
RNA Interference (RNAi): a posttranscriptional gene silencing mechanism in eukaryotes, which can be triggered by small RNA molecules such as microRNA (miRNA), small hairpin RNA (shRNA) and small interfering RNA (siRNA). These small RNA molecules usually function as gene silencers, interfering with expression of intracellular genes containing either completely or partially complementarity to the small RNAs.
Non-coding RNA: an RNA transcript that cannot be used to synthesize peptides or proteins through intracellular translation machineries.
MicroRNA (miRNA): single-stranded RNAs capable of binding to targeted gene transcripts that have partial complementarity to the miRNA. MiRNA is usually about 17-27 oligonucleotides in length and is able to either directly degrade its intracellular mRNA target(s) or suppress the protein translation of its targeted mRNA, depending on the complementarity between the miRNA and its target mRNA. Natural miRNAs are found in almost all eukaryotes, functioning as a defense against viral infections and allowing regulation of gene expression during development of plants and animals.
Pre-miRNA: hairpin-like single-stranded RNAs containing stem-arm and stem-loop regions for interacting with intracellular RNaseIII endoribonucleases to produce one or multiple microRNAs (miRNAs) capable of silencing a targeted gene or genes complementary to the microRNA sequence(s). The stem-arm of a pre-miRNA can form either a perfectly (100%) or a partially (mis-matched) hybrid duplexes, while the stem-loop connects one end of the stem-arm duplex to form a circle or hairpin-loop conformation.
Small interfering RNA (siRNA): short double-stranded RNAs sized about 18-25 perfectly base-paired ribonucleotide duplexes and capable of degrading target gene transcripts with almost perfect complementarity.
Small or short hairpin RNA (shRNA): single-stranded RNAs that contain a pair of partially or completely matched stem-arm nucleotide sequences divided by an unmatched loop oligonucleotide to form a hairpin-like structure. Many natural miRNAs are derived from hairpin-like RNA precursors, namely precursor microRNA (pre-miRNA).
Vector: a recombinant nucleic acid composition such as recombinant DNA (rDNA) capable of movement and residence in different genetic environments. Generally, another nucleic acid is operatively linked therein. The vector can be capable of autonomous replication in a cell in which case the vector and the attached segment is replicated. One type of preferred vector is an episome, i.e., a nucleic acid molecule capable of extrachromosomal replication. Preferred vectors are those capable of autonomous replication and expression of nucleic acids. Vectors capable of directing the expression of genes encoding for one or more polypeptides and/or non-coding RNAs are referred to herein as "expression vectors". Particularly important vectors allow cloning of cDNA from mRNAs produced using a reverse transcriptase.
Cistron: a sequence of nucleotides in a DNA molecule coding for an amino acid residue sequence and including upstream and downstream DNA expression control elements.
Promoter: a nucleic acid to which a polymerase molecule recognizes, perhaps binds to, and initiates synthesis. For the purposes of the instant invention, a promoter can be a known polymerase binding site, an enhancer and the like, any sequence that can initiate synthesis by a desired polymerase.
Antibody: a peptide or protein molecule having a pre-selected conserved domain structure coding for a receptor capable of binding a pre-selected ligand.

### B. Compositions

A recombinant nucleic acid composition for expressing an isolated mir-302 agent and then inducing mir-302-mediated gene silencing in mammalian cells comprises:
a) A recombinant transgene, wherein said transgene encodes a recombinant non-coding RNA homologous to members of the mir-302 family; and
b) An expression-competent vector, wherein said vector can be used to deliver and express the recombinant transgene in mammalian cells.

The above recombinant transgene, further comprises:
a) A plurality of exons, wherein said exons can be linked to form a gene transcript possessing a desired function; and
b) At least an intron, wherein said intron contains a recombinant mir-302 homologue and can be cleaved out of the exons through intracellular RNA splicing and processing mechanisms.

The intron of the above recombinant transgene, further comprises:
a) A 5'-donor splice site for spliceosomal binding;
b) A gene-silencing effector insert homologous to members of the mir-302 family;
c) A branch point motif for spliceosomal recognition;
d) A poly-pyrimidine tract for spliceosomal interaction;
e) A 3'-acceptor splice site for spliceosomal binding; and
f) A plurality of linkers for connecting each of the above components in a 5' to 3' direction.

Preferably, the gene-silencing effector encodes a nucleic acid composition homologous to 5'-UAAGUGCUUC CAUGUUU-3' (SEQ.ID.NO.3). The 5'-donor splice site is a nucleotide sequence containing or homologous to either 5'-GTAAGAGK-3' (SEQ.ID.NO.4) or GU(A/G)AGU motifs (such as 5'-GTAAGAGGAT-3' (SEQ.ID.NO.37), 5'-GTAAGAGT-3', 5'-GTAGAGT-3' and 5'-GTAAGT-3'), while the 3'-acceptor splice site is a nucleotide sequence containing or homologous to either GWKSCYRCAG (SEQ.ID.NO.5) or CT(A/G)A(C/T)NG motifs (such as 5'-GATATCCTGC AG-3' (SEQ.ID.NO.42), 5'-GGCTGCAG-3' and 5'-CCACAG-3'). Moreover, a branch point sequence is located between the 5'- and 3'-splice sites, containing a motif homologous to 5'-TACTWAY-3' (SEQ.ID.NO.6), such as 5'-TACTAAC-3' and 5'-TACTTAT-3'. Furthermore, a poly-pyrimidine tract is closely located between the branch-point and 3'-splice site, containing a high T or C content sequence homologous to either 5'-(TY)m(C/-)(T)nS(C/-)-3' (SEQ.ID.NO.7) or 5'-(TC)nNCTAG(G/-)-3' (SEQ.ID.NO.8). The symbols of "m" and "n" indicate multiple repeats ≥ 1; most preferably, the m number is equal to 1∼3 and the n number is equal to 7∼12. The symbol "-" refers a nucleotide that can be skipped in the sequence. Based on the guideline of 37 CFR 1.822 for symbols and format to be used for nucleotide and/or amino acid sequence data, the symbol W refers to an adenine (A) or thymine (T)/uracil (U), the symbol K refers to a guanine (G) or thymine (T)/uracil (U), the symbol S refers to a cytosine (C) or guanine (G), the symbol Y refers to a cytosine (C) or thymine (T)/uracil (U), the symbol R refers to an adenine (A) or guanine (G), and the symbol N refers to an adenine (A), cytosine (C), guanine (G) or thymine (T)/uracil (U)." For all of the above spliceosomal recognition components, the deoxythymidine (T) nucleotide is replaceable with uridine (U).

### C. Methods

A method for reprogramming mammalian cells into pluripotent stem cells, using a recombinant nucleic acid composition capable inducing specific gene silencing effects on the genes targeted by mir-302, comprises the steps of:
a) Providing: i) a cell substrate expressing a plurality of developmental and cell differentiation-associated genes targeted by mir-302, and ii) a recombinant nucleic acid composition capable of being delivered, transcribed and processed into non-coding RNAs homologous to the mir-302 in the cell substrate;
b) Treating the cell substrate with the recombinant nucleic acid composition under conditions such that the mir-302-targeted gene functions in the cell substrate are inhibited.

Preferably, the recombinant nucleic acid composition is a drug-inducible *Tet-On* or *Tet-Off* vector capable of expressing a recombinant transgene, which encodes either a recombinant mir-302 family cluster (pre-mir-302s; linked hybrid of SEQ.ID.NOs.29-36) or a manually re-designed mir-302 shRNA homologue (hybrid of SEQ.ID.NOs.27 and 28). The cell substrate may express the pre-mir-302s/mir-302 shRNA and its target genes either *in vitro, ex vivo* or *in vivo.*

### EXAMPLES

The following examples serve to illustrate certain preferred embodiments and aspects of the present invention and are not to be construed as limiting the scope thereof.

In the experimental disclosure which follows, the following abbreviations apply: M (molar); mM (millimolar); µm (micromolar); mol (moles); pmol (picomolar); gm (grams); mg (milligrams) µg (micrograms); ng (nanograms); L (liters); ml (milliliters); µl (microliters); °C (degrees Centigrade); cDNA (copy or complementary DNA); DNA (deoxyribonucleic acid); ssDNA (single stranded DNA); dsDNA (double-stranded DNA); dNTP (deoxyribonucleotide triphosphate); RNA (ribonucleic acid); PBS (phosphate buffered saline); NaCl (sodium chloride); HEPES (N-2-hydroxyethylpiperazine-N-2-ethanesulfonic acid); HBS (HEPES buffered saline); SDS (sodium dodecylsulfate); Tris-HCl (tris-hydroxymethylaminomethane-hydrochloride); and ATCC (American Type Culture Collection, Rockville, MD).

### EXAMPLE 1

### Construction of SpRNAi-Containing: Recombinant RGFP Gene (SpRNAi-RGFP)

Synthetic oligonucleotides used for generating the *SpRNAi* introns containing either sense-, antisense- or hairpin-*EGFP* insert were listed as follows: N1-sense, 5'-GTAAGAGGAT CCGATCGCAG GAGCGCACCA TCTTCTTCAA GA-3' (SEQ.ID.NO.14); N1-antisense, 5'-CGCGTCTTGA AGAAGATGGT GCGCTCCTGC GATCGGATCC TCTTAC-3' (SEQ.ID.NO.15); N2-sense, 5'-GTAAGAGGAT CCGATCGCTT GAAGAAGATG GTGCGCTCCT GA-3' (SEQ.ID.NO.16); N2-antisense, 5'-CGCGTCAGGA GCGCACCATC TTCTTCAAGC GATCGGATCC TCTTAC-3' (SEQ.ID.NO.17); N3-sense, 5'-GTAAGAGGAT CCGATCGCAG GAGCGCACCA TCTTCTTCAA GTTAACTTGA AGAAGATGGT GCGCTCCTGA-3' (SEQ.ID.NO.18); N3-antisense, 5'-CGCGTCAGGA GCGCACCATC TTCTTCAAGT TAACTTGAAG AAGATGGTGC GCTCCTGCGA TCGGATCCTC TTAC-3' (SEQ.ID.NO.19); N4-sense, 5'-CGCGTTACTA ACTGGTACCT CTTCTTTTTT TTTTTGATAT CCTGCAG-3' (SEQ.ID.NO.20); N4-antisense, 5'-GTCCTGCAGG ATATCAAAAA AAAAAGAAGA GGTACCAGTT AGTAA-3' (SEQ.ID.NO.21). All sequences listed from SEQ.ID.NO.14 to SEQIDNO.21 are phosphorylated in their 5'-ends.

In addition, two RGFP exons were generated by cleavage of *DraII* restriction enzyme in a red fluorescent *RGFP* gene (SEQ.ID.NO.22) at its 208th nucleotide (nt) site. The 5' *RGFP* exon was further blunt-ended by T4 DNA polymerases. The *RGFP* referred to a novel red-shifted fluorescent chromoprotein gene generated by insertion of an extra aspartate at the 69th amino acid (a.a.) site of the HcRed1 chromoprotein isolated from *Heteractis crispa* (BD Biosciences, CA), resulting in less aggregate and almost twice intense infar-red fluorescent emission at the 570-nm wavelength.

Hybridization of N1-sense to N1-antisense, N2-sense to N2-antisense, N3-sense to N3-antisense, and N4-sense to N4-antisense was separately performed by heating each complementary mixture of sense and antisense (1:1) sequences to 94°C for 2 min and then 70°C for 10 min in 1 x PCR buffer (*e.g.* 50 mM Tris-HCl, pH 9.2 at 25°C, 16 mM (NH₄)₂SO₄, 1.75 mM MgCl₂). Immediately after that, sequential ligation of either N1, N2 or N3 hybrid to the N4 hybrid was performed by gradually cooling the mixture of N1+N4, N2+N4 or N3+N4 (1:1) hybrids, respectively, from 50°C to 10°C over a period of 1 hour, and then T₄ DNA ligase and buffer (Roche, IN) were added into the mixture and incubated together for 12 hours at 12°C. This formed the *SpRNAi* introns. Then, the two *RGFP* exons were added into the reaction (1:1:1) and T4 DNA ligase and buffer were adjusted accordingly to reiterate the ligation reaction for another 12 hours at 12°C.

For cloning the correctly recombinant *SpRNAi*-inserted *RGFP* (*SpRNAi-RGFP*) gene, 10 ng of the ligated sequences were amplified by PCR with a pair of *RGFP*-specific primers 5'-CTCGAGCATG GTGAGCGGCC TGCTGAA-3' (SEQ.ID.NO.23) and 5'-TCTAGAAGTT GGCCTTCTCG GGCAGGT-3' (SEQ.ID.NO.24) at 94°C, 1 min, 52-57°, 1 min and then 68°C, 2 min for 25-30 cycles. The resulting PCR products were fractionated on a 2% agarose gel, and a 900-1100 bp nucleotide sequences was extracted and purified using a gel extraction kit (Qiagen, CA). The composition of this ∼1-kb *SpRNAi-RGFP* gene was further confirmed by sequencing. Preferably, in the absence of intronic insertion, the sense strand of the *SpRNAi* intron sequence is 5'-GTAAGTGGTC CGATCGTCGC GACGCGTCAT TACTAACTAT CAATATCTTA ATCCTGTCCC TTTTTTTTCC ACAGTAGGAC CTTCGTGCA-3' (SEQ.ID.NO.25), while the antisense strand of the *SpRNAi* intron sequence is 5'-TGCACGAAGG TCCTACTGTG GAAAAAAAAG GGACAGGATT AAGATATTGA TAGTTAGTAA TGACGCGTCG CGACGATCGG ACCACTTAC-3' (SEQ.ID.NO.26).

Alternatively, the recombinant *SpRNAi-RGFP* transgene can be directly made by ligation of the hybrid of SEQ.ID.NO.25 and SEQ.ID.NO.26 (*SpRNAi*) with the restriction fragments of the *DraII*-cleaved *RGFP* exons, and following the same protocol as shown above. The *SpRNAi-RGFP* transgene used for testing the manually re-designed mir-302 shRNA insert (encoding SEQ.ID.NO.9) was formed by this way.

Because the recombinant *SpRNAi-RGFP* transgene possesses an *XhoI* and an *XbaI* restriction site at its 5'- and 3'-end, respectively, it can be easily cloned into a vector with cohesive ends to the *XhoI* and *XbaI* cloning sites. The vector must be an expressing-competent organism or suborganism selected from the group consisted of DNA transgenes, plasmids, jumping genes, transposons and viral vectors. Moreover, because the insertion site within the intron is flanked with a *PvuI* and an *MluI* restriction site at its 5'- and 3'-end, respectively, we can remove and replace the intronic insert with another different insert sequence possessing cohesive ends to the *PvuI* and *MluI* cloning sites. The insert sequence is preferably a hairpin-like gene silencing effector containing high complementarity to a target gene selected from the group consisted of fluorescent protein (*GFP*) genes, luciferase genes, lac-Z genes, viral genes, bacterial genes, plant genes, animal genes and human genes. The complementarity and/or homology rate between the gene-silencing effector and its targeted gene is ranged from about 30%-100%, more preferably 35%-49% for a hairpin-shRNA insert and 90%-100% for both sense-RNA and antisense-RNA inserts.

### EXAMPLE 2

### Cloning of the SpRNAi-RGFP Genes into A Expression-Competent Vector and Insertion of Recombinant mir-302 Homologues into the SpRNAi-RGFP Gene

Because the recombinant *SpRNAi-RGFP* transgene possessed an *XhoI* and an *XbaI* restriction site at its 5'- and 3'-end, respectively, it can be easily cloned into a vector with relatively cohesive ends to the *XhoI* and *XbaI* restriction sites. As shown in Fig.3A, we incorporated the *SpRNAi-RGFP* transgene into a *XhoI*/*XbaI*-linearized ∼6,900-bp *pTet-On-tTS* plasmid at 1:1 (w/w) ratio, cooled the mixture from 65°C to 15°C over a period of 50 min, and then added T₄ ligase and buffer accordingly into the mixture for ligation at 12°C for 12 hours. This formed an inducible *SpRNAi-RGFP* expression vector. The composition of the vector was confirmed by PCR with the *RGFP*-specific primers SEQ.ID.NO.23 and SEQ.ID.NO.24 at 94°C, 1 min and then 68°C, 2 min for 30 cycles, and further sequencing. For cloning into a retroviral vector, the same restriction and ligation procedures were performed except using an *XhoI*/*XbaI*-linearized *pLNCX2* retroviral vector (BD Clontech) instead. Since the insertion site of the *SpRNAi* intron was flanked with a *PvuI* and a *MluI* restriction site at its 5'- and 3'-end, respectively, we could remove and replace the *anti-EGFP* shRNA insert with a manually re-designed mir-302 shRNA insert possessing cohensive ends to the *PvuI* and *MluI* cloning sites. The re-designed mir-302 shRNA insert contained a sequence homologous to 5'-UAAGUGCUUC CAUGUUU-3' (SEQ.ID.NO.3), including 5'-UAAGUGCUUC CAUGUUUUAG UGU-3' (SEQ.ID.NO.9), 5'-UAAGUGCUUC CAUGUUUUGG UGA-3' (SEQ.ID.NO.10), 5'-UAAGUGCUUC CAUGUUUUAG UAG-3' (SEQ.ID.NO.11), 5'-UAAGUGCUUC CAUGUUUCAG UGG-3' (SEQ.ID.NO.12), or 5'-UAAGUGCUUC CAUGUUUGAG UGU-3' (SEQ.ID.NO.13). Most preferably, the re-designed mir-302 shRNA insert contained 5'-UAAGUGCUUC CAUGUUUUAG UGU-3' (SEQ.ID.NO.9). In other words, the gene silencing effector includes at least a recombinant RNA sequence homologous to the group consisting of SEQ.ID.NO.9, SEQ.ID.NO.10, SEQ.ID.NO.11, SEQ.ID.NO.12, SEQ.ID.NO.13, and a combination thereof.

Synthetic oligonucleotides used for DNA recombination of either the recombinant mir-302 familial pre-miRNA or the manually re-designed mir-302 shRNA insert were listed as follows: mir-302a-sense, 5'-GTCCGATCGT CCCACCACTT AAACGTGGAT GTACTTGCTT TGAAACTAAA GAAGTAAGTG CTTCCATGTT TTGGTGATGG ATCTCGAGCT C-3' (SEQ.ID.NO.29); mir-302a-antisense, 5'-GAGCTCGAGA TCCATCACCA AAACATGGAA GCACTTACTT CTTTAGTTTC AAAGCAAGTA CATCCACGTT TAAGTGGTGG GACGATCGGA C-3' (SEQ.ID.NO.30); mir-302b-sense, 5'-ATCTCGAGCT CGCTCCCTTC AACTTTAACA TGGAAGTGCT TTCTGTGACT TTGAAAGTAA GTGCTTCCAT GTTTTAGTAG GAGTCGCTAG CGCTA-3' (SEQ.ID.NO.31); mir-302b-antisense, 5'-TAGCGCTAGC GACTCCTACT AAAACATGGA AGCACTTACT TTCAAAGTCA CAGAAAGCAC TTCCATGTTA AAGTTGAAGG GAGCGAGCTC GAGAT-3' (SEQ.ID.NO.32); mir-302c-sense, 5'-CGCTAGCGCT ACCTTTGCTT TAACATGGAG GTACCTGCTG TGTGAAACAG AAGTAAGTGC TTCCATGTTT CAGTGGAGGC GTCTAGACAT-3' (SEQ.ID.NO.33); mir-302c-antisense, 5'-ATGTCTAGAC GCCTCCACTG AAACATGGAA GCACTTACTT CTGTTTCACA CAGCAGGTAC CTCCATGTTA AAGCAAAGGT AGCGCTAGCG-3' (SEQ.ID.NO.34); mir-302d-sense, 5'-CGTCTAGACA TAACACTCAA ACATGGAAGC ACTTAGCTAA GCCAGGCTAA GTGCTTCCAT GTTTGAGTGT TCGACGCGTC AT-3' (SEQ.ID.NO.35); mir-302d-antisense, 5'-ATGACGCGTC GAACACTCAA ACATGGAAGC ACTTAGCCTG GCTTAGCTAA GTGCTTCCAT GTTTGAGTGT TATGTCTAGA CG-3' (SEQ.ID.NO.36); and miR-302s-sense, 5'-GTCCGATCGT CATAAGTGCT TCCATGTTTT AGTGTGCTAA GCCAGGCACA CTAAAACATG GAAGCACTTA TCGACGCGTC AT-3' (SEQ.ID.NO.27); mir-302s-antisense, 5'-ATGACGCGTC GATAAGTGCT TCCATGTTTT AGTGTGCCTG GCTTAGCACA CTAAAACATG GAAGCACTTA TGACGATCGG AC-3' (SEQ.ID.NO.28) (Sigma-Genosys, MO). All these synthetic sequences were purified with PAGE gel extraction before ligation. In other words, the gene silencing effector is formed by ligation linkage of the hybrids of SEQ.ID.NO.29, SEQ.ID.NO.30, SEQ.ID.NO.31, SEQ.ID.NO.32, SEQ.ID.NO.33, SEQ.ID.NO.34, SEQ.ID.NO.35, SEQ.ID.NO.36, and a combination thereof.

The recombinant mir-302 familial pre-miRNA cluster was formed by linkage of four mir-302a-d hybrids, including mir-302a-sense and mir-302a-antisense, mir-302b-sense and mir-302b-antisense, mir-302c-sense and mir-302c-antisense, and mir-302d-sense and mir-302d-antisense. The hybrids of mir-302a, mir-302b, mir-302c, and mir-302d were digested by *PvuI*/*XhoI*, *XhoI*/*NheI*, *NheI*/*XbaI*, and *XbaI*/*MluI* restriction enzymes, respectively, and collected together by a gel extraction filter column in 35 µl autoclaved ddH₂O (Qiagen, CA). Immediately after that, the mixed hybrids were ligated to form a cluster of the mir-302 familial pre-miRNA insert with T4 DNA ligase (Roche, 20U) and further inserted into the *PvuI*/*MluI*-linearized *SpRNAi-RGFP* expression vectors. Alternatively, the manually re-designed mir-302 shRNA was made by hybridizing two synthetic sequences of the SEQ.ID.NO.27 and SEQ.ID.NO.28, and then cleaved with *PvuI*/*MluI* restriction enzymes for insertion into the *PvuI*/*MluI*-linearized *SpRNAi-RGFP* expression vectors. The inducible *SpRNAi-RGFP* expression vector containing the recombinant mir-302 familial pre-miRNA (*i.e. pTet-On-tTS-mir302s*) was transgenically delivered into hHFC cells, whereas the vector containing the re-designed mir-302 shRNA was introduced into Colo 829 cells, following the procedure listed in the Figs.2A and 2B.

The *SpRNAi-RGFP* expression vectors could be propagated in *E.coli* DH5α LB culture containing 100 µg/ml ampicillin (Sigma Chemical, St. Louis, MO). The propagated *SpRNAi-RGFP* expression vectors were isolated and purified using a mini-prep or maxi-prep plasmid extraction kit (Qiagen, CA). For *pLNCX2* retroviral vectors, we could also use a packaging cell line GP2-293 (Clontech, CA) for producing infectious but replication-incompetent virus. The transfected GP2-293 cells were grown in 1x DMEM medium supplemented with charcoal-stripped 10% fetal bovine serum (FBS) with 4 mM L-glutamine, 1 mM sodium pyruvate, 100 µg/ml streptomycin sulfate and 50 µg/ml neomycin (Sigma Chemical, MO) at 37°C under 5% CO₂. The viral titer was measured to be over multiplicity of infection (MOI) 30 before transfection, following the protocol of a retro-X qRT-PCR titration kit (Clontech, CA).

### EXAMPLE 3

### Cell Culture and Transgenic Delivery of mir-302s

Human cancer PC3 and Colo 829 cell lines were obtained from the American Type Culture Collection (ATCC, Rockville, MD), while hHFC and hpESC cells were prepared by collagenase/trypsin (4:1) dissociation of either two-ten hair follicle roots or a 2 mm³ skin explant from the inventor's hairs or arm, respectively. The cells were cultivated in RPMI 1640 medium supplemented with 10% fetal bovine serum (FBS), 4 mM L-glutamine, 1 mM sodium pyruvate and 100 µg/ml gentamycin (Sigma Chemical, MO), at 37°C under 5% CO₂. Cultures were passaged at 70%∼80% confluency by exposing cells to trypsin-EDTA solution for 1 min and rinsing once with RPMI, and the detached cells were replated at 1:10 dilution in fresh growth medium. For transgenic mir-302s delivery with electroporation, the *pTet-On-tTS-mir3O2s* vector (10-30 µg) was mixed with the host cells (200-2000) in a hypoosmolar PH buffer (400 µl; Eppendorf) and electroporation was performed at 400-450 V for 100 µsec to deliver the vector into the host cell genomes. Positively transgenic mirPS cells were isolated and collected 72 hours later, using FACS flow cytometry selection with anti-RGFP monoclonal antibodies (Fig.3C). The success rate of this novel mir-302s transgene approach is measured to be over 91%.

Alternatively, for retroviral vector delivery, we first cultured the *pVSV-G* co-transfected GP2-293 cells (Clontech, CA) with the *SpRNAi-RGFP*-inserted *pLNCX2* retroviral vectors containing a recombinant mir-302 familial pre-miRNA insert. After 36-hour incubation at 37°C under 5% CO₂, the cultural mediums (10 ml each) of the GP2-293 cells were filtrated (0.25 µm) and directly transferred into the tested cell cultures for 12 hours at 37°C under 5% CO₂. After that, fresh mirPS cell culture medium was added in place of the virus medium and replaced every three days. Since the mediums contained very high titers of the designed retroviral vectors, almost all the tested cells (99.4%-99.8%) were transgenically infected by the vectors and started to express the intronic inserts and RGFP within 24 hours. Positively transgenic mirPS cells were isolated and collected 24 hours post-infection, using FACS flow cytometry sorting with anti-RGFP monoclonal antibodies (Clontech, CA).

### EXAMPLE 4

### Northern Blot Analysis

Total RNAs (20 µg) were fractionated on 1% formaldehyde-agarose gels and transferred onto nylon membranes (Schleicher & Schuell, Keene, NH). Synthetic LNA-DNA probes (Sigma-Genosys, MO) complementary to either the 75-bp junction sequence flanking between the *RGFP* 5'-exons or the designed pre-miRNA/shRNA insert, or a target gene transcript, were labeled with the Prime-It II kit (Stratagene, La Jolla, CA) by random primer extension in the presence of [³²P]-dATP (> 3000 Ci/mM, Amersham International, Arlington Heights, IL), and purified with 10 bp-cutoff Micro Bio-Spin chromatography columns (Bio-Rad, Hercules, CA). Hybridization was carried out in the mixture of 50% freshly deionized formamide (pH 7.0), 5 x Denhardt's solution, 0.5% SDS, 4 x SSPE and 250 mg/mL denatured salmon sperm DNA fragments (18 hr, 42°C). Membranes were sequentially washed twice in 2x SSC, 0.1% SDS (15 min, 25°C), and once in 0.2 x SSC, 0.1% SDS (45 min, 37°C) before autoradiography. The results were shown in Figs.4B and 6B.

### EXAMPLE 5

### SDS-PAGE and Western Blot Analysis

For immunoblotting of target proteins (Figs.8C and 9B), isolated cells at ∼70% confluency were rinsed with ice cold PBS after growth medium was removed, and then lysed a CelLytic-M lysis/extraction reagent (Sigma-Aldrich, MO) supplemented with protease inhibitors, Leupeptin, TLCK, TAME and PMSF. The cells were incubated at room temperature on a shaker for 15 min, scraped into microtubes, and centrifuged for 5 min at 12,000 xg to pellet the cell debris. Protein-containing cell lysate were collected and stored at -70°C until use. Protein determinations were measured with SOFTmax software package on an E-max microplate reader (Molecular Devices, Sunnyvale, CA). Each 30 µg of cell lysate was added to SDS-PAGE sample buffer under reducing (+50 mM DTT) and nonreducing (no DTT) conditions, and boiled for 3 min before loading onto 6%-8% polyacylamide gels; molecular weights were determined by comparison to standard proteins (Bio-Rad, Hercules, CA). SDS-polyacrylamide gel electrophoresis was performed according to the standard protocols. Proteins resolved by PAGE were electroblotted onto a nitrocellulose membrane and incubated in Odyssey blocking reagent (Li-Cor Biosciences, Lincoln, NB) for 2 hours at room temperature. Then, we applied a primary antibody to the reagent and incubated the mixture at 4°C. Primary antibodies used included Oct3/4 (1:500, Santa Cruz), SSEA-3 (1:500, Santa Cruz), SSEA-4 (1:500, Santa Cruz), Sox2 (1:500, Santa Cruz), Nanog (1:500, Santa Cruz), Klf4 (1:200, Santa Cruz), β-actin (1:2000, Chemicon, Temecula, CA), and RGFP (1:1000, Clontech). After overnight, the membrane was rinsed three times with TBS-T and then exposed to goat anti-mouse IgG conjugated secondary antibody to Alexa Fluor 680 reactive dye (1:2,000; Invitrogen-Molecular Probes), for 1 hour at the room temperature. After three additional TBS-T rinses, fluorescent scanning of the immunoblot and image analysis were conducted using Li-Cor Odyssey Infrared Imager and Odyssey Software v. 10.

### EXAMPLE 6

### Intronic RNA-mediated Gene Silencing in Zebrafish

Tg(*actin*-GAL4:UAS-gfp) strain zebrafish larvae were raised in a fish container with 10 ml of 0.2x serum-free RPMI 1640 medium during transfection. A transfection pre-mix was prepared by gently dissolving 60 µl of a FuGene liposomal transfection reagent (Roche Biochemicals, Indianapolis, IN) in 1 ml of 1x serum-free RPMI 1640 medium. The *SpRNAi-RGFP* vectors (20 µg) with an anti-EGFP pre-miRNA insert, as shown in Examples 1-2, were then mixed with the pre-mix solution, stayed on ice for 30 min and directly applied to the Tg(*actin*-GAL4:UAS-gfp) fish larvae in the container. Total three dosages were given in a 12-hour interval (total 60 µg). Samples were collected 60 hours after the first transfection. The result was shown in Fig.1B.

### EXAMPLE 7

### Flow Cytometry Assay

After desired experiments, cells were trypsinized, pelleted and fixed by re-suspending in 1 ml of pre-chilled 70% methanol in PBS for 1 hour at -20°C. The cells were pelleted and washed once with 1 ml of PBS. The cells were pelleted again and resuspended in 1 ml of 1 mg/ml propidium iodide, 0.5 mg/ml RNase in PBS for 30 min at 37°C. Approximately 15,000 cells were then analyzed on a BD FACSCalibur flow cytometer (San Jose, CA). Cell doublets were excluded by plotting pulse width versus pulse area and gating on the single cells. The collected data were analyzed using the software package Flowjo using the "Watson Pragmatic" algorithm. As shown in Fig.5A, the first (left) and second (right) peaks of the flow cytometry charts represented the levels of resting G0/G1 and mitotic M phase cell populations in the entire tested cell population, respectively.

### EXAMPLE 8

### DNA Demethylation Assays

Genomic DNAs from about two million cells were isolated with a DNA isolation kit (Roche) and divided into two aliquots. One of the DNA aliquot (2 µg) was digested with a CCGG-cutting restriction enzyme, *HpaII*, and then assessed with 1% agarose gel electrophoresis to determine genome-wide demethylation (Fig.7A). The other aliquot (2 µg) was used for PCR cloning the complete 9,400 base-pair (bp) 5'-regulatory region of the *Oct3*/*4* promoter (NT_007592 nucleotides 21992184-22001688), before and after bisulfite modification. Bisulfite modification was performed with a CpGenome DNA medification kit (Chemicon, CA). The treatment of bisulfite to DNA converted all unmethylated cytosines to uracils while methylated cytosines remained as cytosines. For example, unmethylated ACGT sites, but not methylated ACGT, were changed into AUGT sites. PCR primers specific to the target *Oct3*/*4* 5'-promoter region before and after bisulfite modification had been designed and tested, including two forward primers 5'-GAGGAGTTGA GGGTACTGTG-3'(SEQ.ID.NO.44) (for bisulfite-modified DNAs) and 5'-GAGGAGCTGA GGGCACTGTG-3' (SEQ.ID.NO.45) (for non-modified DNAs) and one reverse primer 5'-GTAGAAGTGC CTCTGCCTTC C-3' (SEQ.ID.NO.46). For PCR cloning, the genomic DNAs (50 ng), either bisulfite-treated or untreated, were first mixed with the primers (total 150 pmole) in 1x PCR buffer, heated to 94°C for 4 min, and immediately cooled on ice. After that, 25 cycles of PCR were performed as follows: at 92°C for 1 min, at 55°C for 1 min and then at 70°C for 5 min, using a long template PCR extension kit (Roche). The resulting products were collected with a PCR purification kit (Qiagen) and 2 µg of the DNAs were digested with an equal mixture (5U each) of multiple ACGT-cutting restriction enzymes, containing *AclI* (AACGTT), *BmgBI* (CACGTC), *PmlI* (CACGTG), *SnaBI* (TACGTA) and *HpyCH4IV* (ACGT). Then the digested fragments were assessed using 3% agarose gel electrophoresis (Fig.7B).

For bisulfite DNA sequencing analysis (Fig.7C), we further amplified a 467-bp target region flanking the *Oct3*/*4* transcription initiation site (NT_007592 nucleotides 21996577-21997043), using quantitative PCR (qPCR). Primers used were one forward primer 5'-GAGGCTGGAG TAGAAGGATT GCTTTGG-3'(SEQ.ID.NO.47) and one reverse primer 5'-CCCTCCTGAC CCATCACCTC CACCACC-3'(SEQ.ID.NO.48). The above cloned 9,400-bp *Oct3*/*4* 5'-promoter region (50 ng) was mixed with the qPCR primers (total 100 pmole) in 1x PCR buffer, heated to 94°C for 2 min, and immediately cooled on ice. Then, 20 cycles of PCR were performed as follows: at 94°C for 30 sec and at 68°C for 1 min, using a high-fidelity PCR extension kit (Roche). The amplified DNA products with a correct 467-bp size were further fractionized by 3% agarose gel electrophoresis, purified with a gel extraction kit (Qiagen), and then used in DNA sequencing. A detailed profile of the DNA methylation sites was generated by comparing the unchanged cytosines in the bisulfite-modified DNA to those in the non-modified DNA sequence.

### EXAMPLE 9

### MicroRNA (miRNA) Microarray Analysis

At 70% confluency, small RNAs from each cell culture were isolated, using the *mir*Vana™ miRNA isolation kit (Ambion). The purity and quantity of the isolated small RNAs were assessed, using 1% formaldehyde-agarose gel electrophoresis and spectrophotometer measurement (Bio-Rad), and then immediately frozen in dry ice and submitted to LC Sciences (San Diego, CA) for miRNA microarray analysis. Each microarray chip was hybridized a single sample labeled with either Cy3 or Cy5 or a pair of samples labeled with Cy3 and Cy5, respectively. Background subtraction and normalization were performed. For a dual sample assay, a *p*-value calculation was performed and a list of differentially expressed transcripts more than 3-fold was produced. In the Cy3 and Cy5 intensity images (blue background) of the Fig.6A, as signal intensity increased from level 1 to level 65,535 the corresponding color changed from blue to green, to yellow, and to red. The levels above 23,000 were considered to be positive calls in gene expression. In the Cy5/Cy3 ratio image (black background), when Cy3 level was higher than Cy5 level the color was green; when Cy3 level was equal to Cy5 level the color was yellow; and when Cy5 level was higher than Cy3 level the color was red.

### EXAMPLE 10

### Genome-Wide Microarray Analysis of Global Cellular Gene Expression Patterns

Human genome GeneChip U133A&B and plus 2.0 arrays (Affymetrix, Santa Clara, CA) containing over 54,000 oligonucleotide probes were used to detect the expression patterns of genome-wide 47,000 human gene transcripts in mirPS cells, as shown in Figs.8A and 9A. Each sample was tested in triplicate and the same experiment was repeated for four times. Total RNAs from each tested sample were isolated using RNeasy spin columns (Qiagen). To prepare labeled probes for microarray hybridization, the extracted total RNAs (2 µg) were converted into double-stranded cDNAs with a synthetic oligo(dT)₂₄-T7 promoter primer, 5'-GGCCAGTGAA TTGTAATACG ACTCACTATA GGGAGGCGG-(dT)₂₄-3'(SEQ.ID.NO.49), using Superscript Choice system (Invitrogen). The resulting cDNAs were purified by phenol/chloroform extractions, precipitated with ethanol, and resuspended at a concentration of 0.5 µg/µl in diethyl pyrocarbonate (DEPC)-treated ddH₂O. Then, *in-vitro* transcription was performed, containing 1 µg of the dsDNAs, 7.5 mM unlabeled ATP and GTP, 5 mM unlabeled UTP and CTP, and 2 mM biotin-labeled CTP and UTP (biotin-11-CTP, biotin-16-UTP, Enzo Diagnostics), and 20 U of T7 RNA polymerase. Reactions were carried out for 4 hours at 37°C, and the resulting cRNAs were purified by RNeasy spin columns (Qiagen). A part of the cRNA sample was separated on a 1% agarose gel to check the size range, and then 10 µg of the cRNAs were fragmented randomly to an average size of 50 bases by heating at 94°C for 35 min in 40 mM Tris-acetate, pH 8.0, 100 mM KOAc/30 mM MgOAc. Hybridizations were completed in 200 µl of AFFY buffer (Affymetrix) at 40°C for 16 hours with constant mixing. After hybridization, arrays were rinsed three times with 200 µl of 6x SSPE-T buffer (1x 0.25 M sodium chloride/15 mM sodium phosphate, pH 7.6/1 mM HDTA/0.005% Triton) and then washed with 200 µl of 6x SSPE-T for 1 hour at 50°C. The arrays were further rinsed twice with 0.5X SSPE-T and washed with 0.5x SSPE-T at 50°C for 15 min. Then, staining assays were done with 2 µg/ml streptavidin-phycoerythrin (Invitrogen-Molecular Probes) and 1 mg/ml acetylated BSA (Sigma) in 6x SSPE-T (pH 7.6). The arrays were read at 7.5 µm with a confocal scanner (Molecular Dynamics).

To identify the background variations, we duplicated the microarray tests using the same sample and selected two hundred genes (white dots in Fig.8A), which were slightly presented in one side of the tests, for further comparison. The sample signals were normalized using the total average difference between perfectly matched probes and mismatched probes. Then, alterations of overall genome-wide gene expression patterns (green dots in Fig.8A) were analyzed using Affymetrix Microarray Suite version 5.0, Expression Console™ version 1.1.1 (Affymetrix) and Genesprings (Silicon Genetics) softwares. Changes in gene expression rates more than 1-fold were considered as positive differential genes. In gene clustering assays, a plug-in program Genetrix (Epicenter Software) was used in conjunction with the Affymetrix softwares. Signals of the sample were normalized with the internal house-keeping control average in each microarray. After normalization, as signal intensity increased from level 1 to level 65,535, the corresponding color changed from green to black, and to red. The level above 23,000 (in red) was considered to be a positive call in that a northern blotting assay could positively detect.

### EXAMPLE 11

### Cell Differentiation and Immunodetection Assays

In absence of any treatment except the feeder-free mirPS culture medium, xenograft transplantation of the mirPS-derived embryoid bodies into the uterus or peritoneal cavity of a 6-week-old female pseudopregnant immunocompromised SCID-beige mouse formed teratoma-like cysts (Fig.10). The use of immunocompromised nude mice was to provide an *in vivo* environment mimicking transplantation therapy. The pseudopregnant mice were made by intraperitoneally injection of 1 IU human menopausal gonadotrophin (HMG) for two days and then human chorionic gonadotrophin (hCG) for one more day. For *In vitro* molecular guidance of stem cell differentiation into the germ line lineage, mirPS cells were maintained on polyornithine/ laminin-coated dishes in DMEM/F12 (1:1; high glucose) medium supplemented with charcoal-stripped 10% FBS, 4 mM L-glutamine, 1 mM sodium pyruvate, 5 ng/ml activin and 50 ng/ml dihydrotestosterone (DHT) for 12 hours, at 37°C under 5% CO₂. Then the cells were trypsinized, washed with 1x PBS, and collected in four aliquots of chilled Matrigel (100 µl each) and one aliquots of 100 µl 1x PBS. Immediately after that, we transplanted the cells into the hind limb muscle, peritoneum, uterus, subcutaneous neck skin (with Matrigel) and tail vein (with PBS) of 6-week-old immunocompromised SCID-beige nude mice. The mice were anesthetized with diethyl ether during experimental processing. One week later, spermatogonia-like cells were found only in the uterus area. For fibroblast differentiation, we followed the same procedure as shown above, except using regular phenol red-free DMEM medium supplemented with 10% FBS, 4 mM L-glutamine, 1 mM sodium pyruvate, 5 ng/ml noggin and 100 ng/ml transforming growth factor-betal (TGF-β1) for 6 hours before xenotransplantation. Fibroblasts were found in the uterus one week later. For chondrocyte differentiation, we performed the same procedure as before but using regular RPMI 1640 medium supplemented with 10% FBS, 4 mM L-glutamine, 1 mM sodium pyruvate and 100 ng/ml bone morphogenetic protein 4 (BMP4) for 6 hours. Chondrocytes were found only in the liver area.

For immunodetection of specific tissue markers, the tissue samples were fixed in 4% paraformaldehyde overnight at 4°C. The samples were washed sequentially with 1x PBS, methanol, isopropanol and tetrahydronaphthalene before embedded in paraffin wax. The embedded samples were then cut on a microtome at 7-10 µm thickness and mounted on clean TESPA-coated slides. Then, the slides were dewaxed with xylene and mounted under coverslips using mounting media (Richard Allan Scientific, Kalamazoo, MI) and stained by hematoxylin and eosin (H&E, Sigma) for morphological observation. Immunohistochemical (IHC) staining kits were purchased from Imgenex (San Diego, CA). Processes for antibody dilution and immunostaining were performed according to the manufacturers' suggestions. Primary antibodies used included Tuj1 (1:500, Abcam Inc., Cambridge, MA), ABCA2 (1:100, Santa Cruz Biotechnology, Santa Cruz, CA), Dazla (1:100, Abcam), EE2 (1:100, Santa Cruz), atlastin1 (1:200, Santa Cruz), COL1A1 (1:500, Santa Cruz), COL2A1 (1:500, Santa Cruz), tropoelastin (1:200, Abcam), and RGFP (1:500, Clontech). Fluorescent dye-labeled goat anti-rabbit or horse anti-mouse antibody was used as the secondary antibody (1:2,000, Invitrogen-Molecular Probes). Positive results were observed under a 100x microscope with whole field scanning and measured at 200x or 400x magnification for quantitative analysis by a Metamorph Imaging program (Nikon 80i and TE2000 microscopic quantitation systems).

### EXAMPLE 12

### Cell Migration Assay

In a 96-well culture plate, we placed one PC3 and one mirPS-PC3 cell together in each well and then recorded their movement and interaction. Both of the cells were grown in RPMI 1640 medium supplemented with 10% charcoal-stripped FBS, 4 mM L-glutamine, I mM sodium pyruvate, 5 ng/ml activin, 5 ng/ml noggin, 3 ng/ml bFGF and 0.5 µM GSK-3 inhibitor XV, at 37°C under 5% CO₂. The cells were individually isolated and collected using a pair set of MO-188NE 3D hydraulic fine micromanipulators with a cell holder under a TE2000 invert microscopic system (Nikon). The whole micromanipulator and microscopic system was placed on an anti-vibration table. The pictures were recorded every 15 seconds for six hours at 400x and 600x magnification. The cell migration was determined by the tracking of cell movement in the pictures and the morphology of the cell. As shown in Fig.7D, the metastatic cancer PC3 cell presented a quick spindle-shape movement as described by the ATCC, whereas the mirPS-PC3 cell stayed in the placed location showing a round resting phenotype.

### EXAMPLE 13

### Statistical Analysis

Results were presented as mean ± SE. Statistical analysis of data was performed by one-way ANOVA. When main effects were significant, the Dunnett's post-hoc test was used to identify the groups that differed significantly from the controls. For pairwise comparison between two treatment groups, the two-tailed student t test was used. For experiments involving more than two treatment groups, ANOVA was performed followed by a post-hoc multiple range test. Probability values of *p* < 0.05 were considered significant. All p values were determined from two-tailed tests.

### REFERENCES

1. Thomson et al. (1998) Science 282: 1145-1147.
2. Takahashi and Yamanaka. (2006) Cell 126: 663-676.
3. Okita et al. (2007) Nature 448: 313-317.
4. Wernig et al. (2007) Nature 448: 318-324.
5. Yu et al. (2007) Science 318: 1917-1920.
6. Meissner et al. (2006) Nature 439: 212-215.
7. Hanna et al. (2007) Science 318: 1920-1923.
8. Suh et al. (2004) Dev. Biol. 270: 488-498.
9. Tang et al. (2007) Genes Dev. 21: 644-648.
10. Bartel, D.P. (2004) Cell 116: 281-297.
11. Simonsson and Gurdon. (2004) Nat Cell Biol. 6: 984-990.
12. Murchison et al. (2007) Genes Dev. 21: 682-693.
13. Ghosh et al. (2000) RNA 6: 1325-1334.
14. Lin et al. (2004) Novel RNAi therapy - Intron-derived microRNA drugs. Drug Design Reviews 1: 247-255.
15. Lin et al. (2003) A novel RNA splicing-mediated gene silencing mechanism potential for genome evolution. Biochem Biophys Res Commun. 310: 754-760.
16. Lin et al. (2005) Asymmetry of intronic pre-microRNA structures in functional RISC assembly. Gene 356: 32-38.
17. Lin et al. (2006a) Gene silencing in vitro and in vivo using intronic microRNAs. Methods Mol Biol. 342: 295-312.
18. Lin et al. (2006b) Transgene-like animal model using intronic microRNAs. Methods Mol Biol. 342: 321-334.
19. Lin et al. (2008a) Intron-mediated RNA interference and microRNA (miRNA). Frontiers in Bioscience 13: 2216-2230.
20. Lin et al. (2008b) Mir-302 reprograms human skin cancer cells into a pluripotent ES-cell-like state. RNA 14: 2115-2124.
21. Rodriguez et al. (2004) Genome Res. 14: 1902-1910.
22. Ghosh et al. (2000) RNA 6: 1325-1334.
23. Danin-Kreiselman et al. (2003) Mol Cell 11: 1279-1289.
24. Ruby et al. (2007) Nature 448: 83-86.
25. Tang, G. (2005) Trends Biochem Sci. 30: 106-114.
26. Lee et al. (2003) Nature 425: 415-419.
27. Lewin B. (2000) Genes, Seventh Edition, Oxford University press, pp688-690.
28. Scholer et al. (1989) EMBO J. 8: 2543-2550.
29. Rosner et al. (1990) Nature 345: 686-692.
30. Solter et al. (1978) Proc. Natl. Acad. Sci. USA 75: 5565-5569.
31. Shevinsky et al. (1982) Cell 30: 697-705.
32. Boyer et al. (2005) Cell 122: 947-956.
33. Hochedlinger et al. (2006) Nature 441: 1061-1067.
34. Stitzel et al. (2007) Science 316: 407-408.
35. O'Farrell et al. (2004) Curr. Biol. 14: R35-45.
36. U.S. Pat. No. 5,843,780, 6,200,806, 7,029,913, and 7,220,584 to Thomson.
37. U.S. Pat. No. 6,090,622, 6,245,566, and 6,331,406 to Gearhart.
38. U.S. Pat. No. 6,875,607 to Reubinoff.
39. U.S. Pat. No. 7,250,255 to Shinya Yamanaka.

## Claims

1. An in vitro method for reprogramming at least a cell into at least a non-tumor pluripotent stem cell, comprises these steps of:
(a) constructing a recombinant nucleic acid composition capable of being delivered, and processed into at least an RNA molecule containing the sequence of SEQ.ID.NO. 3 capable of silencing a plurality of genes targeted by mir-302, in particular CDK2, cyclin D1 and cyclin D2 genes;
(b) treating the cell in vitro with said recombinant nucleic acid composition to silence the plurality of genes including CDK2, cyclin D1 and cyclin D2 genes.

2. The method as defined in Claim 1, wherein said cell is selected from the group consisting of mammalian cell, human cell, normal somatic cell, diseased somatic cell, tumor cell, cancerous cell, human hair follicle, human skin cell, and human pluripotent stem cell.

3. The method as defined in Claim 1, wherein said recombinant nucleic acid composition includes a vector selected from the group consisting of plasmid, viral vector, and retrotransposon.

4. The method as defined in Claim 1, wherein said recombinant nucleic acid composition includes a drug-inducible gene expression promoter.

5. The method as defined in Claim 4, wherein said drug-inducible gene expression promoter is controlled by a tetracycline derivative or equivalent.

6. The method as defined in Claim 1, wherein said recombinant nucleic acid composition includes a 5'-donor splice site, an intronic insert site, a branch point motif, a poly-pyrimidine tract, and a 3'-acceptor splice site.

7. The method as defined in Claim 6, wherein said intronic insert site encodes said RNA molecule containing homology to mir-302.

8. The method as defined in Claim 1, wherein said RNA molecule further contains the sequence of either SEQ.ID.NO. 1 or SEQ.ID.NO2.

9. The method as defined in Claim 1, wherein said RNA molecule is a recombinant hairpin-like RNA including the sequence of SEQ.ID.NO9.

10. The method as defined in Claim 1, wherein said RNA molecule is a recombinant nucleic acid sequence comprising the RNA form of the sequence of SEQ.ID.NO.27 and SEQ.ID.NO.28.

11. The method as defined in Claim 1, wherein said RNA molecule contains the sequence of SEQ.ID.NO.9, SEQ.ID.NO10, SEQ.ID.NO.11, SEQ.ID.NO12, or SEQ.ID.NO13.

12. The method as defined in Claim 1, wherein said RNA molecule is formed by, comprises or contains the RNA form of the sequence of SEQ.ID.NO29, SEQ.ID.NO30, SEQ.ID.NO31, SEQ.ID.NO.32, SEQ.ID.NO.33, SEQ.ID.NO34, SEQ.ID.NO.35, and SEQ.ID.NO.36

13. The method as defined in Claim 1, wherein said pluripotent stem cells differentiate into germ line-like cells or normal somatic cells.

14. The method as defined in Claim 1, wherein said pluripotent stem cell is selectively isolated by using mir-302 microRNAs and Oct3/4 as markers.

## Patentansprüche

1. In-vitro-Verfahren zum Umprogrammieren mindestens einer Zelle zu mindestens einer pluripotenten Nicht-Tumor-Stammzelle, umfassend die folgenden Schritte:
(a) Herstellen einer rekombinanten Nukleinsäurezusammensetzung mit Befähigung zur Abgabe und Verarbeitung zu mindestens einem die Sequenz von SEQ ID Nr. 3 enthaltenden RNA-Molekül mit Befähigung zum Abschalten einer Mehrzahl von Genen, die von mir-302-, insbesondere CDK2-, Cyclin-D1- und Cyclin-D2-Genen, targetiert werden;
(b) Behandeln der Zelle in vitro mit besagter rekombinanter Nukleinsäurezusammensetzung, um die Mehrzahl von Genen einschließlich CDK2-, Cyclin-D1- und Cyclin-D2-Genen abzuschalten.

2. Verfahren nach Anspruch 1, worin besagte Zelle ausgewählt ist aus der Gruppe bestehend aus Säugetierzelle, menschlicher Zelle, normaler somatischer Zelle, erkrankter somatischer Zelle, Tumorzelle, Krebszelle, menschlichem Haarfollikel, menschlicher Hautzelle und menschlicher pluripotenter Stammzelle.

3. Verfahren nach Anspruch 1, worin besagte rekombinante Nukleinsäurezusammensetzung einen aus der Gruppe bestehend aus Plasmid, viralem Vektor und Retrotransposon ausgewählten Vektor beinhaltet.

4. Verfahren nach Anspruch 1, worin besagte rekombinante Nukleinsäurezusammensetzung einen Arzneimittel-induzierbaren Genexpressionspromoter beinhaltet.

5. Verfahren nach Anspruch 4, worin besagter Arzneimittel-induzierbarer Genexpressionspromoter von einem Tetracyclin-Derivat oder Äquivalent kontrolliert wird.

6. Verfahren nach Anspruch 1, worin besagte rekombinante Nukleinsäurezusammensetzung eine 5'-Donor-Spleißstelle, eine intronische Insertstelle, ein Verzweigungspunkt-Motiv, einen Poly-Pyrimidin-Trakt und eine 3'-Akzeptor-Spleißstelle beinhaltet.

7. Verfahren nach Anspruch 6, worin besagte intronische Insertstelle besagtes RNA-Molekül, das Homologie zu mir-302 enthält, codiert.

8. Verfahren nach Anspruch 1, worin besagtes RNA-Molekül ferner die Sequenz von entweder SEQ ID Nr. 1 oder SEQ ID Nr. 2 beinhaltet.

9. Verfahren nach Anspruch 1, worin besagtes RNA-Molekül eine die Sequenz von SEQ ID Nr. 9 beinhaltende rekombinante haarnadelartige RNA ist.

10. Verfahren nach Anspruch 1, worin besagtes RNA-Molekül eine die RNA-Form der Sequenz von SEQ ID Nr. 27 und SEQ ID Nr. 28 umfassende rekombinante Nukleinsäuresequenz ist.

11. Verfahren nach Anspruch 1, worin besagtes RNA-Molekül die Sequenz von SEQ ID Nr. 9, SEQ ID Nr. 10, SEQ ID Nr. 11, SEQ ID Nr. 12 oder SEQ ID Nr. 13 enthält.

12. Verfahren nach Anspruch 1, worin besagtes RNA-Molekül von der RNA-Form der Sequenz von SEQ ID Nr. 29, SEQ ID Nr. 30, SEQ ID Nr. 31, SEQ ID Nr. 32, SEQ ID Nr. 33, SEQ ID Nr. 34, SEQ ID Nr. 35 und SEQ ID Nr. 36 gebildet ist, diese umfasst oder enthält.

13. Verfahren nach Anspruch 1, worin besagte pluripotente Stammzellen zu keimbahnartigen Zellen oder normalen somatischen Zellen differenzieren.

14. Verfahren nach Anspruch 1, worin besagte pluripotente Stammzelle durch Verwendung von mir-302-Mikro-RNAs und Oct3/4 als Markern selektiv isoliert wird.

## Revendications

1. Procédé in vitro de reprogrammation d'au moins une cellule dans au moins une cellule-souche pluripotente non tumorale, comprenant les étapes consistant à :
(a) construire une composition d'acide nucléique recombinant capable d'être administrée, et traitée dans au moins une molécule d'ARN contenant la séquence SEQ. ID. NO 3 capable de silencer une pluralité de gènes ciblés par mir-302, en particulier les gènes CDK2, de la cycline D1 et de la cycline D2 ;
(b) traiter la cellule in vitro avec ladite composition d'acide nucléique recombinant pour silencer la pluralité de gènes comprenant les gènes CDK2, de la cycline D1 et de la cycline D2.

2. Procédé selon la revendication 1, dans lequel ladite cellule est choisie dans le groupe constitué d'une cellule de mammifère, d'une cellule humaine, d'une cellule somatique normale, d'une cellule somatique malade, d'une cellule tumorale, d'une cellule cancéreuse, d'un follicule pileux humain, d'une cellule cutanée humaine et d'une cellule-souche pluripotente humaine.

3. Procédé selon la revendication 1, dans lequel ladite composition d'acide nucléique recombinant comprend un vecteur choisi dans le groupe constitué d'un plasmide, d'un vecteur viral et d'un rétrotransposon.

4. Procédé selon la revendication 1, dans lequel ladite composition d'acide nucléique recombinant comprend un promoteur d'expression génique inductible par médicament.

5. Procédé selon la revendication 4, dans lequel ledit promoteur d'expression génique inductible par médicament est contrôlé par un dérivé de tétracycline ou un équivalent.

6. Procédé selon la revendication 1, dans lequel ladite composition d'acide nucléique recombinant comprend un site donneur 5' d'épissage, un site d'insertion intronique, un motif de point de ramification, un tractus polypyrimidine et un site récepteur 3' d'épissage.

7. Procédé selon la revendication 6, dans lequel ledit site d'insertion intronique code ladite molécule d'ARN contenant un homologue de mir-302.

8. Procédé selon la revendication 1, dans lequel ladite molécule d'ARN contient en outre la séquence de SEQ ID NO 1 ou SEQ ID NO 2.

9. Procédé selon la revendication 1, dans lequel ladite molécule d'ARN est un ARN recombinant en épingle à cheveux contenant la séquence de SEQ ID NO 9.

10. Procédé selon la revendication 1, dans lequel ladite molécule d'ARN est une séquence d'acide nucléique recombinant comprenant la forme d'ARN de la séquence de SEQ ID NO 27 et SEQ ID NO 28.

11. Procédé selon la revendication 1, dans lequel ladite molécule d'ARN contient la séquence de SEQ ID NO 9, SEQ ID NO 10, SEQ ID NO 11, SEQ ID NO 12 ou SEQ ID NO 13.

12. Procédé selon la revendication 1, dans lequel ladite molécule d'ARN est formée par, comprend ou contient la forme d'ARN de la séquence de SEQ ID NO 29, SEQ ID NO 30, SEQ ID NO 31, SEQ ID NO 32, SEQ ID NO 33, SEQ ID NO 34, SEQ ID NO 35 et SEQ ID NO 36.

13. Procédé selon la revendication 1, dans lequel lesdites cellules-souches pluripotentes se différencient en cellules de type de la lignée germinale ou en cellules somatiques normales.

14. Procédé selon la revendication 1, dans lequel ladite cellule-souche pluripotente est isolée de manière sélective au moyen de microARN de mir-302 et d'Oct3/4 en tant que marqueurs.
